# EUROPEAN PATENT APPLICATION

(11) **EP 2 898 880 A1**
(43) Date of publication of application: **29.07.2015**
(21) Application number: 14198890.7
(22) Date of filing: 06.12.2012
(51) Int. Cl.: A61K 31/047, A61K 31/165, A61K 31/192, A61K 31/216, A61P 31/04

(54) **Beta-O/S/N fatty acid based compounds as antibacterial and antiprotozoal agents**

(30) Priority: 06.12.2011 EP 11192121
(62) Divisional of application: 12799150.3
(71) Applicant: Ludwig-Maximilians-Universität München, 80539 München (DE); Technische Universität München, 80333 München (DE)
(72) Inventor: Böttcher, Thomas, 82216 Maisach (DE); Korotkov, Vadim, 81669 München (DE); Lorenz-Baath, Katrin, 85238 Petershausen (DE); Baron, Oliver, 80539 München (DE); Sieber, Stephan, 86919 Utting am Ammersee (DE)
(74) Representative: Ehnis, Tobias

(57) **Abstract**

The present invention relates to beta-O/S/N fatty acids and derivatives thereof, in particular the compounds of formula (I) as described and defined herein, and their pharmaceutical use, including their use in the treatment or prevention of bacterial as well as protozoan infections, in particular the treatment or prevention of infections with Gram-positive and/or Gram-negative bacteria and infectious diseases caused by and/or related to Gram-positive and/or Gram-negative bacteria. The invention further relates to the use of these compounds for preventing or eliminating biofilms.

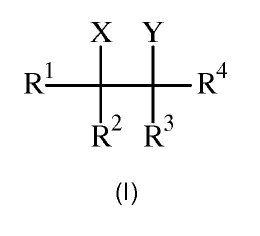

## Description

The present invention relates to beta-O/S/N fatty acids and derivatives thereof, in particular the compounds of formula (I) or (Ia) as described and defined herein, and their pharmaceutical use, including their use in the treatment or prevention of bacterial as well as protozoan infections, in particular the treatment or prevention of infections with Gram-positive and/or Gram-negative bacteria and infectious diseases caused by and/or related to Gram-positive and/or Gram-negative bacteria. The invention further relates to the use of these compounds for preventing or eliminating biofilms.

The development of multi-drug resistant bacterial pathogens has become a major problem for the treatment of bacterial infections and poses a severe threat for human health (G. Taubes, Science 2008, 321, 356). Rising pathogens like *Staphylococcus aureus* are characterized by diverse strains with resistances against virtually all available antibiotics and increasing aggressiveness leading to severe and often fatal infections. Especially the multi-resistant strains of S. *aureus* (MRSA) have become a global problem. Every year, about 500,000 people are infected with MRSA and, due to the lack of treatment options, 100,000 of them die (C. f. D. AHRQ, Organization, and Markets, Healthcare Cost and Utilization Project, Nationwide Inpatient Sample, 1993-2005). These bacteria have recently become even more aggressive and are leading not only to hospital acquired infections but also to community acquired infections which often cause chronic or even fatal diseases. The lack of effective treatment options is the result of the exhaustive exploitation of existing cellular targets and decreasing effectiveness of currently existing antibiotics. As resistances are evolving fast and spreading rapidly, innovative treatment strategies are urgently needed. It is thus a formidable aim to develop new compound classes that address novel yet unexploited cellular targets.

An innovative strategy is to target the virulence of pathogenic bacteria instead of viability (T. Bottcher, S. A. Sieber, J Am Chem Soc 2008, 130, 14400; T. Böttcher, S. A. Sieber, Chembiochem 2009, 10, 663). As the bacteria are not eliminated but disarmed, there is no direct selective pressure that would lead to rapid development of resistance. Furthermore, the beneficial human body flora, the microbiome, is preserved. This is a major problem of antibiotics which cause severe side-effects by destroying commensal and mutualistic microorganisms of the host endogenous microbiome. When the bacteria are disarmed, their ability to attack the human organism and escape the human immune response is impaired and the immune system finally eliminates the pathogens.

This novel and promising strategy of targeting virulence rather than viability has been developed recently using certain beta-lactones to inhibit the protease CIpP, the central regulator of virulence in many pathogenic bacteria (T. Bottcher, S. A. Sieber, J Am Chem Soc 2008, 130, 14400; T. Bottcher, S. A. Sieber, Chembiochem 2009, 10, 663; WO 2009/106211). The active site of CIpP is covalently modified by these beta-lactones creating a stable acyl-derivative of the active site serine residue which abolishes activity of the protease. Thereby, pathogenic bacteria are disarmed and become harmless for the human host. This strategy is expected to circumvent the problems of antibiotic treatment, like rapid resistance development and destruction of the host endogenous microbiome.

However, a drawback of beta-lactones is their intrinsic reactivity that makes them prone to hydrolysis in aqueous and biological matrices as well as their mechanism of covalently binding their target CIpP and potential cellular off-targets. Beta-lactones are suicide inhibitors and are thus consumed by the mechanism of enzyme inhibition.

The present invention provides a novel approach using a beta-O/S/N fatty acid or a derivative thereof, in particular a compound of formula (I) or (Ia) as described and defined herein, to inhibit the virulence of pathogenic bacteria or protozoa, in particular *Staphylococcus aureus.* The compounds of the invention, including the compounds of formula (I) and (Ia), have been found to be able to effectively inhibit the production of hemolysins, which are key virulence factors of S. *aureus* and important for infection of the human host.

Moreover, the compounds of the present invention do not suffer from the above described drawbacks associated with the use of beta-lactones as their structures are not reactive *per se.* They present thus a higher stability in solution, in particular in plasma, potentially leading to a higher bioavailability and thereby allowing for a reduction of the amount of compound to be used for therapy, as also indicated by the *in vitro* data in Example 2 which show decreased EC₅₀ values in virulence factor inhibition for compounds of the invention. The high stability of the compounds of the present invention allow for a systemic application of the compounds, e. g. for the treatment or prevention of an infection. Also, the compounds of the invention do not covalently bind to targets and potential off-targets when applied to a subject, leading to a reduction of potential side effects. The present invention thus solves the problem of providing therapeutic agents having an improved stability in aqueous solution and avoiding the covalent and, hence, irreversible binding of targets and potential off-targets, which agents are effective, *inter alia,* in the treatment or prevention of bacterial and/or protozoan infections.

Accordingly, the present invention provides a compound of the following formula (I) 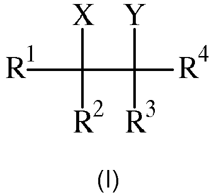 or a pharmaceutically acceptable salt, solvate or prodrug thereof.

In formula (I), X is selected from -CO-NR^{x1}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-O(aryl), -CO-O(heteroaryl), -CO-SH, -CO-S(C₁₋₆ alkyl), -CO-S(C₂₋₆ alkenyl), -CO-S(C₂₋₆ alkynyl), -CO-S(aryl), -CO-S(heteroaryl), -CH(-R^{x3})-O(C₁₋₆ alkyl), -CH(-R^{x3})-O(C₂₋₆ alkenyl), -CH(-R^{x3})-O(C₂₋₆ alkynyl), -CH(-R^{x3})-O(aryl), -CH(-R^{x3})-O(heteroaryl), -CH(-R^{x3})-SH, -CH(-R^{x3})-S(C₁₋₆ alkyl), -CH(-R^{x3})-S(C₂₋₆ alkenyl), -CH(-R^{x3})-S(C₂₋₆ alkynyl), -CH(-R^{x3})-S(aryl), -CH(-R^{x3})-S(heteroaryl), or -CH(-R^{x3})-NR^{x1}R^{x2}.

Preferably, X is selected from -CO-NR^{x1}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-O(aryl), -CO-O(heteroaryl), -CO-SH, -CO-S(C₁₋₆ alkyl), -CO-S(C₂₋₆ alkenyl), -CO-S(C₂₋₆ alkynyl), -CO-S(aryl), -CO-S(heteroaryl), -CH(-R^{x3})-SH, -CH(-R^{x3})-S(C₁₋₆ alkyl), or -CH(-R^{x3})-NR^{x1}R^{x2}. More preferably, X is selected from -CO-NR^{x1}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-SH, -CO-S(C₁₋₆ alkyl), -CO-S(C₂₋₆ alkenyl), -CO-S(C₂₋₆ alkynyl), -CH(-R^{x3})-SH, or -CH(-R^{x3})-NR^{x1}R^{x2}. Even more preferably, X is selected from -CO-NH₂, -COOH, -CH₂-OH, -CO-O(C₁₋₄ alkyl), -CO-SH, -CO-S(C₁₋₄ alkyl), -CO-NH(C₁₋₄ alkyl), -CO-N(C₁₋₄ alkyl)(C₁₋₄ alkyl), -CH₂-SH, -CH₂-NH₂, -CH₂-NH(C₁₋₄ alkyl) or -CH₂-N(C₁₋₄ alkyl)(C₁₋₄ alkyl). Even more preferably, X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl). Even more preferably, X is selected from -CO-NH₂, -COOH, -CH₂-OH, -CO-SH, -CH₂-SH, or -CH₂-NH₂. Yet even more preferably, X is -CO-NH₂, -COOH, or -CH₂-OH.

Y is selected from -OH, -O-CO-(C₁₋₆ alkyl), -O(C₁₋₆ alkyl), -O(C₂₋₆ alkenyl), -O(C₂₋₆ alkynyl), -O-CO-(C₂₋₆ alkenyl), -O-CO-(C₂₋₆ alkynyl), -O(aryl), -O(heteroaryl), -SH, -S(C₁₋₆ alkyl), -S(C₂₋₆ alkenyl), -S(C₂₋₆ alkynyl), -S-CO-(C₁₋₆ alkyl), -S-CO-(C₂₋₆ alkenyl), -S-CO-(C₂₋₆ alkynyl), -S(aryl), -S(heteroaryl), or-NR^{y1}R^{y2}.

Preferably, Y is selected from -OH, -O-CO-(C₁₋₆ alkyl), -O(C₁₋₆ alkyl), -SH, -S(C₁₋₆ alkyl), - S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or - N(C₁₋₆ alkyl)-CO-(C₁₋₆ alkyl). More preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Even more preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ and R³ are each independently selected from C₂₋₁₂ alkenyl, -(C₁₋₁₂ alkylene)-aryl, C₁₋₁₂ alkyl, C₂₋₁₂ alkynyl, aryl, heteroaryl, -(C₂₋₁₂ alkenylene)-aryl, -(C₂₋₁₂ alkynylene)-aryl, -(C₁₋₁₂ alkylene)-heteroaryl, -(C₂₋₁₂ alkenylene)-heteroaryl, -(C₂₋₁₂ alkynylene)-heteroaryl, a polyethylene glycol moiety, or C₃₋₁₂ alkyl in which at least one and up to one third of the C atoms are in each case substituted by an atom or a moiety independently selected from-O-, -S-, -NH-, -SO-, or -SO₂-, wherein said C₁₋₁₂ alkyl, said C₂₋₁₂ alkenyl, said C₂₋₁₂ alkynyl, said aryl, said heteroaryl, said -(C₁₋₁₂ alkylene)-aryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, said -(C₂₋₁₂ alkynylene)-heteroaryl, said polyethylene glycol moiety, or said C₃₋₁₂ alkyl may be substituted with one or more groups (e.g., one, two, three or four groups) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, heteroaryl, polyethylene glycol moiety, alkylene-polyethylene glycol moiety or polyethylene glycol-alkyl moiety. If X is -CH₂-OH at least one of R¹ and R³ can be said -(C₁₋₁₂ alkylene)-aryl, said aryl, said heteroaryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, or said -(C₂₋₁₂ alkynylene)-heteroaryl or may comprise at least 4 consecutively arranged C-atoms that are covalently linked together.
R¹ may be -CH₂CH₂OCH₂CH₂OCH₃.

Preferably, R¹ and R³ are each independently selected from C₃₋₁₂ alkenyl, -(C₁₋₆ alkylene)-phenyl, C₃₋₁₂ alkyl, C₃₋₁₂ alkynyl, phenyl, -(C₂₋₆ alkenylene)-phenyl, or -(C₂₋₆ alkynylene)-phenyl, wherein said C₃₋₁₂ alkyl, said C₃₋₁₂ alkenyl, said C₃₋₁₂ alkynyl, said phenyl, said -(C₁₋₆ alkylene)-phenyl, said -(C₂₋₆ alkenylene)-phenyl or said -(C₂₋₆ alkynylene)-phenyl is optionally substituted with one or more groups (preferably one or two groups, more preferably one group) independently selected from halogen, -CN,-CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl. It is furthermore preferred that the aforementioned groups (i.e., the groups listed above for R¹ and R³) are unsubstituted. More preferably, one of R¹ and R³ is C₃₋₁₂ alkenyl, C₃₋₁₂ alkyl, C₃₋₁₂ alkynyl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, and the other one of R¹ and R³ is -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety. Even more preferably, one of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl. Even more preferably, one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl. Yet even more preferably, one of R¹ and R³ is linear C₇₋₁₀ alkenyl (such as, e.g., -(CH₂)₇-CH=CH₂) or linear C₇₋₁₀ alkyl, and the other one of R¹ and R³ is -(linear C₁₋₄ alkylene)-phenyl (such as, e.g., -(CH₂)₂-phenyl).

In one embodiment R¹ and R³ are each independently selected from C₃₋₁₂ alkenyl, C₃₋₁₂ alkyl, C₃₋₁₂ alkynyl, a polyethylene glycol moiety, C₃₋₁₂ alkyl in which at least one and up to one third of the C atoms are in each case substituted by an atom or a moiety independently selected from -O-, -S-, -NH-, -SO-, or -SO₂-, -(C₁₋₆ alkylene)-phenyl, -(C₂₋₆ alkenylene)-phenyl, -(C₂₋₆ alkynylene)-phenyl or phenyl, wherein at least one of R¹ and R³ is said -(C₁₋₆ alkylene)-phenyl, said -(C₂₋₆ alkenylene)-phenyl, said -(C₂₋₆ alkynylene)-phenyl or said phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety.

At least one of R¹ and R³ may comprise at least 4 consecutively arranged C-atoms that are covalently linked together.

R² and R⁴ are each independently selected from -H, C₁₋₆ alkyl, C₂₋₆ alkenyl, C₂₋₆ alkynyl, -O-(C₁₋₆ alkyl), -S-(C₁₋₆ alkyl), -NH₂, -NH(C₁₋₆ alkyl), -N(C₁₋₆ alkyl)(C₁₋₆ alkyl), aryl, heteroaryl, halogen, -CN, or -CF₃, wherein said C₁₋₆ alkyl, said C₂₋₆ alkenyl, said C₂₋₆ alkynyl, the alkyl moiety of said -O-(C₁₋₆ alkyl), the alkyl moiety of said -S-(C₁₋₆ alkyl), the alkyl moiety of said -NH(C₁₋₆ alkyl), one or both of the alkyl moieties of said -N(C₁₋₆ alkyl)(C₁₋₆ alkyl), said aryl or said heteroaryl is optionally substituted with one or more groups independently selected from halogen, -CN, -CF₃, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl.

Preferably, R² and R⁴ are each independently selected from -H, C₁₋₄ alkyl, C₂₋₄ alkenyl, C₂₋₄ alkynyl, -O-(C₁₋₄ alkyl), -S-(C₁₋₄ alkyl), -NH₂, -NH(C₁₋₄ alkyl), -N(C₁₋₄ alkyl)(C₁₋₄ alkyl), halogen, -CN, or -CF₃. More preferably, R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or-CF₃. Even more preferably, R² and R⁴ are each independently -H or C₁₋₄ alkyl. Yet even more preferably, R² and R⁴ are each -H.

R^{x1} and R^{x2} are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), -O-(C₂₋₆ alkynyl), aryl, heteroaryl, -(C₁₋₆ alkylene)-aryl, -(C₂₋₆ alkenylene)-aryl, -(C₂₋₆ alkynylene)-aryl, -(C₁₋₆ alkylene)-heteroaryl, -(C₂₋₆ alkenylene)-heteroaryl, -(C₂₋₆ alkynylene)-heteroaryl, -O-(C₁₋₆ alkylene)-aryl, -O-(C₂₋₆ alkenylene)-aryl, -O-(C₂₋₆ alkynylene)-aryl, -O-(C₁₋₆ alkylene)-heteroaryl, -O-(C₂₋₆ alkenylene)-heteroaryl, -O-(C₂₋₆ alkynylene)-heteroaryl, -CO-(C₁₋₆ alkylene)-aryl, -CO-(C₂₋₆ alkenylene)-aryl, -CO-(C₂₋₆ alkynylene)-aryl, -CO-(C₁₋₆ alkylene)-heteroaryl, -CO-(C₂₋₆ alkenylene)-heteroaryl, or -CO-(C₂₋₆ alkynylene)-heteroaryl.

Preferably, R^{x1} and R^{x2} are each independently selected from -H, C₁₋₄ alkyl, C₂₋₄ alkenyl, or C₂₋₄ alkynyl. More preferably, R^{x1} and R^{x2} are each independently selected from -H or C₁₋₄ alkyl. It is particularly preferred that R^{x1} and R^{x2} are each -H.

R^{x3} is selected from -H, C₁₋₆ alkyl, C₂₋₆ alkenyl, C₂₋₆ alkynyl, -O-(C₁₋₆ alkyl), -S-(C₁₋₆ alkyl), -NH₂, -NH(C₁₋₆ alkyl), -N(C₁₋₆ alkyl)(C₁₋₆ alkyl), aryl, heteroaryl, halogen, -CN, or -CF₃, wherein said 1C₁₋₆ alkyl, said C₂₋₆ alkenyl, said C₂₋₆ alkynyl, the alkyl moiety of said -O-(C₁₋₆ alkyl), the alkyl moiety of said -S-(C₁₋₆ alkyl), the alkyl moiety of said -NH(C₁₋₆ alkyl), one or both of the alkyl moieties of said -N(C₁₋₆ alkyl)(C₁₋₆ alkyl), said aryl or said heteroaryl is optionally substituted with one or more groups independently selected from halogen, -CN, -CF₃, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl.

Preferably, R^{x3} is selected from -H, C₁₋₄ alkyl, C₂₋₄ alkenyl, C₂₋₄ alkynyl, -O-(C₁₋₄ alkyl), -S-(C₁₋₄ alkyl), -NH₂, -NH(C₁₋₄ alkyl), -N(C₁₋₄ alkyl)(C₁₋₄ alkyl), halogen, -CN, or -CF₃. More preferably, R^{x3} is selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Even more preferably, R^{x3} is -H or C₁₋₄ alkyl. Yet even more preferably, R^{x3} is -H.

R^{y1} and R^{y2} are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), -O-(C₂₋₆ alkynyl), aryl, heteroaryl, -(C₁₋₆ alkylene)-aryl, -(C₂₋₆ alkenylene)-aryl, -(C₂₋₆ alkynylene)-aryl, -(C₁₋₆ alkylene)-heteroaryl, -(C₂₋₆ alkenylene)-heteroaryl, -(C₂₋₆ alkynylene)-heteroaryl, -O-(C₁₋₆ alkylene)-aryl, -O-(C₂₋₆ alkenylene)-aryl, -O-(C₂₋₆ alkynylene)-aryl, -O-(C₁₋₆ alkylene)-heteroaryl, -O-(C₂₋₆ alkenylene)-heteroaryl, -O-(C₂₋₆ alkynylene)-heteroaryl, -CO-(C₁₋₆ alkylene)-aryl, -CO-(C₂₋₆ alkenylene)-aryl, -CO-(C₂₋₆ alkynylene)-aryl, -CO-(C₁₋₆ alkylene)-heteroaryl, -CO-(C₂₋₆ alkenylene)-heteroaryl, or -CO-(C₂₋₆ alkynylene)-heteroaryl.

Preferably, R^{y1} and R^{y2} are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), or -O-(C₂₋₆ alkynyl). More preferably, R^{y1} and R^{y2} are each independently selected from -H, C₁₋₆ alkyl, or -CO-(C₁₋₆ alkyl). Even more preferably, R^{y1} and R^{y2} are each independently selected from -H, C₁₋₄ alkyl, or -CO-(C₁₋₄ alkyl) (such as, e.g., -CO-CH₃).

It is particularly preferred that the compound of formula (I) is a compound, wherein X is selected from -COOH, -CONH₂, or -COOCH₃, Y is -OH, R¹ is selected from -(CH₂)₇-CH=CH₂, -(CH₂)₇-C≡CH, -(CH₂)₈-CH₃, or -(CH₂)₂-phenyl, R² is -H, R³ is selected from-CH₂-phenyl, -(CH₂)₂-phenyl, or -(CH₂)₃-phenyl, wherein said phenyl is optionally substituted with -CN₂CF₃ or benzoyl, and R⁴ is -H. The compound may be a compound of one of the following formulae or a pharmaceutically acceptable salt, solvate or prodrug thereof, wherein Me is methyl:

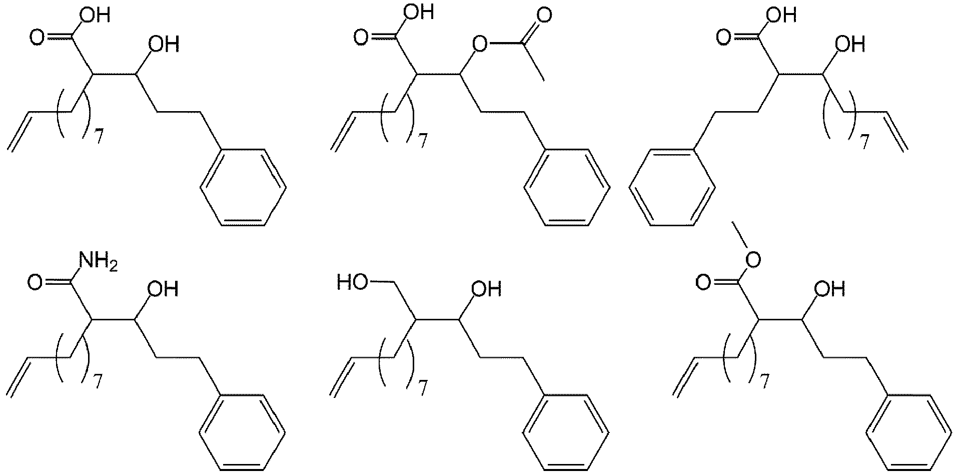

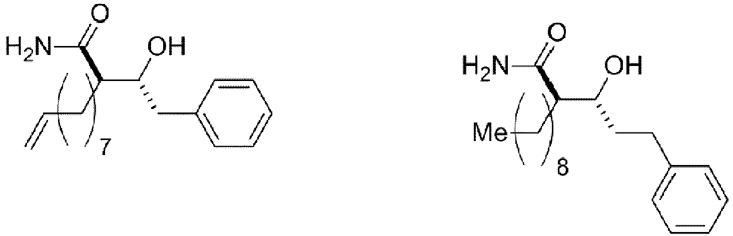

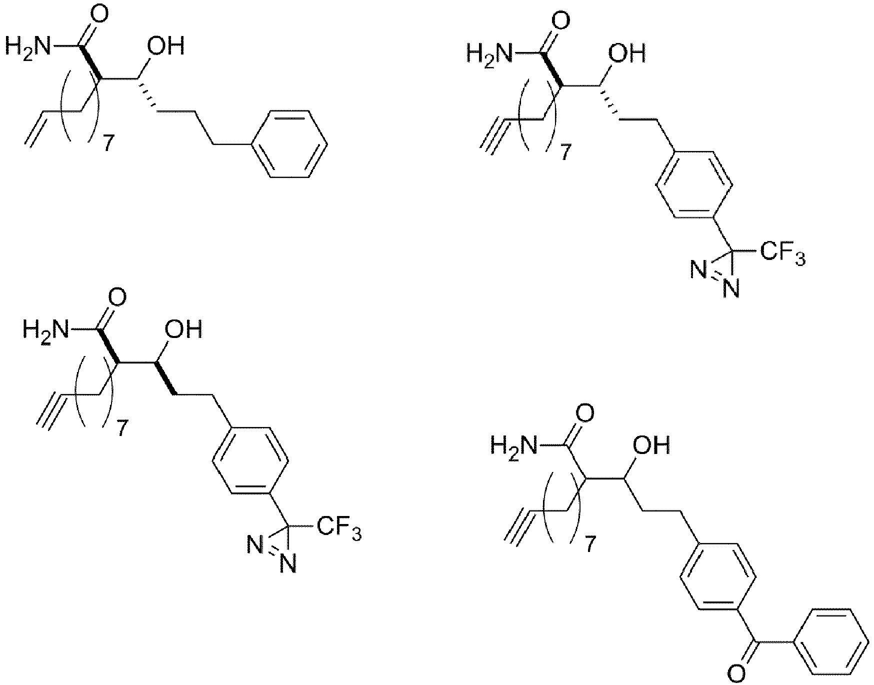

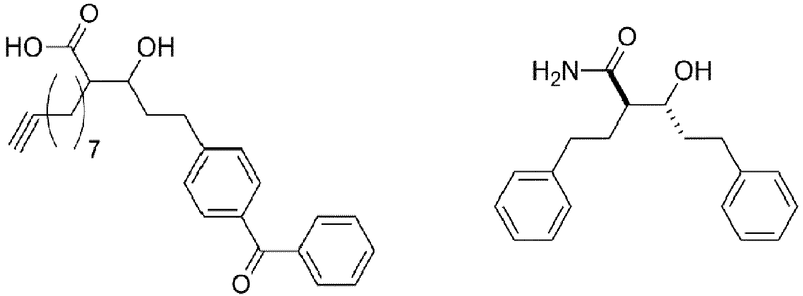

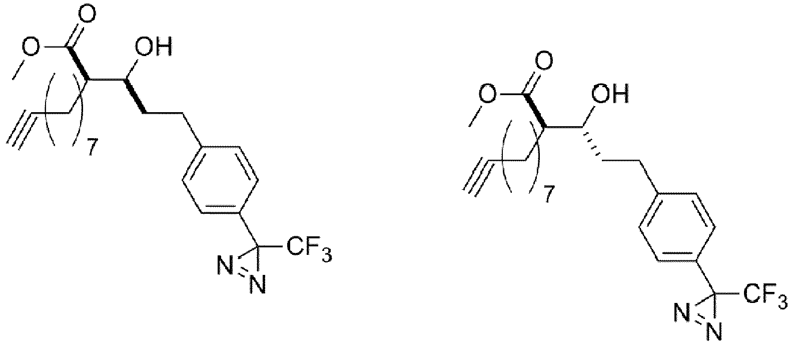

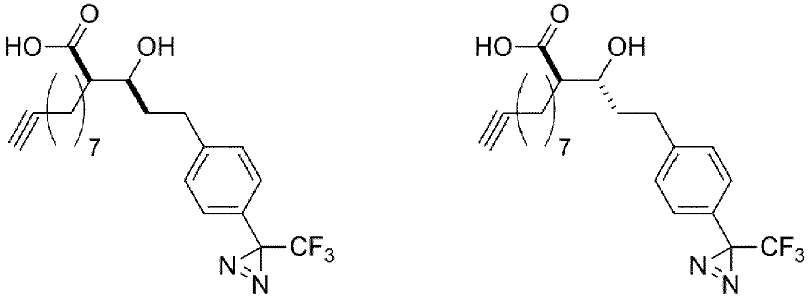

Further particularly preferred compounds of formula (I) are the compounds depicted above, wherein the terminal carbon-to-carbon double bond is replaced by a carbon-to-carbon single bond, as well as pharmaceutically acceptable salts, solvates and prodrugs thereof.

These compounds of formula (I) preferably have the following stereo-configuration:

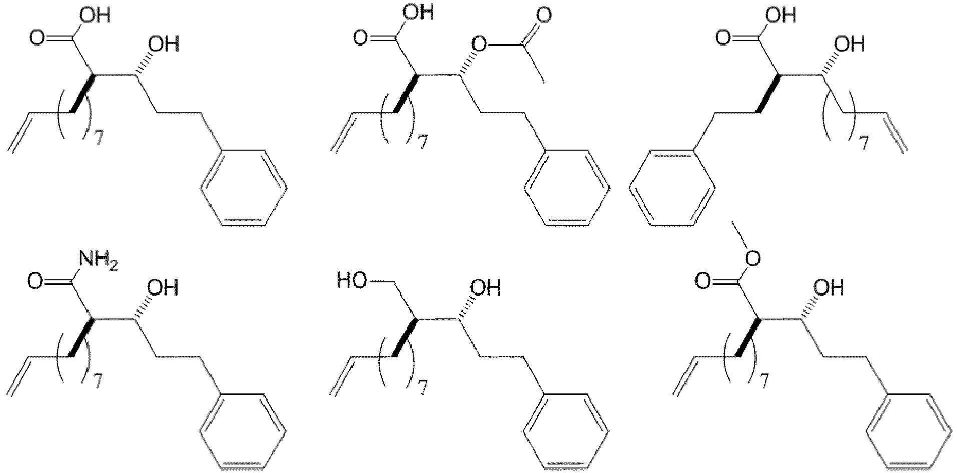

It is generally preferred that the compounds of formula (I) or (Ia), as described and defined herein, are present in the trans-configuration with respect to the groups R¹ and Y, in accordance with the configuration of the preferred compounds depicted above.

A particularly preferred compound of formula (I) is the following compound (also referred to as "AVU1AA") or a pharmaceutically acceptable salt, solvate or prodrug thereof:

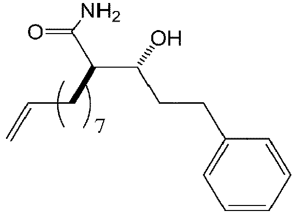

The invention also relates to a compound having the structure shown for AVU1AA above, in which the terminal carbon-to-carbon double bond is replaced by a carbon-to-carbon single bond, or a pharmaceutically acceptable salt, solvate or prodrug thereof.

In a specific embodiment, the groups R¹, R², R³, R⁴, X, Y, R^{x1}, R^{x2} and R^{x3} comprised in the compound of formula (I) have the following meanings:
X is selected from -CO-NR^{x1}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-SH, -CO-S(C₁₋₆ alkyl), -CO-S(C₂₋₆ alkenyl), -CO-S(C₂₋₆ alkynyl), -CH(-R^{x3})-SH, or -CH(-R^{x3})-NR^{x1}R^{x2}. Preferably, X is selected from -CO-NH₂, -COOH, -CH₂-OH, -CO-O(C₁₋₄ alkyl), -CO-SH, -CO-S(C₁₋₄ alkyl), -CO-NH(C₁₋₄ alkyl), -CO-N(C₁₋₄ alkyl)(C₁₋₄ alkyl), -CH₂-SH, -CH₂-NH₂, -CH₂-NH(C₁₋₄ alkyl) or -CH₂-N(C₁₋₄ alkyl)(C₁₋₄ alkyl). More preferably, X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl). Even more preferably, X is selected from -CO-NH₂, -COOH, -CH₂-OH, -CO-SH, -CH₂-SH, or -CH₂-NH₂. Yet even more preferably, X is -CO-NH₂, -COOH, or -CH₂-OH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ and R³ are each independently selected from C₃₋₁₂ alkenyl, -(C₁₋₆ alkylene)-phenyl, C₃₋₁₂ alkyl, C₃₋₁₂ alkynyl, phenyl, -(C₂₋₆ alkenylene)-phenyl, or -(C₂₋₆ alkynylene)-phenyl, wherein said C₃₋₁₂ alkyl, said C₃₋₁₂ alkenyl, said C₃₋₁₂ alkynyl, said phenyl, said -(C₁₋₆ alkylene)-phenyl, said -(C₂₋₆ alkenylene)-phenyl or said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl. Preferably, one of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). More preferably, one of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl. Even more preferably, one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl. Yet even more preferably, one of R¹ and R³ is linear C₇₋₁₀ alkenyl (such as, e.g., -(CH₂)₇-CH=CH₂) or linear C₇₋₁₀ alkyl, and the other one of R¹ and R³ is -(linear C₁₋₄ alkylene)-phenyl (such as, e.g., -(CH₂)₂-phenyl). For example, one of R¹ and R³ may be -(CH₂)₇-CH=CH₂ and the other one of R¹ and R³ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

R^{x1} and R^{x2} are each independently selected from -H, C₁₋₄ alkyl, C₂₋₄ alkenyl, or C₂₋₄ alkynyl. Preferably, R^{x1} and R^{x2} are each independently selected from -H or C₁₋₄ alkyl. More preferably, R^{x1} and R^{x2} are each -H.

R^{x3} is selected from -H, C₁₋₄ alkyl, C₂₋₄ alkenyl, C₂₋₄ alkynyl, -O-(C₁₋₄ alkyl), -S-(C₁₋₄ alkyl), -NH₂, -NH(C₁₋₄ alkyl), -N(C₁₋₄ alkyl)(C₁₋₄ alkyl), halogen, -CN, or -CF₃. Preferably, R^{x3} is selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. More preferably, R^{x3} is -H or C₁₋₄ alkyl. Even more preferably, R^{x3} is -H.

In a further specific embodiment, the groups R¹, R², R³, R⁴, X and Y comprised in the compound of formula (I) have the following meanings:
X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl). Preferably, X is selected from -CO-NH₂, -COOH, -CH₂-OH, -CO-SH, -CH₂-SH, or -CH₂-NH₂. More preferably, X is -CO-NH₂, -COOH, or -CH₂-OH.

Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). More preferably, Y is -OH or -O-CO-(C₁₋₆ alkyl). Even more preferably, Y is -OH or -O-CO-CH₃.

One of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, one of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl. More preferably, one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl. Even more preferably, one of R¹ and R³ is linear C₇₋₁₀ alkenyl (such as, e.g., -(CH₂)₇-CH=CH₂) or linear C₇₋₁₀ alkyl, and the other one of R¹ and R³ is -(linear C₁₋₄ alkylene)-phenyl (such as, e.g., -(CH₂)₂-phenyl). For example, one of R¹ and R³ may be -(CH₂)₇-CH=CH₂ and the other one of R¹ and R³ may be -(CH₂)₂-phenyl.

R² and R⁴ are each independently -H or C₁₋₄ alkyl. Preferably, R² and R⁴ are each -H.

In a further specific embodiment, the groups R¹, R², R³, R⁴, X and Y comprised in the compound of formula (I) have the following meanings:
X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl). Preferably, X is selected from -CO-NH₂, -COOH, -CH₂-OH, -CO-SH, -CH₂-SH, or -CH₂-NH₂. More preferably, X is -CO-NH₂, -COOH, or -CH₂-OH.

Y is selected from -OH, -O-CO-(C₁₋₆ alkyl), -NH₂, or -NH-CO-(C₁₋₆ alkyl). Preferably, Y is -OH or -O-CO-(C₁₋₆ alkyl). More preferably, Y is -OH or -O-CO-CH₃.

R¹ and R³ are each independently selected from C₃₋₁₂ alkenyl, -(C₁₋₆ alkylene)-phenyl, C₃₋₁₂ alkyl, C₃₋₁₂ alkynyl, phenyl, -(C₂₋₆ alkenylene)-phenyl, or -(C₂₋₆ alkynylene)-phenyl. For example, R¹ and R³ may each be selected independently from C₃₋₁₂ alkyl, C₃₋₁₂ alkenyl, or -(C₁₋₆ alkylene)-phenyl. Preferably, one of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl. More preferably, one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl. Even more preferably, one of R¹ and R³ is linear C₇₋₁₀ alkenyl (such as, e.g., -(CH₂)₇-CH=CH₂) or linear C₇₋₁₀ alkyl, and the other one of R¹ and R³ is -(linear C₁₋₄ alkylene)-phenyl (such as, e.g., -(CH₂)₂-phenyl). For example, one of R¹ and R³ may be -(CH₂)₇-CH=CH₂ and the other one of R¹ and R³ may be -(CH₂)₂-phenyl.

R² and R⁴ are each -H.

In a further specific embodiment, the groups R¹, R², R³, R⁴, X, Y, R^{x1}, R^{x2} and R^{x3} comprised in the compound of formula (I) have the following meanings:
X is selected from -CO-NR^{x}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-SH, -CO-S(C₁₋₆ alkyl), -CO-S(C₂₋₆ alkenyl), -CO-S(C₂₋₆ alkynyl), -CH(-R^{x3})-SH, or -CH(-R^{x3})-NR^{x1}R^{x2}. Preferably, X is selected from -CO-NH₂, -COOH, -CH₂-OH, -CO-O(C₁₋₄ alkyl), -CO-SH, -CO-S(C₁₋₄ alkyl), -CO-NH(C₁₋₄ alkyl), -CO-N(C₁₋₄ alkyl)(C₁₋₄ alkyl), -CH₂-SH, -CH₂-NH₂, -CH₂-NH(C₁₋₄ alkyl) or -CH₂-N(C₁₋₄ alkyl)(C₁₋₄ alkyl). More preferably, X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl). Even more preferably, X is selected from -CO-NH₂, -COOH, -CH₂-OH, -CO-SH, -CH₂-SH, or -CH₂-NH₂. Yet even more preferably, X is -CO-NH₂, -COOH, or -CH₂-OH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl. More preferably, R¹ is selected from linear C₃₋₁₂ alkenyl, linear C₃₋₁₂ alkyl, or linear C₃₋₁₂ alkynyl, and R³ is selected from -(linear C₁₋₆ alkylene)-phenyl, -(linear C₂₋₆ alkenylene)-phenyl, -(linear C₂₋₆ alkynylene)-phenyl or phenyl. Even more preferably, R¹ is linear C₇₋₁₀ alkenyl (such as, e.g., -(CH₂)₇-CH=CH₂) or linear C₇₋₁₀ alkyl, and R³ is -(linear C₁₋₄ alkylene)-phenyl (such as, e.g., -(CH₂)₂-phenyl). For example, R¹ may be -(CH₂)₇-CH=CH₂ and R³ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

R^{x1} and R^{x2} are each independently selected from -H, C₁₋₄ alkyl, C₂₋₄ alkenyl, or C₂₋₄ alkynyl. Preferably, R^{x1} and R^{x2} are each independently selected from -H or C₁₋₄ alkyl. More preferably, R^{x1} and R^{x2} are each -H.

R^{x3} is selected from -H, C₁₋₄ alkyl, C₂₋₄ alkenyl, C₂₋₄ alkynyl, -O-(C₁₋₄ alkyl), -S-(C₁₋₄ alkyl), -NH₂, -NH(C₁₋₄ alkyl), -N(C₁₋₄ alkyl)(C₁₋₄ alkyl), halogen, -CN, or -CF₃. Preferably, R^{x3} is selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. More preferably, R^{x3} is -H or C₁₋₄ alkyl. Even more preferably, R^{x3} is -H.

In a further specific embodiment, the groups R¹, R², R³, R⁴, X, Y, R^{x1}, R^{x2} and R^{x3} comprised in the compound of formula (I) have the following meanings:
X is selected from -CO-NR^{x}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-SH, -CO-S(C₁₋₆ alkyl), -CO-S(C₂₋₆ alkenyl), -CO-S(C₂₋₆ alkynyl), -CH(-R^{x3})-SH, or -CH(-R^{x3})-NR^{x1}R^{x2}. Preferably, X is selected from -CO-NH₂, -COOH, -CH₂-OH, -CO-O(C₁₋₄ alkyl), -CO-SH, -CO-S(C₁₋₄ alkyl), -CO-NH(C₁₋₄ alkyl), -CO-N(C₁₋₄ alkyl)(C₁₋₄ alkyl), -CH₂-SH, -CH₂-NH₂, -CH₂-NH(C₁₋₄ alkyl) or -CH₂-N(C₁₋₄ alkyl)(C₁₋₄ alkyl). More preferably, X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl). Even more preferably, X is selected from -CO-NH₂, -COOH, -CH₂-OH, -CO-SH, -CH₂-SH, or -CH₂-NH₂. Yet even more preferably, X is -CO-NH₂, -COOH, or -CH₂-OH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl. More preferably, R³ is selected from linear C₃₋₁₂ alkenyl, linear C₃₋₁₂ alkyl, or linear C₃₋₁₂ alkynyl, and R¹ is selected from -(linear C₁₋₆ alkylene)-phenyl, -(linear C₂₋₆ alkenylene)-phenyl, -(linear C₂₋₆ alkynylene)-phenyl or phenyl. Even more preferably, R³ is linear C₇₋₁₀ alkenyl (such as, e.g., -(CH₂)₇-CH=CH₂) or linear C₇₋₁₀ alkyl, and R¹ is -(linear C₁₋₄ alkylene)-phenyl (such as, e.g., -(CH₂)₂-phenyl). For example, R³ may be -(CH₂)₇-CH=CH₂ and R¹ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

R^{x1} and R^{x2} are each independently selected from -H, C₁₋₄ alkyl, C₂₋₄ alkenyl, or C₂₋₄ alkynyl. Preferably, R^{x1} and R^{x2} are each independently selected from -H or C₁₋₄ alkyl. More preferably, R^{x1} and R^{x2} are each -H.

R^{x3} is selected from -H, C₁₋₄ alkyl, C₂₋₄ alkenyl, C₂₋₄ alkynyl, -O-(C₁₋₄ alkyl), -S-(C₁₋₄ alkyl), -NH₂, -NH(C₁₋₄ alkyl), -N(C₁₋₄ alkyl)(C₁₋₄ alkyl), halogen, -CN, or -CF₃. Preferably, R^{x3} is selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. More preferably, R^{x3} is -H or C₁₋₄ alkyl. Even more preferably, R^{x3} is -H.

In a further specific embodiment, the groups R¹, R², R³, R⁴, X and Y comprised in the compound of formula (I) have the following meanings:
X is -CO-NH₂.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl. More preferably, R¹ is selected from linear C₃₋₁₂ alkenyl, linear C₃₋₁₂ alkyl, or linear C₃₋₁₂ alkynyl, and R³ is selected from -(linear C₁₋₆ alkylene)-phenyl, -(linear C₂₋₆ alkenylene)-phenyl, -(linear C₂₋₆ alkynylene)-phenyl or phenyl. Even more preferably, R¹ is linear C₇₋₁₀ alkenyl (such as, e.g., -(CH₂)₇-CH=CH₂) or linear C₇₋₁₀ alkyl, and R³ is -(linear C₁₋₄ alkylene)-phenyl (such as, e.g., -(CH₂)₂-phenyl). For example, R¹ may be -(CH₂)₇-CH=CH₂ and R³ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

In a further specific embodiment, the groups R¹, R², R³, R⁴, X and Y comprised in the compound of formula (I) have the following meanings:
X is -CO-NH₂.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl. More preferably, R³ is selected from linear C₃₋₁₂ alkenyl, linear C₃₋₁₂ alkyl, or linear C₃₋₁₂ alkynyl, and R¹ is selected from -(linear C₁₋₆ alkylene)-phenyl, -(linear C₂₋₆ alkenylene)-phenyl, -(linear C₂₋₆ alkynylene)-phenyl or phenyl. Even more preferably, R³ is linear C₇₋₁₀ alkenyl (such as, e.g., -(CH₂)₇-CH=CH₂) or linear C₇₋₁₀ alkyl, and R¹ is -(linear C₁₋₄ alkylene)-phenyl (such as, e.g., -(CH₂)₂-phenyl). For example, R³ may be -(CH₂)₇-CH=CH₂ and R¹ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

In a further specific embodiment, the groups R¹, R², R³, R⁴, X and Y comprised in the compound of formula (I) have the following meanings:
X is -COOH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl. More preferably, R¹ is selected from linear C₃₋₁₂ alkenyl, linear C₃₋₁₂ alkyl, or linear C₃₋₁₂ alkynyl, and R³ is selected from -(linear C₁₋₆ alkylene)-phenyl, -(linear C₂₋₆ alkenylene)-phenyl, -(linear C₂₋₆ alkynylene)-phenyl or phenyl. Even more preferably, R¹ is linear C₇₋₁₀ alkenyl (such as, e.g., -(CH₂)₇-CH=CH₂) or linear C₇₋₁₀ alkyl, and R³ is -(linear C₁₋₄ alkylene)-phenyl (such as, e.g., -(CH₂)₂-phenyl). For example, R¹ may be -(CH₂)₇-CH=CH₂ and R³ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

In a further specific embodiment, the groups R¹, R², R³, R⁴, X and Y comprised in the compound of formula (I) have the following meanings:
X is -COOH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl. More preferably, R³ is selected from linear C₃₋₁₂ alkenyl, linear C₃₋₁₂ alkyl, or linear C₃₋₁₂ alkynyl, and R¹ is selected from -(linear C₁₋₆ alkylene)-phenyl, -(linear C₂₋₆ alkenylene)-phenyl, -(linear C₂₋₆ alkynylene)-phenyl or phenyl. Even more preferably, R³ is linear C₇₋₁₀ alkenyl (such as, e.g., -(CH₂)₇-CH=CH₂) or linear C₇₋₁₀ alkyl, and R¹ is -(linear C₁₋₄ alkylene)-phenyl (such as, e.g., -(CH₂)₂-phenyl). For example, R³ may be -(CH₂)₇-CH=CH₂ and R¹ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

In a further specific embodiment, the groups R¹, R², R³, R⁴, X and Y comprised in the compound of formula (I) have the following meanings:
X is -CH₂-OH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R¹ is selected from C₃₋₁₂ alkenyl, C₃₋₁₂ alkyl, or C₃₋₁₂ alkynyl, and R³ is selected from -(C₁₋₆ alkylene)-phenyl, -(C₂₋₆ alkenylene)-phenyl, -(C₂₋₆ alkynylene)-phenyl or phenyl. More preferably, R¹ is selected from linear C₃₋₁₂ alkenyl, linear C₃₋₁₂ alkyl, or linear C₃₋₁₂ alkynyl, and R³ is selected from -(linear C₁₋₆ alkylene)-phenyl, -(linear C₂₋₆ alkenylene)-phenyl, -(linear C₂₋₆ alkynylene)-phenyl or phenyl. Even more preferably, R¹ is linear C₇₋₁₀ alkenyl (such as, e.g., -(CH₂)₇-CH=CH₂) or linear C₇₋₁₀ alkyl, and R³ is -(linear C₁₋₄ alkylene)-phenyl (such as, e.g., -(CH₂)₂-phenyl). For example, R¹ may be -(CH₂)₇-CH=CH₂ and R³ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

In a further specific embodiment, the groups R¹, R², R³, R⁴, X and Y comprised in the compound of formula (I) have the following meanings:
X is -CH₂-OH.

Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), or -CO-N(C₁₋₆ alkyl)(C₁₋₆ alkyl). Preferably, R³ is selected from C₃₋₁₂ alkenyl, C₃₋₁₂ alkyl, or C₃₋₁₂ alkynyl, and R¹ is selected from -(C₁₋₆ alkylene)-phenyl, -(C₂₋₆ alkenylene)-phenyl, -(C₂₋₆ alkynylene)-phenyl or phenyl. More preferably, R³ is selected from linear C₃₋₁₂ alkenyl, linear C₃₋₁₂ alkyl, or linear C₃₋₁₂ alkynyl, and R¹ is selected from -(linear C₁₋₆ alkylene)-phenyl, -(linear C₂₋₆ alkenylene)-phenyl, -(linear C₂₋₆ alkynylene)-phenyl or phenyl. Even more preferably, R³ is linear C₇₋₁₀ alkenyl (such as, e.g., -(CH₂)₇-CH=CH₂) or linear C₇₋₁₀ alkyl, and R¹ is -(linear C₁₋₄ alkylene)-phenyl (such as, e.g., -(CH₂)₂-phenyl). For example, R³ may be -(CH₂)₇-CH=CH₂ and R¹ may be -(CH₂)₂-phenyl.

R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Preferably, R² and R⁴ are each independently -H or C₁₋₄ alkyl. More preferably, R² and R⁴ are each -H.

In a preferred embodiment, the compound of formula (I) is a compound of formula (Ia) or a pharmaceutically acceptable salt, solvate or prodrug thereof:

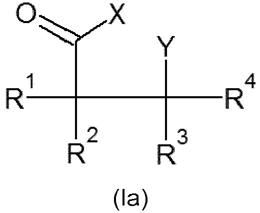

In formula (Ia), X is selected from -NR^{x1}R^{x2}, -OH, -O(C₁₋₆ alkyl), -O(C₂₋₆ alkenyl), -O(C₂₋₆ alkynyl), -O(aryl), -O(heteroaryl), -SH, -S(C₁₋₆ alkyl), -S(C₂₋₆ alkenyl), -S(C₂₋₆ alkynyl), -S(aryl), or -S(heteroaryl). Preferably, X is selected from -NR^{x1}R^{x2}, -OH, -O(C₁₋₆ alkyl), -O(C₂₋₆ alkenyl), -O(C₂₋₆ alkynyl), -SH, -S(C₁₋₆ alkyl), -S(C₂₋₆ alkenyl), or -S(C₂₋₆ alkynyl). More preferably, X is selected from -NH₂, -OH, -O(C₁₋₄ alkyl), -SH, -S(C₁₋₄ alkyl), -NH(C₁₋₄ alkyl), or -N(C₁₋₄ alkyl)(C₁₋₄ alkyl). Even more preferably, X is selected from -NH₂, -OH, -NH(C₁₋₄ alkyl), -N(C₁₋₄ alkyl)(C₁₋₄ alkyl), or -SH. Even more preferably, X is selected from -NH₂, -OH, or -SH. Yet even more preferably, X is -NH₂ or -OH.

Y in formula (Ia) is selected from -OH, -O-CO-(C₁₋₆ alkyl), -O(C₁₋₆ alkyl), -O(C₂₋₆ alkenyl), -O(C₂₋₆ alkynyl), -O-CO-(C₂₋₆ alkenyl), -O-CO-(C₂₋₆ alkynyl), -O(aryl), -O(heteroaryl), -SH, -S(C₁₋₆ alkyl), -S(C₂₋₆ alkenyl), -S(C₂₋₆ alkynyl), -S-CO-(C₁₋₆ alkyl), -S-CO-(C₂₋₆ alkenyl), -S-CO-(C₂₋₆ alkynyl), -S(aryl), -S(heteroaryl), or -NR^{y1}R^{y2}. Preferably, Y is selected from -OH, -O-CO-(C₁₋₆ alkyl), -O(C₁₋₆ alkyl), -SH, -S(C₁₋₆ alkyl), -S-CO-(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). More preferably, Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl). Even more preferably, Y is selected from -OH, -O-CO-(C₁₋₆ alkyl) (such as, e.g., -O-CO-CH₃), -NH₂, or -NH-CO-(C₁₋₆ alkyl) (such as, e.g., -NH-CO-CH₃). Even more preferably, Y is -OH or -O-CO-(C₁₋₆ alkyl). Yet even more preferably, Y is -OH or -O-CO-CH₃.

R¹ and R³ in formula (Ia) are each independently selected from C₂₋₁₂ alkenyl, -(C₁₋₁₂alkylene)-aryl, C₁₋₁₂ alkyl, C₂₋₁₂ alkynyl, aryl, heteroaryl, -(C₂₋₁₂ alkenylene)-aryl, -(C₂₋₁₂ alkynylene)-aryl, -(C₁₋₁₂ alkylene)-heteroaryl, -(C₂₋₁₂ alkenylene)-heteroaryl, -(C₂₋₁₂ alkynylene)-heteroaryl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, wherein said C₁₋₁₂ alkyl, said C₂₋₁₂ alkenyl, said C₂₋₁₂ alkynyl, said aryl, said heteroaryl, said -(C₁₋₁₂ alkylene)-aryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, said -(C₂₋₁₂ alkynylene)-heteroaryl, said polyethylene glycol moiety, or said C₃₋₁₂ alkyl may be substituted with one or more groups (e.g., one, two, three or four groups) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, heteroaryl, polyethylene glycol moiety, alkylene-polyethylene glycol moiety or polyethylene glycol-alkyl moiety. At least one of R¹ and R³ can be said -(C₁₋₁₂alkylene)-aryl, said aryl, said heteroaryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, or said -(C₂₋₁₂ alkynylene)-heteroaryl or may comprise at least 4 consecutively arranged C-atoms that are covalently linked together.

R¹ may be -CH₂CH₂OCH₂CH₂OCH₃.

Preferably, R¹ and R³ are each independently selected from C₃₋₁₂ alkenyl, -(C₁₋₆ alkylene)-phenyl, C₃₋₁₂ alkyl, C₃₋₁₂ alkynyl, phenyl, -(C₂₋₆ alkenylene)-phenyl, or -(C₂₋₆ alkynylene)-phenyl, wherein said C₃₋₁₂ alkyl, said C₃₋₁₂ alkenyl, said C₃₋₁₂ alkynyl, said phenyl, said -(C₁₋₆ alkylene)-phenyl, said -(C₂₋₆ alkenylene)-phenyl or said -(C₂₋₆ alkynylene)-phenyl is optionally substituted with one or more groups (preferably one or two groups, more preferably one group) independently selected from halogen, -CN,-CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl. It is furthermore preferred that the aforementioned groups (i.e., the groups listed above for R¹ and R³) are unsubstituted. More preferably, one of R¹ and R³ is C₃₋₁₂ alkenyl, C₃₋₁₂ alkyl, C₃₋₁₂ alkynyl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, and the other one of R¹ and R³ is -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups (preferably one or two groups, more preferably one group) independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety. Even more preferably, one of R¹ and R³ is C₃₋₁₂ alkenyl, C₃₋₁₂ alkyl, or C₃₋₁₂ alkynyl, and the other one of R¹ and R³ is -(C₁₋₆ alkylene)-phenyl, -(C₂₋₆ alkenylene)-phenyl, -(C₂₋₆ alkynylene)-phenyl or phenyl. Even more preferably, one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl. Yet even more preferably, one of R¹ and R³ is linear C₇₋₁₀ alkenyl (such as, e.g., -(CH₂)₇-CH=CH₂) or linear C₇₋₁₀ alkyl, and the other one of R¹ and R³ is -(linear C₁₋₄ alkylene)-phenyl (such as, e.g., -(CH₂)₂-phenyl).

In one embodiment R¹ and R³ are each independently selected from C₃₋₁₂ alkenyl, C₃₋₁₂ alkyl, C₃₋₁₂ alkynyl, a polyethylene glycol moiety, C₃₋₁₂ alkyl in which at least one and up to one third of the C atoms are in each case substituted by an atom or a moiety independently selected from -O-, -S-, -NH-, -SO-, or -SO₂-, -(C₁₋₆ alkylene)-phenyl, -(C₂₋₆ alkenylene)-phenyl, -(C₂₋₆ alkynylene)-phenyl or phenyl, wherein at least one of R¹ and R³ is said -(C₁₋₆ alkylene)-phenyl, said -(C₂₋₆ alkenylene)-phenyl, said -(C₂₋₆ alkynylene)-phenyl or said phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety.

R² and R⁴ in formula (Ia) are each independently selected from -H, C₁₋₆ alkyl, C₂₋₆ alkenyl, C₂₋₆ alkynyl, -O-(C₁₋₆ alkyl), -S-(C₁₋₆ alkyl), -NH₂, -NH(C₁₋₆ alkyl), -N(C₁₋₆ alkyl)(C₁₋₆ alkyl), aryl, heteroaryl, halogen, -CN, or -CF₃, wherein said C₁₋₆ alkyl, said C₂₋₆ alkenyl, said C₂₋₆ alkynyl, the alkyl moiety of said -O-(C₁₋₆ alkyl), the alkyl moiety of said -S-(C₁₋₆ alkyl), the alkyl moiety of said -NH(C₁₋₆ alkyl), one or both of the alkyl moieties of said -N(C₁₋₆ alkyl)(C₁₋₆ alkyl), said aryl or said heteroaryl is optionally substituted with one or more groups independently selected from halogen, -CN, -CF₃, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl. Preferably, R² and R⁴ are each independently selected from -H, C₁₋₄ alkyl, C₂₋₄ alkenyl, C₂₋₄ alkynyl, -O-(C₁₋₄ alkyl), -S-(C₁₋₄ alkyl), -NH₂, -NH(C₁₋₄ alkyl), -N(C₁₋₄ alkyl)(C₁₋₄ alkyl), halogen, -CN, or -CF₃. More preferably, R² and R⁴ are each independently selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Even more preferably, R² and R⁴ are each independently -H or C₁₋₄ alkyl. Yet even more preferably, R² and R⁴ are each -H.

R^{x1} and R^{x2} in formula (Ia) are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), -O-(C₂₋₆ alkynyl), aryl, heteroaryl, -(C₁₋₆ alkylene)-aryl, -(C₂₋₆ alkenylene)-aryl, -(C₂₋₆ alkynylene)-aryl, -(C₁₋₆ alkylene)-heteroaryl, -(C₂₋₆ alkenylene)-heteroaryl, -(C₂₋₆ alkynylene)-heteroaryl, -O-(C₁₋₆ alkylene)-aryl, -O-(C₂₋₆ alkenylene)-aryl, -O-(C₂₋₆ alkynylene)-aryl, -O-(C₁₋₆ alkylene)-heteroaryl, -O-(C₂₋₆ alkenylene)-heteroaryl, -O-(C₂₋₆ alkynylene)-heteroaryl, -CO-(C₁₋₆ alkylene)-aryl, -CO-(C₂₋₆ alkenylene)-aryl, -CO-(C₂₋₆ alkynylene)-aryl, -CO-(C₁₋₆ alkylene)-heteroaryl, -CO-(C₂₋₆ alkenylene)-heteroaryl, or -CO-(C₂₋₆ alkynylene)-heteroaryl. Preferably, R^{x1} and R^{x2} are each independently selected from -H, C₁₋₄ alkyl, C₂₋₄ alkenyl, or C₂₋₄ alkynyl. More preferably, R^{x1} and R^{x2} are each independently selected from -H or C₁₋₄ alkyl. It is particularly preferred that R^{x1} and R^{x2} are each -H.

R^{x3} in formula (Ia) is selected from -H, C₁₋₆ alkyl, C₂₋₆ alkenyl, C₂₋₆ alkynyl, -O-(C₁₋₆ alkyl), - S-(C₁₋₆ alkyl), -NH₂, -NH(C₁₋₆ alkyl), -N(C₁₋₆ alkyl)(C₁₋₆ alkyl), aryl, heteroaryl, halogen, -CN, or -CF₃, wherein said C₁₋₆ alkyl, said C₂₋₆ alkenyl, said C₂₋₆ alkynyl, the alkyl moiety of said -O-(C₁₋₆ alkyl), the alkyl moiety of said -S-(C₁₋₆ alkyl), the alkyl moiety of said -NH(C₁₋₆ alkyl), one or both of the alkyl moieties of said -N(C₁₋₆ alkyl)(C₁₋₆ alkyl), said aryl or said heteroaryl is optionally substituted with one or more groups independently selected from halogen, -CN, -CF₃, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl. Preferably, R^{x3} is selected from -H, C₁₋₄ alkyl, C₂₋₄ alkenyl, C₂₋₄ alkynyl, -O-(C₁₋₄ alkyl), -S-(C₁₋₄ alkyl), -NH₂,-NH(C₁₋₄ alkyl), -N(C₁₋₄ alkyl)(C₁₋₄ alkyl), halogen, -CN, or -CF₃. More preferably, R^{x3} is selected from -H, C₁₋₄ alkyl, -O-(C₁₋₄ alkyl), -NH₂, -NH(C₁₋₄ alkyl), -N(C₁₋₄ alkyl)(C₁₋₄ alkyl), halogen, or -CF₃. Even more preferably, R^{x3} is -H or C₁₋₄ alkyl. Yet even more preferably, R^{x3} is -H.

R^{y1} and R^{y2} in formula (Ia) are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), -O-(C₂₋₆ alkynyl), aryl, heteroaryl, -(C₁₋₆ alkylene)-aryl, -(C₂₋₆ alkenylene)-aryl, -(C₂₋₆ alkynylene)-aryl, -(C₁₋₆ alkylene)-heteroaryl, -(C₂₋₆ alkenylene)-heteroaryl, -(C₂₋₆ alkynylene)-heteroaryl, -O-(C₁₋₆ alkylene)-aryl, -O-(C₂₋₆ alkenylene)-aryl, -O-(C₂₋₆ alkynylene)-aryl, -O-(C₁₋₆ alkylene)-heteroaryl, -O-(C₂₋₆ alkenylene)-heteroaryl, -O-(C₂₋₆ alkynylene)-heteroaryl, -CO-(C₁₋₆ alkylene)-aryl, -CO-(C₂₋₆ alkenylene)-aryl, -CO-(C₂₋₆ alkynylene)-aryl, -CO-(C₁₋₆ alkylene)-heteroaryl, -CO-(C₂₋₆ alkenylene)-heteroaryl, or -CO-(C₂₋₆ alkynylene)-heteroaryl. Preferably, R^{y1} and R^{y2} are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), or -O-(C₂₋₆ alkynyl). More preferably, R^{y1} and R^{y2} are each independently selected from -H, C₁₋₆ alkyl, or -CO-(C₁₋₆ alkyl). Even more preferably, R^{y1} and R^{y2} are each independently selected from -H, C₁₋₄ alkyl, or -CO-(C₁₋₄ alkyl) (such as, e.g., -CO-CH₃).

The compounds of the present invention, including the compounds of formula (I) and the compounds of formula (Ia), can be prepared by methods known in the field of synthetic chemistry. For example, the compounds of formula (I) or (Ia) can be prepared in accordance with or in analogy to the synthetic routes described in Example 1. Accordingly, the compounds of the invention can, e.g., be prepared by hydrolysis, aminolysis or reduction of the corresponding β-lactones which, in turn, can be prepared as described, e.g., in WO 2009/106211.

The present invention also relates to a pharmaceutical composition comprising a compound of formula (I) or (Ia), as described and defined herein, or a pharmaceutically acceptable salt, solvate or prodrug thereof, and a pharmaceutically acceptable excipient. Accordingly, the invention relates to a compound of formula (I) or (Ia) or a pharmaceutically acceptable salt, solvate or prodrug thereof, or a pharmaceutical composition comprising any of the aforementioned entities and a pharmaceutically acceptable excipient, for use as a medicament.

The invention further relates to a compound of formula (I) or (Ia) or a pharmaceutically acceptable salt, solvate or prodrug thereof, or a pharmaceutical composition comprising any of the aforementioned entities and a pharmaceutically acceptable excipient, for use in the treatment or prevention of a bacterial or protozoan infection, particularly a bacterial infection, as well as the treatment or prevention of an infectious disease caused by and/or related to bacteria or protozoa.

Moreover, the present invention relates to a method of treating or preventing a disease or disorder, in particular a bacterial or protozoan infection or an infectious disease caused by and/or related to bacteria or protozoa, the method comprising the administration of a compound of formula (I) or (Ia) or a pharmaceutically acceptable salt, solvate or prodrug thereof, or a pharmaceutical composition comprising any of the aforementioned entities and a pharmaceutically acceptable excipient, to a subject (preferably, a human) in need of such treatment or prevention.

The compounds of the present invention, including the compounds of formula (I) and the compounds of formula (Ia), have been found to be highly effective in reducing or inhibiting the virulence of bacterial and/or protozoan pathogens, particularly of Gram-positive bacteria such as, e.g., *S. aureus,* as also shown in Example 2, but constitute a different class of chemical substances than the known beta-lactones. This makes them preferred lead structures for anti-virulence therapies with a wide spectrum of possible administrations. There is no inherent reactivity under physiological conditions and, thus, no stability problems are encountered. In particular, the compounds of the present invention have been found to show improved activity in virulence inhibition as compared to corresponding beta-lactone compounds, as also described in Example 2 with respect to, e.g., the inhibition of *S. aureus* hemolysin production. The compounds of the present invention thus have improved properties with respect to stability, activity and application as compared to known anti-virulence drugs such as beta-lactones.

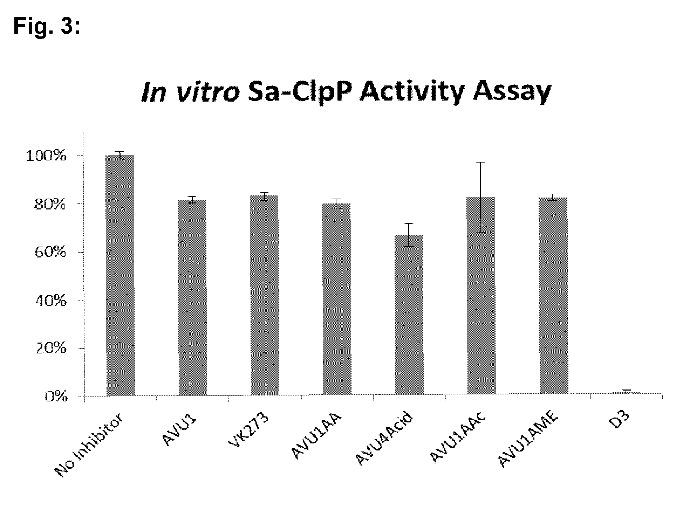

In a CIpP activity assay with purified *S. aureus* CIpP recombinantly overexpressed in *E. coli* using SLY-AMC (i.e., N-succinyl-Leu-Tyr-7-amido-4-methylcoumarin) as fluorogenic model substrate, compounds of the present invention showed an at least comparable target enzyme inhibition to reference beta-lactones such as AVU1 ("U1"; WO 2009/106211), which are the most potent virulence inhibitors in living bacteria, as also shown in Example 3 and Figure 3. To date, highly potent *in vivo* candidate beta-lactone structures have been shown to inhibit CIpP enzyme activity only partially, as do the compounds of the present invention.

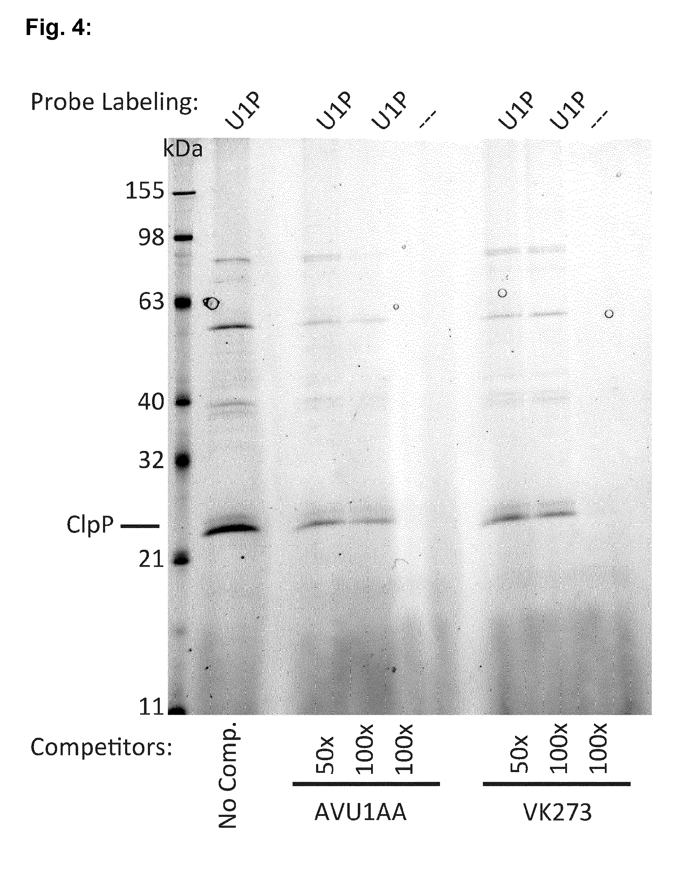

The inventors further demonstrated that compounds of the present invention are able to reduce CIpP target binding of a covalently binding probe in a competitive ABPP (activity based protein profiling) proteome labeling experiment, as also shown in Example 4 and Figure 4, which indicates the activity of the compounds of the invention on the CIpP target enzyme. The inventors thus concluded that the main target of the compounds provided herein is CIpP.

CIpP proteases are highly conserved proteins which can be found not only in bacteria but, *inter alia,* also in protozoa including, e.g., protozoa of the phylum *Apicomplexa.* For example, *Plasmodium* CIpP and *Staphylococcus* CIpP are highly homologous and share a conserved binding site (Vedadi M, et al. Mol Biochem Parasitol. 2007. 151(1):100-110; EI Bakkouri M, et al. J Mol Biol. 2010. 404(3):456-477; Lin W, et al. Parasitol Res. 2009. 105(6):1715-1722). The compounds of the present invention, including the compounds of formula (I) or (Ia), are thus considered to bind to homologs of CIpP in protozoa and, accordingly, are envisaged to be used in the treatment or prevention of protozoan infections, as also described and discussed in more detail herein below.

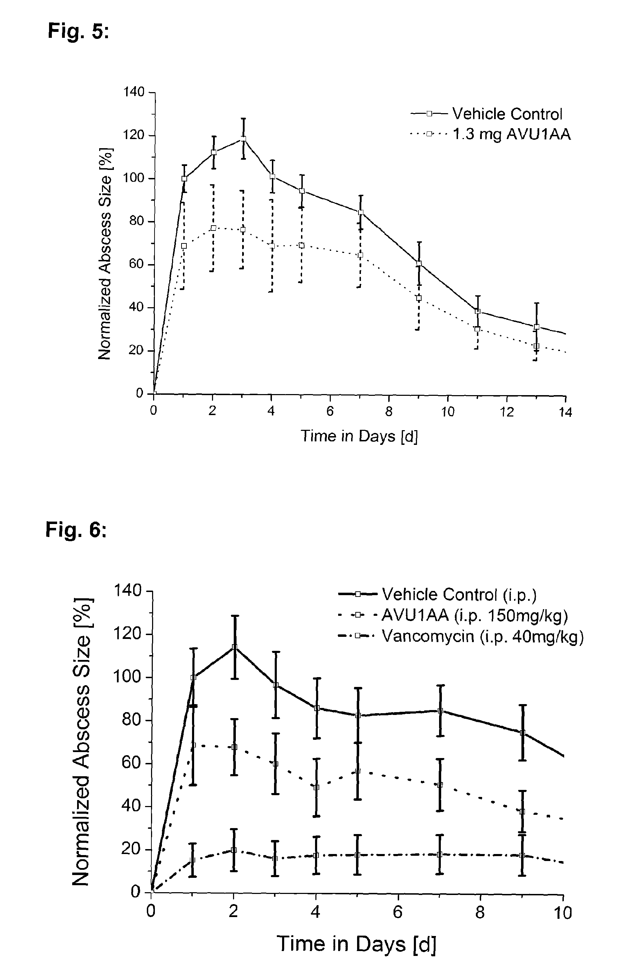

First *in vivo* efficacy experiments in mice further support the therapeutic activity of the compounds according to the invention. Using an infection model on mice subcutaneously infected with *S. aureus* pathogens, it could be shown that compounds of the invention effectively reduced the size of abscesses formed as a result of the infections as shown in Example 5 and Figure 5 and Example 6 and Figure 6.

It is known that many antibiotics, especially beta-lactams like methicillins lead in subinhibitory concentrations to increased virulence of *S. aureus* (K. Ohlsen et al., Antimicrobial Agents Chemother. 1998, 42, 2817). This may cause severe problems for the management of various diseases such as sepsis or toxic shock syndrome in which from the beginning of treatment on any further production of toxins must be avoided. The compounds of the present inventions can advantageously be used in combination with existing antibiotics to ameliorate or abolish their adverse effects on bacterial virulence. Additionally reducing virulence by the compounds of the present invention can also be used as pre-treatment of diseases like sepsis to prevent the liberation of toxins from lysed bacteria during antibacterial treatment with antibiotics.

It is known in the art that bacteria may develop multiple resistances against known antibiotics. For example, specific multi-resistant *S. aureus* strains are resistant to all known beta-lactam antibiotics (such as methicillin, oxacillin, flucloxacillin) and are accordingly called methicillin-resistant (or multidrug-resistant) *Staphylococcus aureus* (MRSA) strains. Only few antibiotics (inter alia, glycopeptides, such as vancomycin or teicoplanin) can be used to treat infections which are, *inter alia,* caused by MRSA strains. However, some MRSA strains have become resistant to vancomycin, too (vancomycin intermediate *Staphylococcus aureus* (VISA) and vancomycin resistant *Staphylococcus aureus* (VRSA) strains). Also vancomycin resistant Enterococci (VRE), penicillin resistant pneumococci and multiple resistant gram negative bacteria are known in the art. In the context of the present invention, the term "Enterococcus" refers to a genus of Gram-positive, lactic acid bacteria of the phylum Firmicutes. The term "pneumococci" used herein refers to bacteria belonging to the Gram-positive species Streptococcus pneumoniae.

The compounds of the present invention can advantageously be used for treating or preventing bacterial infections caused by multi-drug resistant bacteria, including, e.g., multi-drug resistant strains of any of the specific bacteria described herein, and particularly the above-described multi-resistant bacteria. Accordingly, the present invention relates to a compound of formula (I) or a pharmaceutically acceptable salt, solvate or prodrug thereof, or a pharmaceutical composition comprising any of the aforementioned entities and a pharmaceutically acceptable excipient, for use in the treatment or prevention of a bacterial infection caused by multidrug-resistant (MDR) and/or extensively drug-resistant (XDR) bacteria, particularly a bacterial infection caused by multidrug-resistant *Staphylococcus aureus* (MRSA).

Some bacteria are also intrinsically resistant (i.e. resistance is not acquired) to antibiotics. For example, Gram-positive Leuconostoc and Pediococcus species and most Lactobacillus species as well as most gram-negative bacteria are intrinsically resistant to vancomycin. Accordingly, the compound of the present invention may be particularly useful for the treatment of bacterial infections which are, *inter alia,* caused by intrinsically resistant bacteria.

It is known that antibiotics, in particular penicillin and derivatives thereof, may elicit allergic reactions in about 1 out of 7000 patients. The compounds of the present invention may thus be advantageous for the treatment of bacterial infections, whereby the subjects to be treated are allergic to antibiotics known in the art.

Antibiotics have been used to target the viability of bacteria, for example, by inhibiting cell wall synthesis. As many bacteria have become resistant to known antibiotics targeting their viability, it is highly desirable to prevent the formation of resistant strains. In contrast to such antibiotics known in the art, the compounds of the invention do not target bacterial viability but target virulence factors essential for infection, which are required to cause host damage and disease. For example, the compounds of the invention have been found to effectively reduce the hemolytic activity of *S. aureus* on sheep blood agar, as also shown in Example 2. Accordingly, the compounds of the present invention are advantageous in that they may exert less selective pressure on pathogenic bacteria which may result in decreased resistance.

As a further advantageous property, the compounds of the present invention may be used to treat or prevent bacterial infections caused by a broad range of Gram-positive and/or Gram-negative bacteria.

The present invention relates to a compound of formula (I) or (Ia) as described and defined herein for use in the treatment or prevention of a bacterial or protozoan infection, particularly a bacterial infection, or an infectious disease caused by and/or related to bacteria or protozoa, in particular an infectious disease caused by and/or related to bacteria. The terms "bacterial infection" and "protozoan infection" used in the context of the present invention mean transfer, lodgement and penetration of bacteria or protozoa, respectively, in a macroorganism such as, e.g., a human or an animal and propagation of the bacteria or the protozoa in said macroorganism. The meaning of the term "infection" may also be deduced from standard textbooks, such as Pschyrembel (Klinisches Wörterbuch, 257. edition, 1994). A bacterial infection which causes pain or suffering in a subject may generally be considered as "bacterial infectious disease". The treatment or prevention of a bacterial infection, as referred to herein, also includes the treatment or prevention of a disease induced by and/or related to a bacterial infection. The compounds of the present invention may also be used for the treatment or prevention of a bacterial infection even if the infection does not cause pain or suffering in a subject. Accordingly, the treatment or prevention of a protozoan infection, as referred to herein, also includes the treatment or prevention of a disease induced by and/or related to a protozoan infection.

The bacterial infection to be treated or prevented using a compound of the present invention may be caused by one ore more bacterial species. The term "bacteria" used herein means prokaryotic, unicellular organisms and refers to the evolutionary domains "Bacteria" and "Archaea". Exemplary phyla of bacteria are Acidobacteria, Actinobacteria, Aquificae, Bacteroidetes, Chlamydiae, Chlorobi, Chloroflexi, Chrysiogenetes, Cyanobacteria, Deferribacteres, Deinococcus-Thermus, Dictyoglomi, Fibrobacteres, Firmicutes, Fusobacteria, Gemmatimonadetes, Nitrospirae, Planctomycetes, Proteobacteria, Spirochaetes, Thermodesulfobacteria, Thermomicrobia, Thermotogae, and Verrucomicrobia.

It is envisaged that the bacterial infection to be treated or prevented in accordance with the present invention may be caused by Gram-positive or Gram-negative bacteria, in particular pathogenic Gram-positive or Gram-negative bacteria. The terms "Gram-positive" and "Gram-negative" bacteria are well known in the art.

Generally, Gram-positive bacteria are bacteria that appear violet (or blue or purple) after Gram-staining by retaining a crystal violet dye in their cell wall during the staining process. The cell wall of Gram-positive bacteria comprises a thick peptidoglycan layer outside their cytoplasmic membrane and has no additional outer membrane. In contrast, Gram-negative bacteria appear pink (or red) after Gram-staining. Their cell wall comprises a thin peptidoglycan layer and an outer membrane.

Gram-positive bacteria comprise, with very few exceptions, the groups of Firmicutes and Actinobacteria (Actinomycetes), while all Proteobacteria species are Gram-negative. Also the terms "Firmicutes", "Actinobacteria" and "Proteobacteria" are well known in the art.

Gram-positive Firmicutes comprise, for example, the "Bacilli", including but not limited to the genera Bacillus, Listeria, Staphylococcus, Enterococcus, Lactobacillus, Lactococcus, Leuconostoc, Pediococcus, and Streptococcus, "Clostridia" (e.g. the genera Acetobacterium, Clostridium, Eubacterium, Heliobacterium, Heliospirillum, Pectinatus, and Sporomusa) and Mollicutes (e.g. genera Mycoplasma, Spiroplasma, Ureaplasma, and Erysipelothrix). Actinobacteria, include for example the Actinomyces, Arthrobacter, Corynebacterium, Frankia, Micrococcus, Micromonospora, Nocardia, Propionibacterium, Streptomyces genera and Mycobacterium. Mycobacteria-induced diseases to be treated in accordance with the present invention include, *inter alia,* tuberculosis, leprosy, tropical skin ulcer, ulceration, abscess, pulmonary disease, granulomatous (skin) disease, opportunistic infections with non-tuberculous mycobacteria as well as diseases elicited by atypical mycobacteria such as *M. avium* including pulmonary disease, lymphadenitis, cutaneous and disseminated diseases, e.g. in immunocompromised patients. The use is not restricted to mycobacteria-induced diseases in humans, but comprises also the use of the present invention in animal diseases, like bovine tuberculosis. Furthermore, the compounds of the present invention are useful in the medical intervention of bacterial infections in general and, in particular, infections with Gram-positive bacteria, including particularly Firmicutes and Actinobacteria, like Staphylococcus spec or Listeria spec.

Non-limiting examples of bacterial species which belong to the groups of Firmicutes and Actinobacteria are *Listeria monocytogenes, Listeria welshimeri, Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Mycobacterium tuberculosis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroides,* and *Rhodococcus equi.* Accordingly, the invention relates to the treatment or prevention of infections with the above described bacteria using a compound of formula (I) or (Ia).

Exemplary Gram-negative bacteria belong, inter alia, to the genera Escherichia, Salmonella, Pseudomonas, Moraxella, Helicobacter, Stenotrophomonas, Bdellovibrio, Legionella, Vibrio, Neisseria, Hemophilus, Chlamydia, Klebsiella, Legionella, Proteus, Enterobacter, Serratia. Further Gram-negative bacteria are, inter alia, acetic acid bacteria. Non-limiting examples of Gram-negative bacteria species are *Neisseria gonorrhoeae, Neisseria meningitidis, Moraxella catarrhalis, Haemophilus influenzae, Klebsiella pneumoniae, Legionella pneumophila, Pseudomonas aeruginosa, Pseudomonas putida, Escherichia coli, Proteus mirabilis, Enterobacter cloaceae, Serratia marcescens, Helicobacter pylori, Salmonella enteritidis,* and *Salmonella typhi.* It is envisaged that the compounds of the present invention, including the compounds of formula (I) and the compounds of formula (Ia), are to be used for inhibiting the virulence of Gram-negative bacteria. Accordingly, the invention relates to the treatment or prevention of infections with the above described Gram-negative bacteria using a compound of formula (I) or (Ia).

The compounds of the present invention are envisaged to be used for the treatment or prevention of bacterial infections caused by extracellular as well as intracellular pathogenic bacteria. As mentioned herein above, such bacterial infections comprise, but are not limited to, infections with *Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Listeria monocytogenes, Listeria welshimeri, Mycobacterium tuberculosis, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroides, Rhodococcus equi, Neisseria gonorrhoeae, Neisseria meningitidis, Moraxella catarrhalis, Haemophilus influenzae, Klebsiella pneumoniae, Legionella pneumophila, Pseudomonas aeruginosa, Pseudomonas putida, Escherichia coli, Proteus mirabilis, Enterobacter cloaceae, Serratia marcescens, Helicobacter pylori, Salmonella enteritidis,* or *Salmonella typhi, in particular with Staphylococcus aureus* or *Listeria monocytogenes.*

Exemplary bacterial infections to be treated or prevented in accordance with the present invention are, inter alia, listeriosis, cranial nerve palsy, encephalitis, meningitis, meningoencephalitis, abscesses, endocarditis, pneumonia, cholera, syphilis, anthrax, leprosy, bubonic plague, sepsis (blood poisoning), septic arthritis, osteitis, inflammation of wounds, furuncles, carbuncles, toxic shock syndrome, Staphylococcal scalded skin syndrome (SSSS), and mastitis. Also disease patterns resulting from unbalanced human microbiome and abnormal bacterial colonization, such as, e.g., atopic dermatitis and/or associated exacerbation or colitis, are envisage to be treated or prevented using the compound of the present invention.

Abscesses to be treated or prevented in accordance with the invention are, inter alia, abscesses in brains, abscesses in lungs and abscesses in kidneys. Generally, it is envisaged herein that inflammations of organs (such as inflammation of bladder, pneumonia, and the like) may be treated or prevented in accordance with the present invention. The term "osteitis" used in context of the present invention refers to an inflammation of bones or inflammation of bone marrow (commonly known as "osteomyelitis"). "Furuncles" used in context of the present invention are also known as "boils" while the term "carbuncles" used herein means a collection of furuncles. The term "toxic shock syndrome" (TSS) used herein refers to a rare but potentially fatal disease caused by a bacterial toxin. Different bacterial toxins may cause toxic shock syndrome, depending on the situation. Said toxins are particularly produced by Gram-positive bacteria such as *Staphylococcus aureus* and *Streptococcus pyogenes* during bacterial infection. TSS is also referred to as Toxic Shock Like Syndrome (TSLS).

The term "virulence" as used in context of the present invention refers to the ability of microbes, such as bacteria, to cause a disease, i.e. to the degree of their pathogenicity. A number of properties of bacteria influence their pathogenicity and virulence, e.g. their ability to adhere to host cells, their ability to form colonies within a host, their ability to invade a host or host cell, their ability to inhibit or evade the immune response of the host, and their ability to produce toxins. In the context of the present invention the term "virulence factor" means intrinsic factors that mediate the virulence of pathogenic bacteria. Such virulence factors are molecules, particularly proteins, produced by the pathogenic bacteria that directly influence the host and allow these bacteria to thrive within a host. Examples of virulence factors include enzymes that inactivate antibiotics, enzymes that disrupt host cells, proteins that influence clot formation, proteins that mediate capsule formation, proteins or protein complexes that mediate attachment to host cells and proteins that mediate evasion of the immune response, e.g. by binding host antibodies. Virulence factors are also involved in biofilm formation.

The compounds of the present invention, including the compounds of formula (I) and the compounds of formula (Ia), are also useful in the inhibition of virulence in protozoa and are thus useful in the treatment or prevention of protozoan infections. Accordingly, protozoan infections, like malaria, plasmodium-related hemoglobinuria, or plasmodium-related diarrhea, may be treated or prevented by the administration of the compounds of formula (I) or (Ia). A protozoan infection to be treated or prevented in accordance with the present invention may be caused by one ore more protozoan species. The term "protozoa" as used herein refers to eukaryotic, unicellular organisms. In particular, the compounds of the invention are envisaged to be useful in the treatment or prevention of infections with: Apicomplexa, such as, e.g., *Plasmodium* spp., particularly *Plasmodium falciparum, Plasmodium malariae, Plasmodium vivax, Plasmodium ovale,* or *Plasmodium knowlesi; Leishmania* spp., particularly *Leishmania major, Leishmania tropica, Leishmania aethiopica, Leishmania mexicana, Leishmania braziliensis, Leishmania donovani, Leishmania infantum,* or *Leishmania chagasi; Trypanosoma* spp., particularly *Trypanosoma brucei, Trypanosoma cruzi, Trypanosoma simiae, Trypanosoma avium, Trypanosoma congolense, Trypanosoma equinum, Trypanosoma equiperdum, Trypanosoma evansi,* or *Trypanosoma suis; Babesia* spp., particularly *Babesia microti,* or *Babesia bigemina;* or *Toxoplasma,* particularly *Toxoplasma gondii.* Hence, said compounds may be useful in the treatment or prevention of infectious diseases caused by and/or related to infections with Apicomplexa (particularly with *Plasmodium* spp., and more particularly with *Plasmodium falciparum, Plasmodium malariae, Plasmodium vivax* or *Plasmodium ovale),* such as, e.g., malaria, plasmodium-related hemoglobinuria, or plasmodium-related diarrhea. Also to be treated, prevented and/or ameliorated in the sense of this invention may be further protozoan infections, like infections with Leishmania, e.g. leishmaniasis (e.g., visceral leishmaniasis, kala-azar, cutaneous leishmaniasis) or infections with Trypanosoma, like sleeping sickness or Chagas disease. Also infections with Toxoplasma (like *Toxoplasma gondii*) or infections with Babesia, e.g. babesiosis, may be treated with the compounds of the invention, particularly the compounds of formula (I) or (Ia).

The compounds of the present invention can also be used for treating or preventing protozoan infections caused by multi-drug resistant protozoa, including, e.g., multi-drug resistant strains of any of the specific protozoa described herein (particularly multidrug-resistant strains of *Plasmodium* spp., such as multidrug-resistant *Plasmodium falciparum).* Accordingly, the present invention relates to a compound of formula (I) or (Ia) or a pharmaceutically acceptable salt, solvate or prodrug thereof, or a pharmaceutical composition comprising any of the aforementioned entities and a pharmaceutically acceptable excipient, for use in the treatment or prevention of a protozoan infection caused by multidrug-resistant protozoa.

It is also envisaged herein that a bacterial infection (or a protozoan infection) which particularly affects animals is to be treated using a compound of the present invention. Non-limiting examples of such bacterial infections are mastitis of dairy cows or laminitis (a disease of digital laminae of feet in horse or cattle). Furthermore, it is also envisaged that non-vertebrates, such as insects, etc. are treated with the compounds of the invention. Accordingly, the compounds of the present invention may also be used in the treatment or prevention of bacterial or protozoan infections of economically relevant insects, like the honey bee. For example, the compounds of the present invention may be used in the treatment or prevention of infections with *Bacillus larvae,* i.e. foul brood or malignant foulbrood of bees.

As described above, the treatment or prevention of diseases which are caused by and/or related to bacterial infections using a compound of the present invention is also envisaged herein. Atopic dermatitis may be considered as an exemplary disease which is caused by and/or related to a bacterial infection. A person skilled in the art will be aware of further diseases caused by and/or related to a bacterial infection which can be treated in accordance with this invention.

It is to be understood that the term "treatment or prevention of a bacterial infection", as used herein, also comprises the treatment or prevention of diseases which are caused by and/or related to bacterial infections, such as, e.g., infections with *Staphylococcus aureus, Staphylococcus epidermidis, Staphylococcus saprophyticus, Staphylococcus lugdunensis, Staphylococcus schleiferi, Staphylococcus caprae, Streptococcus pneumoniae, Streptococcus viridans, Streptococcus pyogenes, Streptococcus agalactiae, Enterococcus faecalis, Enterococcus faecium, Bacillus licheniformis, Bacillus subtilis, Bacillus anthracis, Bacillus cereus, Bacillus thuringiensis, Bacillus larvae, Listeria monocytogenes, Listeria welshimeri, Mycobacterium tuberculosis, Mycobacterium bovis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei, Mycobacterium marinum, Nocardia asteroides, Rhodococcus equi, Neisseria gonorrhoeae, Neisseria meningitidis, Moraxella catarrhalis, Haemophilus influenzae, Klebsiella pneumoniae, Legionella pneumophila, Pseudomonas aeruginosa, Pseudomonas putida, Escherichia coli, Proteus mirabilis, Enterobacter cloaceae, Serratia marcescens, Helicobacter pylori, Salmonella enteritidis,* or *Salmonella typhi,* and in particular with *Staphylococcus aureus* or *Listeria monocytogenes.*

The compounds of the present invention, in particular the compounds of formula (I) and the compounds of formula (Ia), may also be used for cotherapy. For example, one compound of the present invention or two or more compounds of the invention and one or more antibiotics and/or antiseptics may be employed for the treatment or prevention of a bacterial infection. A pharmaceutical composition may comprise the compound(s), antibiotic(s) and/or antiseptic(s). Cotherapy may also include the administration of two or more compounds of the present invention in the absence of further antibiotics or antiseptics. It is also envisaged herein that the compound(s), antibiotic(s) and/or antiseptic(s) might be linked, for example, by formation of conjugates. Accordingly, the compound, antibiotics and/or antiseptics may be administered to a subject simultaneously. Of course, one pharmaceutical composition may comprise only the compound(s) of the invention, while the one or more antibiotics and/or antiseptics are comprised in a second, different pharmaceutical composition. In that case, it may still be possible to administer the inventive compound(s), antibiotics and/or antiseptics simultaneously; however, the compound(s) of the invention may then also be administered before and/or after the one or more antibiotics and/or antiseptics. A person skilled in the art knows how to administer, for example, one or more antibiotics, one or more antiseptics and/or one or more inventive compounds in cotherapy.

It is envisaged that one or more of the compounds according to the invention, particularly the compounds of formula (I) or (Ia) as described herein, may be used in combination with one or more antibiotics and/or one or more antiseptics.

The one or more antibiotics include, for example, tetracycline-derived antibiotics such as, e.g., tetracycline, doxycycline, chlortetracycline, clomocycline, demeclocycline, lymecycline, meclocycline, metacycline, minocycline, oxytetracycline, penimepicycline, rolitetracycline, or tigecycline; amphenicol-derived antiobiotics such as, e.g., chloramphenicol, azidamfenicol, thiamphenicol, or florfenicol; macrolide-derived antiobiotics such as, e.g., erythromycin, azithromycin, spiramycin, midecamycin, oleandomycin, roxithromycin, josamycin, troleandomycin, clarithromycin, miocamycin, rokitamycin, dirithromycin, flurithromycin, telithromycin, cethromycin, tulathromycin, carbomycin A, kitasamycin, midecamicine, midecamicine acetate, tylosin (tylocine), or ketolide-derived antiobiotics such as, e.g., telithromycin, or cethromycin; lincosamide-derived antiobiotics such as, e.g., clindamycin, or lincomycin; streptogramin-derived antiobiotics such as, e.g., pristinamycin, or quinupristin/dalfopristin; oxazolidinone-derived antiobiotics such as, e.g., linezolid, or cycloserine; aminoglycoside-derived antiobiotics such as, e.g., streptomycin, neomycin, framycetin, paromomycin, ribostamycin, kanamycin, amikacin, arbekacin, bekanamycin, dibekacin, tobramycin, spectinomycin, hygromycin B, paromomycin, gentamicin, netilmicin, sisomicin, isepamicin, verdamicin, astromicin, rhodostreptomycin, or apramycin; steroid-derived antiobiotics such as, e.g., fusidic acid, or sodium fusidate; glycopeptide-derived antiobiotics such as, e.g., vancomycin, oritavancin, telavancin, teicoplanin, dalbavancin, ramoplanin, bleomycin, or decaplanin; beta-lactam-derived antiobiotics such as, e.g., amoxicillin, ampicillin, pivampicillin, hetacillin, bacampicillin, metampicillin, talampicillin, epicillin, carbenicillin, carindacillin, ticarcillin, temocillin, azlocillin, piperacillin, mezlocillin, mecillinam, pivmecillinam, sulbenicillin, benzylpenicillin, azidocillin, penamecillin, clometocillin, benzathine benzylpenicillin, procaine benzylpenicillin, phenoxymethylpenicillin, propicillin, benzathine, phenoxymethylpenicillin, pheneticillin, oxacillin, cloxacillin, dicloxacillin, flucloxacillin, meticillin, nafcillin, faropenem, biapenem, doripenem, ertapenem, imipenem, meropenem, panipenem, cefacetrile, cefadroxil, cefalexin, cefaloglycin, cefalonium, cefaloridine, cefalotin, cefapirin, cefatrizine, cefazedone, cefazaflur, cefazolin, cefradine, cefroxadine, ceftezole, cefaclor, cefamandole, cefminox, cefonicid, ceforanide, cefotiam, cefprozil, cefbuperazone, cefuroxime, cefuzonam, cefoxitin, cefotetan, cefmetazole, loracarbef, cefcapene, cefdaloxime, cefdinir, cefditoren, cefetamet, cefixime, cefmenoxime, cefodizime, cefoperazone, cefotaxime, cefpimizole, cefpiramide, cefpodoxime, cefsulodin, ceftazidime, cefteram, ceftibuten, ceftiolene, ceftizoxime, ceftriaxone, flomoxef, latamoxef, cefepime, cefozopran, cefpirome, cefquinome, ceftobiprole, aztreonam, tigemonam, sulbactam, tazobactam, clavulanic acid, ampicillin/sulbactam, sultamicillin, piperacillin/tazobactam, co-amoxiclav, amoxicillin/clavulanic acid, or imipenem/cilastatin; sulfonamide-derived antiobiotics such as, e.g., acetazolamide, benzolamide, bumetanide, celecoxib, chlorthalidone, clopamide, dichlorphenamide, dorzolamide, ethoxzolamide, furosemide, hydrochlorothiazide, indapamide, mafenide, mefruside, metolazone, probenecid, sulfacetamide, sulfadiazine, sulfadimethoxine, sulfadoxine, sulfanilamides, sulfamethoxazole, sulfamethoxypyridazine, sulfasalazine, sultiame, sumatriptan, xipamide, zonisamide, sulfaisodimidine, sulfamethizole, sulfadimidine, sulfapyridine, sulfafurazole, sulfathiazole, sulfathiourea, sulfamoxole, sulfadimethoxine, sulfalene, sulfametomidine, sulfametoxydiazine, sulfaperin, sulfamerazine, sulfaphenazole, or sulfamazone; quinolone-derived antiobiotics such as, e.g., cinoxacin, flumequine, nalidixic acid, oxolinic acid, pipemidic acid, piromidic acid, rosoxacin, ciprofloxacin, enoxacin, fleroxacin, lomefloxacin, nadifloxacin, ofloxacin, norfloxacin, pefloxacin, rufloxacin, balofloxacin, grepafloxacin, levofloxacin, pazufloxacin, sparfloxacin, temafloxacin, tosufloxacin, besifloxacin, clinafloxacin, garenoxacin, gemifloxacin, moxifloxacin, gatifloxacin, sitafloxacin, trovafloxacin, alatrofloxacin, prulifloxacin, danofloxacin, difloxacin, enrofloxacin, ibafloxacin, marbofloxacin, orbifloxacin, pradofloxacin, sarafloxacin, ecinofloxacin, or delafloxacin; imidazole-derived antiobiotics such as, e.g., metronidazole; nitrofuran-derived antiobiotics such as, e.g., nitrofurantoin, or nifurtoinol; aminocoumarin-derived antiobiotics such as, e.g., novobiocin, clorobiocin, or coumermycin A1; ansamycin-derived antiobiotics, including rifamycin-derived antiobiotics such as, e.g., rifampicin (rifampin), rifabutin, rifapentine, or rifaximin; and also further antiobiotics such as, e.g., fosfomycin, bacitracin, colistin, polymyxin B, daptomycin, xibornol, clofoctol, methenamine, mandelic acid, nitroxoline, mupirocin, trimethoprim, brodimoprim, iclaprim, tetroxoprim, or sulfametrole; without being limited thereto.

Furthermore, the one or more antiseptics include, for example, acridine-derived antiseptics such as, e.g., ethacridine lactate, aminoacridine, or euflavine; amidine-derived or biguanide-derived antiseptics such as, e.g., dibrompropamidine, chlorhexidine, propamidine, hexamidine, or polihexanide; phenol-derived antiseptics such as, e.g., phenol, hexachlorophene, policresulen, triclosan, chloroxylenol, or biphenylol; nitrofuran-derived antiseptics such as, e.g., nitrofurazone; iodine-based antiseptics such as, e.g., iodine/octylphenoxypolyglycolether, povidone-iodine, or diiodohydroxypropane; quinoline-derived antiseptics such as, e.g., dequalinium, chlorquinaldol, oxyquinoline, or clioquinol; quaternary ammonium-derived antiseptics such as, e.g., benzalkonium, cetrimonium, cetylpyridinium, cetrimide, benzoxonium chloride, or didecyldimethylammonium chloride; mercurial antiseptics such as, e.g., mercuric amidochloride, phenylmercuric borate, mercuric chloride, mercurochrome, thiomersal, or mercuric iodide; silver-based antiseptics such as, e.g., silver nitrate; alcoholic antiseptics such as, e.g., propanol (including isopropanol), or ethanol; and also further antiseptics such as, e.g., potassium permanganate, sodium hypochlorite, hydrogen peroxide, eosin, tosylchloramide sodium, dichlorobenzyl alcohol, ambazone, benzethonium, myristyl-benzalkonium, hexylresorcinol, or acriflavinium chloride; without being limited thereto.

Cotherapy using the compound(s) of the invention, antibiotic(s) and/or antiseptic(s) may result in a synergistic effect, i.e. the agents acting together may create an effect greater than that predicted by knowing only the separate effects of the individual agents. Such a synergistic effect might be particularly advantageous if less amounts of the compound(s), antibiotic(s) and/or antiseptic(s) may then be used. Thus, possible side-effects of the compound(s), antibiotic(s) and/or antiseptic(s) might be diminished or avoided.

The compounds of the present invention, particularly the compounds of formula (I) or (Ia), are also useful in the cleaning of devices, in particular technical or medical devices, such as catheters, medical implants, stents, contact lenses and the like. Accordingly, the compounds of the invention can also be used in the sterilization of such devices, of laboratory equipment, of benches, of surfaces and the like. Moreover, the compounds of formula (I) or (Ia) are effective and thus useful in preventing the formation of biofilms or eliminating biofilms on surfaces of a technical device.

Accordingly, the present invention provides for a method for sterilizing and/or anti-bacterial cleaning of a technical device or instrument, in particular catheters, medical implants, contact lenses and the like, said method comprising the application of a compound of the present invention (or a composition comprising said compound) on surfaces of said technical device or instrument or on parts thereof. For example, the compound or the composition of the present invention may be applied on surfaces of said technical device or instrument or on parts thereof by spraying, wetting, depositing and/or soaking the technical device or instrument or parts thereof in a composition. All or only some of the various modes of application of the inventive compound or composition as described above may be performed simultaneously or consecutively in arbitrary order. Thereby, the compound or the composition of the present invention may, for example, be applied at concentrations of about 1 mg/L (i.e., 1 mg compound/L) to about 100 mg/L.

In the context of the present invention, the term "biofilm" means a complex aggregation (i.e. community) of microorganisms (e.g. bacteria, fungi, algae and/or protozoae) marked by the excretion of a protective and adhesive matrix. Biofilms are also often characterized by surface attachment, structural heterogeneity, genetic diversity, complex community interactions, and an extracellular matrix of polymeric substances. Exemplary polymeric substances are polysaccharides, proteins, lipids and nucleic acids. The biopolymers of biofilms adhere e.g. to the interface of a liquid and a surface, the interface of a liquid and a gas, or to the interface between two liquids. Biofilms are naturally occurring e.g. in acidic pools, on glaciers, at the bottom of streams or rivers and on teeth (plaque formed mainly by *Streptococcus* species). It is known in the art that many bacteria are capable of forming biofilms either alone ore in combination with other microorganisms.

Biofilms have been found to be induced or to be associated with various bacterial infections, such as, e.g., urinary tract infections, infections in cystic fibrosis, middle-ear infections, formation of dental plaques, gingivitis, caries, endocarditis, catheter infections, contact lens associated eye infections or medical implant associated infections. It is known that, inter alia, Gram-positive bacteria such as *Staphylococcus* species (e.g. *Staphylococcus epidermidis* and *Staphylococcus aureus)* and Gram-negative bacteria (e.g. *Pseudomonas aeruginosa* and *Escherichia coli*) are involved in the formation of undesired and malicious biofilms on medical instruments, catheters, medical implants, contact lenses and the like. Furthermore, biofilms contribute in a major way to the morbidity and mortality associated with endocarditis, infections in cystic fibrosis, and infections associated with medical implants such as joint prostheses and heart valves replacements. A person skilled in the art will know or will be able to identify further bacterial infections which induce the formation of biofilms or which are associated with the formation of biofilms.

In the context of the present invention the term "medical implant" means a medical device which is to replace and to act as a missing biological structure. The term "medical implant" is used herein in the same meaning as "permanent indwelling device". Surfaces of medical implants may be, for example, made of titanium, silicon or apatite. Exemplary medical implants are (subcutenous) drug delivery devices, prosthesis, devices to be placed over or whithin bones to hold a fracture reduction, dental implants, cochlear implants, pacemakers, heart valve replacements and artificial joints (also referred to herein as joint prostheses).

In a preferred embodiment, the present invention relates to the non-therapeutical use of a compound of the invention, particularly a compound of formula (I) or (Ia), or a composition comprising said compound for preventing the formation of biofilms or eliminating biofilms on surfaces of a technical device. In one aspect of this embodiment, said preventing or eliminating of biofilms is performed on devices which are not simultaneously contacting the human or animal body. The non-therapeutical use of the compound or composition of the present invention may be particularly useful for preventing the formation of biofilms or for eliminating biofilms on surfaces of technical devices described herein above which cannot be autoclaved or otherwise sufficiently heat-treated, cleaned or disinfected. The compound or composition of the present invention may be applied on surfaces of technical devices or parts thereof in order to prevent/eliminate biofilms, for example, by spraying, wetting, depositing and/or soaking the technical device or parts thereof in a composition. All or only some of the various modes of application of the inventive compound or composition as described above may be performed simultaneously or consecutively in arbitrary order. Thereby, the compound or the composition of the present invention may, for example, be applied at concentrations of about 1 mg/L (i.e., 1 mg compound/L) to about 100 mg/L.

Provided is also a method for preventing the formation of biofilms or eliminating biofilms on surfaces of a technical device, said method comprising the application of the compound or the composition of the present invention on surfaces of said technical device or on parts thereof. For example, the compound or the composition of the present invention may be applied on surfaces of said technical devices or on parts thereof by spraying, wetting, depositing and/or soaking the technical device or parts thereof in a composition. All or only some of the various modes of application of the inventive compound or composition as described above may be performed simultaneously or consecutively in arbitrary order. Thereby, the compound or the composition of the present invention may, for example, be applied at concentrations of about 1 mg/L (i.e., 1 mg compound/L) to about 100 mg/L.

In the context of the present invention the term "technical device" means any item which is not an alive human or animal. This definition does not preclude that the technical device may contact a living organism, in particular a microorganism. However, it is to be understood that the "technical device" is not within an alive human or animal body. During the procedure of preventing or eliminating biofilms on surfaces of a technical device said device does also not contact an alive human or animal body. Non-limiting examples of technical devices are catheters, surgical instruments, contact lenses, medical implants, and food-processing devices. Medical implants have been described herein above. Also plants or parts thereof (e.g. fruits, vegetables or flowers) or non-living animals or parts thereof may be considered as technical device in the context of the present invention. Such plants, non-living animals or parts thereof may, for example, be used in the production or processing of edibles such as food, beverages and the like.

Biofilms can also occur on surfaces of technical devices such as kerosine or oil tanks, water tanks of ships, wet areas within astronautic vessels, submarines or nuclear power plants and on the surface of ship hulls, which might lead to a significant decrease of the speed of these ships. In addition, biofilms on the surface of e.g. ship hulls can contribute to biofouling. Biofilms are also unwanted on the surface of technical devices (e.g. vessels, instruments, tanks, tubings and other equipment) used in food (e.g. dairy products) and beverage (e.g. beer) production.

By targeting CIpP protease or by inhibition of adhesion factors, it is believed that the compounds of the present invention inhibit the activity of bacteria which form part of a biofilm as described herein above. As biofilms are considered to be an aggregation of various microorganisms (inter alia, bacteria, fungi or algae), the inhibition of bacterial activity may contribute to the prevention or elimination of biofilms.

In the context of biofilm prevention and elimination on the surface of technical devices and, accordingly, in the method for sterilizing and/or anti-bacterial cleaning of a technical device or instrument and also in the method for preventing the formation of biofilms or eliminating biofilms on surfaces of a technical device, the compound or composition of the present invention can be applied either alone or in combination with further means and methods, like heat-treatment, UV-irradiation or disinfection, or in combination with antibiotics. It is preferred herein that said further means and methods prevent the formation of biofilms or eliminate biofilms on the surface of technical devices. It is also envisaged that these methods may destroy biofilms, for example, by mechanical scrubbing or simply cleaning the surface of a technical device, for example, using a composition comprising one or more detergents as described herein above. These further means and methods may be applied simultaneously, after and/or before the inventive compound or composition is applied in accordance with the present invention. A person skilled in the art knows how to apply the further means and methods in order to destroy biofilms.

In particular, heat-labile technical devices may not be heat-treated, e.g. autoclaved. The term "heat-labile technical devices" used in the context of the present invention means technical devices which cannot be heat-treated for disinfection, e.g. by autoclaving, or which are destroyed or deformed at temperatures typical for heat-treatment, e.g. temperatures from about 45 °C to 150 °C. The compound or the composition of the present invention may also be useful in the case that heat-treatment, disinfection and the like might destroy or damage the surfaces of technical devices such as catheters or medical implants. The compound or composition may also be used in food or beverage processing/production, in particular when edibles cannot be (sufficiently) sterilised by means and methods known in the art. For example, such sterilisation might destroy the structure, taste, look or colour of the food or beverage and accordingly is to be avoided or has to be carried out in a way that does not sufficiently remove/inactivate bacteria, in particular pathogenic bacteria.

In the context of the present invention, the term "disinfection" means the destruction of microorganisms such as bacteria. "Disinfectants" are usually applied to non-living objects, while antibiotics generally destroy microorganisms within the body and antiseptics destroy microorganisms on living tissue. Often disinfectants do not sterilise the technical devices completely (i.e. the complete elimination of all microorganisms/bacteria) but merely reduce the number of living microorganisms per area disinfected. Non-limiting examples of disinfectants are aldehydes (e.g. glutaraldehyde), alcohols (such as ethanol, isopropanol and the like), and halogens. Non-limiting examples of halogens are chloramine, chloramine-T, chlorine, hypochlorites and iodine. Also oxidizing agents (e.g. chlorine dioxide, hydrogen peroxide, ozone, acidic electrolyzed water, peracetic acid, and potassium permanganate), phenolics (e.g. phenol, O-phenylphenol, thymol and the like), quaternary ammonium compounds and polyaminopropyl biguanide may be considered as disinfectants. Exemplary antiseptics are alcohols, quaternary ammonium compounds, boric acid, chlorhexidine gluconate, hydrogen peroxide, iodine, mercurochrome, phenol, and sodium chloride.

The compound of the present invention may be particularly useful when the disinfectant employed may not be capable of sterilising the technical device. In a preferred embodiment, the means and methods described herein above (such as heat-treatment, UV irradiation, mechanical scrubbing or disinfection) are not capable of eliminating all bacterial. For example, less than 100 %, more preferably less than 99 %, 98 %, 97 %, 96 %, 95 % or 90 % of the bacteria are eliminated.

It is also envisaged herein that the compound or composition of the present invention may not only be used for preventing the formation of biofilms or eliminating biofilms on surfaces of technical devices but may also be used non-therapeutically for inhibiting the activity of bacteria which do not form part of a biofilm. The compound or composition may, for example, be used as an antibacterial agent and be employed for inhibiting the activity of bacteria (in particular Gram-positive bacteria) which are present on surfaces of technical devices as described herein above. For example, *Listeria* (e.g. *Listeria monocytogenes)* contaminations in food, particularly in meat and dairy products and in fruits and vegetables, like cheese, can cause infections when incorporated by a subject.

The scope of the invention embraces all pharmaceutically acceptable salt forms of the compounds of formula (I) or (Ia) which may be formed, e.g., by protonation of an atom carrying an electron lone pair which is susceptible to protonation, such as an amino group, with an inorganic or organic acid, or as a salt of a carboxylic acid group with a physiologically acceptable cation as they are well-known in the art. Exemplary base addition salts comprise, for example, alkali metal salts such as sodium or potassium salts; alkaline earth metal salts such as calcium or magnesium salts; ammonium salts; aliphatic amine salts such as trimethylamine, triethylamine, dicyclohexylamine, ethanolamine, diethanolamine, triethanolamine, procaine salts, meglumine salts, diethanol amine salts or ethylenediamine salts; aralkyl amine salts such as N,N-dibenzylethylenediamine salts, benetamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts or lysine salts. Exemplary acid addition salts comprise, for example, mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

Moreover, the scope of the invention embraces solid forms of the compounds of formula (I) or (Ia) in any solvated form, including e.g. solvates with water, for example hydrates, or with organic solvents such as, e.g., methanol, ethanol or acetonitrile, i.e. as a methanolate, ethanolate or acetonitrilate, respectively; or in the form of any polymorph.

Furthermore, the formulas in the present specification are intended to cover all possible stereoisomers, including enantiomers and diastereomers, of the indicated compounds.

Thus, all stereoisomers of the compounds of the present invention are contemplated as part of the present invention, either in admixture or in pure or substantially pure form. The scope of the compounds according to the invention embraces all the possible stereoisomers and their mixtures. It particularly embraces the racemic forms and the isolated optical isomers. The racemic forms can be resolved by physical methods, such as, e.g., fractional crystallization, separation or crystallization of diastereomeric derivatives or separation by chiral column chromatography. The individual optical isomers can be obtained from the racemates using conventional methods, such as, e.g., salt formation with an optically active acid followed by crystallization.

Pharmaceutically acceptable prodrugs of the compounds according to the present invention are derivatives which have chemically or metabolically cleavable groups and become, by solvolysis or under physiological conditions, the compounds of formula (I) or (Ia) which are pharmaceutically active in vivo. Prodrugs of compounds of the present invention may be formed in a conventional manner with a functional group of the compounds such as with an amino, hydroxy or carboxy group. The prodrug derivative form often offers advantages of solubility, tissue compatibility or delayed release in a mammalian organism (see, Bundgaard, H., Design of Prodrugs, pp. 7-9, 21-24, Elsevier, Amsterdam 1985). Prodrugs include acid derivatives well known to the person skilled in the art, such as, for example, esters prepared by reaction of the parent acidic compound with a suitable alcohol, or amides prepared by reaction of the parent acid compound with a suitable amine. When a compound employed in the present invention has a carboxyl group, an ester derivative prepared by reacting the carboxyl group with a suitable alcohol or an amide derivative prepared by reacting the carboxyl group with a suitable amine is exemplified as a prodrug. An especially preferred ester derivative as a prodrug is methylester, ethylester, n-propylester, isopropylester, n-butylester, isobutylester, tert-butylester, morpholinoethylester, N,N-diethylglycolamidoester or α-acetoxyethylester. When a compound employed in the present invention has a hydroxy group, an acyloxy derivative prepared by reacting the hydroxyl group with a suitable acylhalide or a suitable acid anhydride is exemplified as a prodrug. An especially preferred acyloxy derivative as a prodrug is -OC(=O)-CH₃, -OC(=O)-C₂H₅, -OC(=O)-(tert-Bu), -OC(=O)-C₁₅H₃₁, -OC(=O)-(m-COONa-Ph), -OC(=O)-CH₂CH₂COONa, -O(C=O)-CH(NH₂)CH₃ or -OC(=O)-CH₂-N(CH₃)₂. When a compound employed in the present invention has an amino group, an amide derivative prepared by reacting the amino group with a suitable acid halide or a suitable mixed anhydride is exemplified as a prodrug. An especially preferred amide derivative as a prodrug is -NHC(=O)-(CH₂)₂OCH₃ or -NHC(=O)-CH(NH₂)CH₃.

The compounds described herein may be administered as compounds per se in their use as pharmacophores or pharmaceutical compositions or may be formulated as medicaments. Within the scope of the present invention are pharmaceutical compositions comprising as an active ingredient a compound of formula (I) or (Ia) as described and defined herein. The pharmaceutical compositions may optionally comprise one or more pharmaceutically acceptable excipients, such as carriers, diluents, fillers, disintegrants, lubricating agents, binders, colorants, pigments, stabilizers, preservatives, or antioxidants.

The pharmaceutical compositions may also comprise one or more solubility enhancers, such as, e.g., poly(ethylene glycol), including poly(ethylene glycol) having a molecular weight in the range of about 200 to about 5,000 Da, ethylene glycol, propylene glycol, non-ionic surfactants, tyloxapol, polysorbate 80, macrogol-15-hydroxystearate, phospholipids, lecithin, dimyristoyl phosphatidylcholine, dipalmitoyl phosphatidylcholine, distearoyl phosphatidylcholine, cyclodextrins, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, carboxyalkyl thioethers, hydroxypropyl methylcellulose, hydroxypropylcellulose, polyvinylpyrrolidone, vinyl acetate copolymers, vinyl pyrrolidone, sodium lauryl sulfate, dioctyl sodium sulfosuccinate, or any combination thereof.

The pharmaceutical compositions can be formulated by techniques known to the person skilled in the art, such as the techniques published in Remington's Pharmaceutical Sciences, 20^{th} Edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds according to the invention, in particular the compounds of formula (I) or (Ia), or the above described pharmaceutical compositions comprising one ore more compounds of formula (I) or (Ia) may be administered to a subject by any convenient route of administration, whether systemically/peripherally or at the site of desired action, including but not limited to one or more of: oral (e.g. as a tablet, capsule, or as an ingestible solution), topical (e.g., transdermal, intranasal, ocular, buccal, and sublingual), parenteral (e. g., using injection techniques or infusion techniques, and including, for example, by injection, e.g. subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g. through mouth or nose), gastrointestinal, intrauterine, intraocular, subcutaneous, ophthalmic (including intravitreal or intracameral), rectal, and vaginal.

If said compounds or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

Alternatively, said compounds or pharmaceutical compositions can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

Said compounds or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides (U.S. Pat. No. 3,773,919), copolymers of L-glutamic acid and gamma-ethyl-L-glutamate (Sidman, U. et al., Biopolymers 22:547-556 (1983)), poly(2-hydroxyethyl methacrylate) (R. Langer et al., J. Biomed. Mater. Res. 15:167-277 (1981), and R. Langer, Chem. Tech. 12:98-105 (1982)), ethylene vinyl acetate (R. Langer et al., Id.) or poly-D-(-)-3-hydroxybutyric acid (EP133988). Sustained-release pharmaceutical compositions also include liposomally entrapped compounds. Liposomes containing a compound of the present invention can be prepared by methods known in the art, such as, e.g., the methods described in any one of: DE3218121; Epstein et al., Proc. Natl. Acad. Sci. (USA) 82:3688-3692 (1985); Hwang et al., Proc. Natl. Acad. Sci. (USA) 77:4030-4034 (1980); EP0052322; EP0036676; EP088046; EP0143949; EP0142641; Japanese Pat. Appl. 83-118008; U.S. Pat. Nos. 4,485,045 and 4,544,545; and EP0102324.

Said compounds or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

It is also envisaged to prepare dry powder formulations of the compounds of the present invention, including the compounds of formula (I) or (Ia), for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to the emulsification/spray drying process disclosed in WO 99/16419 or WO 01/85136. Spray drying of solution formulations of the compounds of the present invention is carried out, for example, as described generally in the "Spray Drying Handbook", 5th ed., K. Masters, John Wiley & Sons, Inc., NY, NY (1991), and in WO 97/41833 or WO 03/053411.

For topical application to the skin, said compounds or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A person skilled in the art knows that the effective amount of a pharmaceutical composition administered to an individual will, inter alia, depend on the nature of the compound according to the invention. For example, the total pharmaceutically effective amount of a pharmaceutical composition administered parenterally per dose will be in the range of about 1 µg compound/kg/day to 500 mg compound/kg/day, in extreme cases up to 4.0 g compound/kg/day of patient body weight, although, as noted above, this will be subject to therapeutic discretion. Typically, the total pharmaceutically effective amount of a pharmaceutical composition administered parenterally per dose will be in the range of about 1 µg compound/kg/day to 100 mg compound/kg/day of patient body weight. More preferably, this dose is at least 0.01 mg compound/kg/day, and most preferably for humans between about 0.01 and 50 mg compound/kg/day. If given continuously, the pharmaceutical composition is typically administered at a dose rate of about 1 µg/kg/hour to about 10 mg/kg/hour; more preferably, at a a dose rate of about 50 µg/kg/hour to about 3 mg/kg/hour; either by 1-4 injections per day or by continuous subcutaneous infusions, for example, using a mini-pump. An intravenous bag solution may also be employed. The length of treatment needed to observe changes and the interval following treatment for responses to occur may vary depending on the desired effect. The particular amounts may be determined by conventional tests which are well known to the person skilled in the art.

The subject or patient, such as the subject in need of treatment or prevention, may be a eukaryote, an animal, a vertebrate animal, a mammal, a rodent (e.g. a guinea pig, a hamster, a rat, a mouse), a murine (e.g. a mouse), a canine (e.g. a dog), a feline (e.g. a cat), an equine (e.g. a horse), a primate, a simian (e.g. a monkey or ape), a monkey (e.g. a marmoset, a baboon), an ape (e.g. gorilla, chimpanzee, orang-utan, gibbon), or a human. The meaning of the terms "eukaryote", "animal", "mammal", etc. is well known in the art and can, for example, be deduced from Wehner und Gehring (1995; Thieme Verlag). In the context of this invention, it is particularly envisaged that animals are to be treated which are economically, agronomically or scientifically important. Scientifically important organisms include, but are not limited to, mice, rats, and rabbits. Non-limiting examples of agronomically important animals are sheep, cattle and pig, while, for example, cats and dogs may be considered as economically important animals. Preferably, the subject/patient is a mammal; more preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orang-utan, a gibbon, a sheep, cattle, or a pig); even more preferably, the subject/patient is a human.

The term "treatment of a disorder or disease" as used herein is well known in the art. "Treatment of a disorder or disease" implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

"Treatment of a disorder or disease" may, for example, lead to a halt in the progression of the disorder or disease (e.g., no deterioration of symptoms) or a delay in the progression of the disorder or disease (in case the halt in progression is of a transient nature only). "Treatment of a disorder or disease" may also lead to a partial response (e.g., amelioration of symptoms) or complete response (e.g., disappearance of symptoms) of the subject/patient suffering from the disorder or disease. "Amelioration" of a disorder or disease may, for example, lead to a halt in the progression of the disorder or disease or a delay in the progression of the disorder or disease. Such a partial or complete response may be followed by a relapse. It is to be understood that a subject/patient may experience a broad range of responses to a treatment (e.g., the exemplary responses as described herein above).

Treatment of a disorder or disease may, inter alia, comprise curative treatment (preferably leading to a complete response and eventually to healing of the disorder or disease) and palliative treatment (including symptomatic relief). In accordance with the present invention, the treatment of bacterial or protozoan infections also includes the prevention of such infections.

Also the term "prevention of a disorder or disease" as used herein is well known in the art. For example, a patient/subject suspected of being prone to suffer from a disorder or disease as defined herein may, in particular, benefit from a prevention of the disorder or disease. The subject/patient may have a susceptibility or predisposition for a disorder or disease, including but not limited to hereditary predisposition. Such a predisposition can be determined by standard assays, using, for example, genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of compounds of the present invention before any clinical and/or pathological symptoms are diagnosed or determined or can be diagnosed or determined by the attending physician.

As used herein, the term "alkyl" refers to a monovalent saturated aliphatic (i.e., nonaromatic) acyclic hydrocarbon group (i.e., a group consisting of carbon atoms and hydrogen atoms) which may be linear or branched and does not comprise any carbon-to-carbon double bond or any carbon-to-carbon triple bond. A "C₁₋₆ alkyl" denotes an alkyl group having 1 to 6 carbon atoms.

As used herein, the term "alkenyl" refers to a monovalent unsaturated aliphatic acyclic hydrocarbon group which may be linear or branched and comprises at least one carbon-to-carbon double bond while it does not comprise any carbon-to-carbon triple bond.

As used herein, the term "alkynyl" refers to a monovalent unsaturated aliphatic acyclic hydrocarbon group which may be linear or branched and comprises at least one carbon-to-carbon triple bond and optionally one or more carbon-to-carbon double bonds.

As used herein, the term "alkylene" refers to a divalent saturated aliphatic acyclic hydrocarbon group which may be linear or branched and does not comprise any carbon-to-carbon double bond or any carbon-to-carbon triple bond.

As used herein, the term "alkenylene" refers to a divalent unsaturated aliphatic acyclic hydrocarbon group which may be linear or branched and comprises at least one carbon-to-carbon double bond while it does not comprise any carbon-to-carbon triple bond.

As used herein, the term "alkynylene" refers to a divalent unsaturated aliphatic acyclic hydrocarbon group which may be linear or branched and comprises at least one carbon-to-carbon triple bond and optionally one or more carbon-to-carbon double bonds.

As used herein, the term "aryl" refers to a monovalent aromatic hydrocarbon group, including bridged ring and/or fused ring systems, containing at least one aromatic ring. "Aryl" may, for example, refer to phenyl, naphthyl or anthracenyl.

As used herein, the term "heteroaryl" refers to monovalent aromatic ring group, including bridged ring and/or fused ring systems, containing at least one aromatic ring and comprising one or more (such as, e.g., one, two, or three) ring heteroatoms independently selected from O, S, or N. The "heteroaryl" may, e.g., have 5 to 14 ring atoms, particularly 5 or 6 ring atoms. "Heteroaryl" may, for example, refer to thiophenyl (thienyl), furanyl (furyl), pyrrolyl, imidazolyl, pyrazolyl, pyridinyl (pyridyl; including, e.g., 2-pyridyl, 3-pyridyl, and 4-pyridyl), pyrazinyl, pyrimidinyl, pyridazinyl, oxazolyl, isoxazolyl, or furazanyl.

As used herein, the term "halogen" refers to -F, -Cl, -Br or -I, and particularly to -F, -Cl or -Br.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The present invention relates, in particular, to the following items:
1. A compound of formula (I) 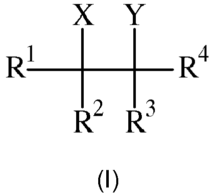 wherein:
   X is selected from -CO-NR^{x1}R^{x2}, -COOH, -CH(-R^{x3})-OH, -CO-O(C₁₋₆ alkyl), -CO-O(C₂₋₆ alkenyl), -CO-O(C₂₋₆ alkynyl), -CO-O(aryl), -CO-O(heteroaryl), -CO-SH, -CO-S(C₁₋₆ alkyl), -CO-S(C₂₋₆ alkenyl), -CO-S(C₂₋₆ alkynyl), -CO-S(aryl), -CO-S(heteroaryl), -CH(-R^{x3})-O(C₁₋₆ alkyl), -CH(-R^{x3})-O(C₂₋₆ alkenyl), -CH(-R^{x3})-O(C₂₋₆ alkynyl), -CH(-R^{x3})-O(aryl), -CH(-R^{x3})-O(heteroaryl), -CH(-R^{x3})-SH, -CH(-R^{x3})-S(C₁₋₆ alkyl), -CH(-R^{x3})-S(C₂₋₆ alkenyl), -CH(-R^{x3})-S(C₂₋₆ alkynyl), -CH(-R^{x3})-S(aryl), -CH(-R^{x3})-S(heteroaryl), or -CH(-R^{x3})-NR^{x1}R^{x2};
   Y is selected from -OH, -O-CO-(C₁₋₆ alkyl), -O(C₁₋₆ alkyl), -O(C₂₋₆ alkenyl), -O(C₂₋₆ alkynyl), -O-CO-(C₂₋₆ alkenyl), -O-CO-(C₂₋₆ alkynyl), -O(aryl), -O(heteroaryl), -SH, -S(C₁₋₆ alkyl), -S(C₂₋₆ alkenyl), -S(C₂₋₆ alkynyl), -S-CO-(C-₁₋₆ alkyl), -S-CO-(C₂₋₆ alkenyl), -S-CO-(C₂₋₆ alkynyl), -S(aryl), -S(heteroaryl), or -N R^{y1}R^{y2};
   R¹ and R³ are each independently selected from C₂₋₁₂ alkenyl, -(C₁₋₁₂alkylene)-aryl, C₁₋₁₂ alkyl, C₂₋₁₂ alkynyl, aryl, heteroaryl, -(C₂₋₁₂ alkenylene)-aryl, -(C₂₋₁₂ alkynylene)-aryl, -(C₁₋₁₂ alkylene)-heteroaryl, -(C₂₋₁₂ alkenylene)-heteroaryl, -(C₂₋₁₂ alkynylene)-heteroaryl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, wherein said C₁₋₁₂ alkyl, said C₂₋₁₂ alkenyl, said C₂₋₁₂ alkynyl, said aryl, said heteroaryl, said -(C₁₋₁₂ alkylene)-aryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, said -(C₂₋₁₂ alkynylene)-heteroaryl, said polyethylene glycol moiety, or said C₃₋₁₂ alkyl is optionally substituted with one or more groups independently selected from halogen, -CN, -CF₃,-CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, heteroaryl, polyethylene glycol moiety, alkylene-polyethylene glycol moiety or polyethylene glycol-alkyl moiety;
   R² and R⁴ are each independently selected from -H, C₁₋₆ alkyl, C₂₋₆ alkenyl, C₂₋₆ alkynyl, -O-(C₁₋₆ alkyl), -S-(C₁₋₆ alkyl), -NH₂, -NH(C₁₋₆ alkyl), -N(C₁₋₆ alkyl)(C₁₋₆ alkyl), aryl, heteroaryl, halogen, -CN, or -CF₃, wherein said C₁₋₆ alkyl, said C₂₋₆ alkenyl, said C₂₋₆ alkynyl, the alkyl moiety of said -O-(C₁₋₆ alkyl), the alkyl moiety of said -S-(C₁₋₆ alkyl), the alkyl moiety of said - NH(C₁₋₆ alkyl), one or both of the alkyl moieties of said -N(C₁₋₆ alkyl)(C₁₋₆ alkyl), said aryl or said heteroaryl is optionally substituted with one or more groups independently selected from halogen, -CN, -CF₃, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl;
   R^{x1} and R^{x2} are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), -O-(C₂₋₆ alkynyl), aryl, heteroaryl, -(C₁₋₆ alkylene)-aryl, -(C₂₋₆ alkenylene)-aryl, -(C₂₋₆ alkynylene)-aryl, -(C₁₋₆ alkylene)-heteroaryl, -(C₂₋₆ alkenylene)-heteroaryl, -(C₂₋₆ alkynylene)-heteroaryl, -O-(C₁₋₆ alkylene)-aryl, -O-(C₂₋₆ alkenylene)-aryl, -O-(C₂₋₆ alkynylene)-aryl, -O-(C₁₋₆ alkylene)-heteroaryl, -O-(C₂₋₆ alkenylene)-heteroaryl, -O-(C₂₋₆ alkynylene)-heteroaryl, -CO-(C₁₋₆ alkylene)-aryl, -CO-(C₂₋₆ alkenylene)-aryl, -CO-(C₂₋₆ alkynylene)-aryl, -CO-(C₁₋₆ alkylene)-heteroaryl, -CO-(C₂₋₆ alkenylene)-heteroaryl, or -CO-(C₂₋₆ alkynylene)-heteroaryl;
   R^{x3} is selected from -H, C₁₋₆ alkyl, C₂₋₆ alkenyl, C₂₋₆ alkynyl, -O-(C₁₋₆ alkyl), -S-(C₁₋₆ alkyl), -NH₂, -NH(C₁₋₆ alkyl), -N(C₁₋₆ alkyl)(C₁₋₆ alkyl), aryl, heteroaryl, halogen, -CN, or -CF₃, wherein said C₁₋₆ alkyl, said C₂₋₆ alkenyl, said C₂₋₆ alkynyl, the alkyl moiety of said -O-(C₁₋₆ alkyl), the alkyl moiety of said -S-(C₁₋₆ alkyl), the alkyl moiety of said -NH(C₁₋₆ alkyl), one or both of the alkyl moieties of said -N(C₁₋₆ alkyl)(C₁₋₆ alkyl), said aryl or said heteroaryl is optionally substituted with one or more groups independently selected from halogen, -CN, -CF₃, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), aryl, or heteroaryl; and
   R^{y1} and R^{y2} are each independently selected from -H, C₁₋₆ alkyl, C₂₋₆ alkenyl, C₂₋₆ alkynyl, -CO-(C₁₋₆ alkyl), -CO-(C₂₋₆ alkenyl), -CO-(C₂₋₆ alkynyl), -O-(C₁₋₆ alkyl), -O-(C₂₋₆ alkenyl), -O-(C₂₋₆ alkynyl), aryl, heteroaryl, -(C₁₋₆ alkylene)-aryl, -(C₂₋₆ alkenylene)-aryl, -(C₂₋₆ alkynylene)-aryl, -(C₁₋₆ alkylene)-heteroaryl, -(C₂₋₆ alkenylene)-heteroaryl, -(C₂₋₆ alkynylene)-heteroaryl, -O-(C₁₋₆ alkylene)-aryl, -O-(C₂₋₆ alkenylene)-aryl, -O-(C₂₋₆ alkynylene)-aryl, -O-(C₁₋₆ alkylene)-heteroaryl, -O-(C₂₋₆ alkenylene)-heteroaryl, -O-(C₂₋₆ alkynylene)-heteroaryl, -CO-(C₁₋₆ alkylene)-aryl, -CO-(C₂₋₆ alkenylene)-aryl, -CO-(C₂₋₆ alkynylene)-aryl, -CO-(C₁₋₆ alkylene)-heteroaryl, -CO-(C₂₋₆ alkenylene)-heteroaryl, or -CO-(C₂₋₆ alkynylene)-heteroaryl;
   or a pharmaceutically acceptable salt, solvate or prodrug thereof.
2. The compound of item 1, wherein X is selected from -CO-NH₂, -COOH, -CH₂-OH, -CO-O(C₁₋₄ alkyl), -CO-SH, -CO-S(C₁₋₄ alkyl), -CO-NH(C₁₋₄ alkyl), -CO-N(C₁₋₄ alkyl)(C₁₋₄ alkyl), -CH₂-SH, -CH₂-NH₂, -CH₂-NH(C₁₋₄ alkyl), or -CH₂-N(C₁₋₄ alkyl)(C₁₋₄ alkyl).
3. The compound of item 1 or 2, wherein X is selected from -CO-NH₂, -COOH, -CH₂-OH, -CO-SH, -CH₂-SH, or-CH₂-NH₂.
4. The compound of any of items 1 to 3, wherein Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl).
5. The compound of any of items 1 to 4, wherein Y is -OH or -O-CO-(C₁₋₆ alkyl).
6. The compound of any of items 1 to 5, wherein one of R¹ and R³ is C₃₋₁₂ alkenyl, C₃₋₁₂ alkyl, C₃₋₁₂ alkynyl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, and the other one of R¹ and R³ is -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl) polyethylene glycol moiety, alkylene-polyethylene glycol moiety or polyethylene glycol-alkyl moiety,
   or
   wherein R¹ and R³ are each independently selected from C₃₋₁₂ alkenyl, C₃₋₁₂ alkyl, C₃₋₁₂ alkynyl, a polyethylene glycol moiety, C₃₋₁₂ alkyl in which at least one and up to one third of the C atoms are in each case substituted by an atom or a moiety independently selected from -O-, -S-, -NH-, -SO-, or -SO₂-, -(C₁₋₆ alkylene)-phenyl, -(C₂₋₆ alkenylene)-phenyl, -(C₂₋₆ alkynylene)-phenyl or phenyl, wherein at least one of R¹ and R³ is said -(C₁₋₆ alkylene)-phenyl, said -(C₂₋₆ alkenylene)-phenyl, said -(C₂₋₆ alkynylene)-phenyl or said phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety.
7. The compound of any of items 1 to 6, wherein one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl.
8. The compound of any of items 1 to 7, wherein at least one of R¹ and R³ comprises at least 4 consecutively arranged C-atoms that are covalently linked together.
9. The compound of any of items 1 to 7 for use as a medicament, wherein R¹ is - CH₂CH₂OCH₂CH₂OCH₃.
10. The compound of any of items 1 to 9, wherein R² and R⁴ are each -H.
11. The compound of item 1, wherein:
   X is selected from -CO-NH₂, -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl) or -CH₂-N(C₁₋₄ alkyl)(C₁₋₄ alkyl);
   Y is selected from -OH, -O-CO-(C₁₋₆ alkyl), -NH₂, or -NH-CO-(C₁₋₆ alkyl);
   R¹ and R³ are each independently selected from C₃₋₁₂ alkenyl, -(C₁₋₆ alkylene)-phenyl, C₃₋₁₂ alkyl, C₃₋₁₂ alkynyl, phenyl, -(C₂₋₆ alkenylene)-phenyl, or -(C₂₋₆ alkynylene)-phenyl,
      or
      R¹ and R³ are each independently selected from C₃₋₁₂ alkenyl, C₃₋₁₂ alkyl, C₃₋₁₂ alkynyl, a polyethylene glycol moiety, C₃₋₁₂ alkyl in which at least one and up to one third of the C atoms are in each case substituted by an atom or a moiety independently selected from -O-, -S-, -NH-, -SO-, or -SO₂-, -(C₁₋₆ alkylene)-phenyl, -(C₂₋₆ alkenylene)-phenyl, -(C₂₋₆ alkynylene)-phenyl or phenyl, wherein at least one of R¹ and R³ is said -(C₁₋₆ alkylene)-phenyl, said -(C₂₋₆ alkenylene)-phenyl, said -(C₂₋₆ alkynylene)-phenyl or said phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl) polyethylene glycol moiety, alkylene-polyethylene glycol moiety or polyethylene glycol-alkyl moiety; and
   R² and R⁴ are each -H.
12. The compound of item 1, wherein said compound is a compound of one of the following formulae, wherein Me is methyl: 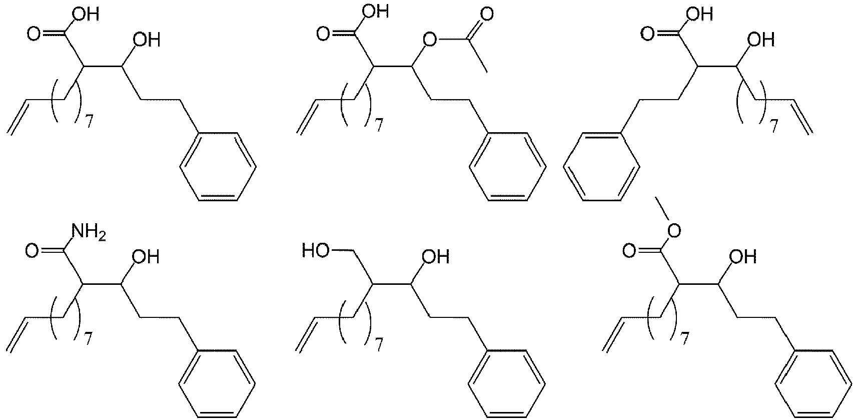 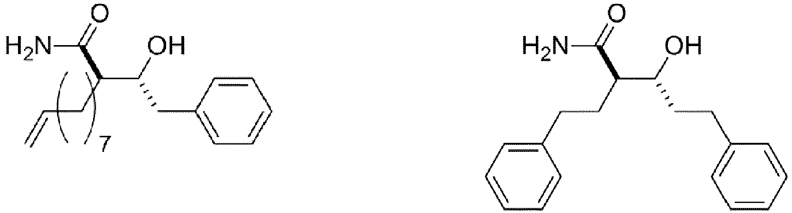 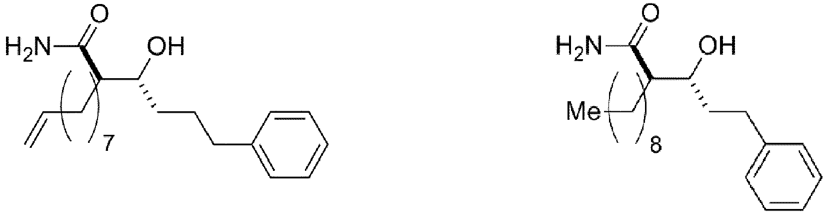 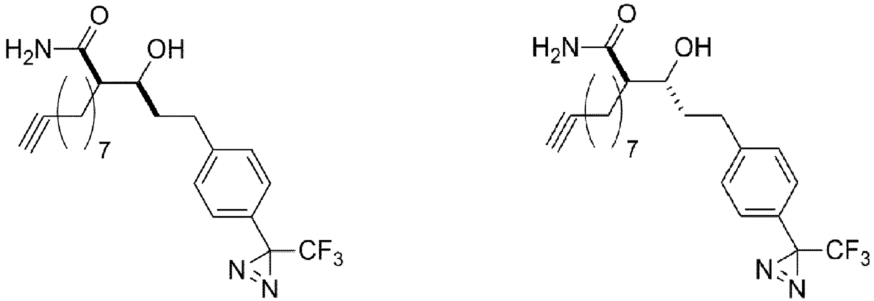 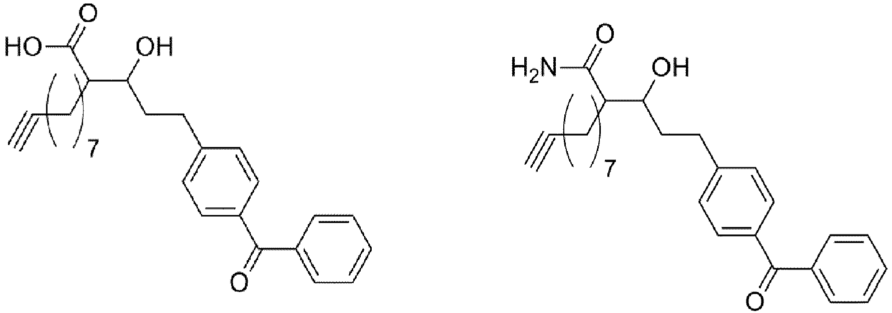 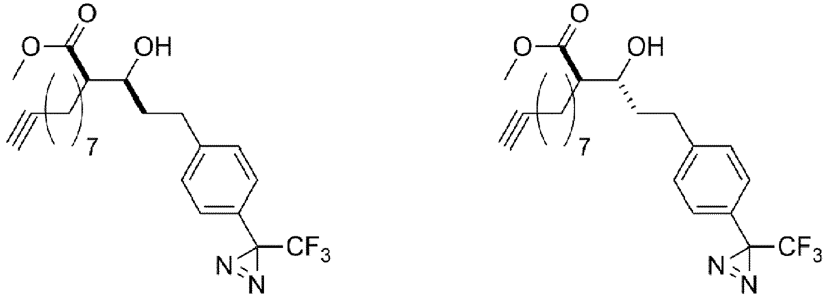 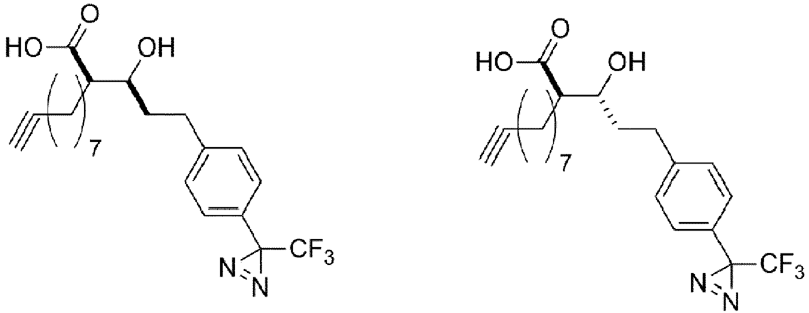 or a pharmaceutically acceptable salt, solvate or prodrug thereof.
13. The compound of item 1, wherein said compound is a compound of the following formula: 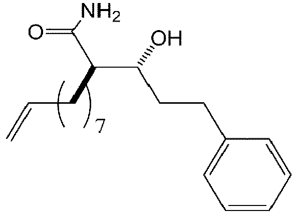 or a pharmaceutically acceptable salt, solvate or prodrug thereof.
14. A pharmaceutical composition comprising the compound of any of items 1 to 13 and a pharmaceutically acceptable excipient.
15. The compound of any of items 1 to 13 or the pharmaceutical composition of item 12 for use as a medicament.
16. A method of treating or preventing a disease or disorder, the method comprising the administration of the compound of any of items 1 to 13 or the pharmaceutical composition of item 14 to a subject in need of such treatment or prevention.
17. The compound of any of items 1 to 13 or the pharmaceutical composition of item 14 for use in the treatment or prevention of a bacterial or protozoan infection.
18. A method of treating or preventing a bacterial or protozoan infection, the method comprising the administration of the compound of any of items 1 to 13 or the pharmaceutical composition of item 14 to a subject in need of such treatment or prevention.
19. The compound of item 17 or the pharmaceutical composition of item 17 or the method of item 18, wherein said bacterial infection is selected from listeriosis, cranial nerve palsy, encephalitis, meningitis, meningoencephalitis, abscesses, endocarditis, pneumonia, cholera, syphilis, anthrax, leprosy, bubonic plague, sepsis, septic arthritis, osteitis, inflammation of wounds, inflammation of organs, furuncles, carbuncles, toxic shock syndrome, Staphylococcal scalded skin syndrome (SSSS), mastitis, atopic dermatitis, or colitis.
20. The compound of item 17 or 19 or the pharmaceutical composition of item 17 or 19 or the method of item 18 or 19, wherein said bacterial infection is caused by Gram-positive bacteria.
21. The compound of item 20 or the pharmaceutical composition of item 20 or the method of item 20, wherein said Gram-positive bacteria are Firmicutes or Actinobacteria.
22. The compound of item 21 or the pharmaceutical composition of item 21 or the method of item 21, wherein said Firmicutes are selected from the group consisting of: Listeria spp., in particular *Listeria monocytogenes,* and *Listeria welshimeri;* Staphylococcus spp., in particular *Staphylococcus aureus, Staphylococcus epidermidis, Staphylococcus saprophyticus, Staphylococcus lugdunensis, Staphylococcus schleiferi,* and *Staphylococcus caprae*; Streptococcus spp., in particular *Streptococcus pneumoniae, Streptococcus viridans, Streptococcus pyogenes,* and *Streptococcus agalactiae;* Enterococcus spp., in particular *Enterococcus faecalis,* and *Enterococcus faecium;* and Bacillus spp., in particular *Bacillus licheniformis, Bacillus subtilis, Bacillus anthracis, Bacillus cereus, Bacillus thuringiensis,* and Bacillus larvae.
23. The compound of any of items 17 or 19 to 22 or the pharmaceutical composition of any of items 17 or 19 to 22 or the method of any of items 18 to 22, wherein said bacterial infection is caused by multidrug-resistant *Staphylococcus aureus* (MRSA).
24. The compound of item 21 or the pharmaceutical composition of item 21 or the method of item 21, wherein said Actinobacteria are selected from the group consisting of: Mycobacterium spp., in particular *Mycobacterium tuberculosis, Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum, Mycobacterium chelonei,* and *Mycobacterium marinum; Nocardia asteroids;* and Rhodococcus equi.
25. The compound of item 17 or 19 or the pharmaceutical composition of item 17 or 19 or the method of item 18 or 19, wherein said bacterial infection is caused by Gram-negative bacteria.
26. The compound of item 25 or the pharmaceutical composition of item 25 or the method of item 25, wherein said Gram-negative bacteria belong to the group Proteobacteria.
27. The compound of item 26 or the pharmaceutical composition of item 26 or the method of item 26, wherein said Proteobacteria are selected from the group consisting of: Neisseria spp., in particular *Neisseria gonorrhoeae,* and *Neisseria meningitides*; *Moraxella catarrhalis*; *Hemophilus influenzae*; Klebsiella spp., in particular *Klebsiella pneumoniae*; Legionella spp., in particular *Legionella pneumophila;* Pseudomonas spp., in particular *Pseudomonas aeruginosa,* and *Pseudomonas putida; Escherichia coli*; *Proteus mirabilis*; *Enterobacter cloaceae*; *Serratia marcescens; Helicobacter pylori*; and Salmonella spp., in particular *Salmonella enteritidis,* and *Salmonella typhi.*
28. The compound of item 17 or the pharmaceutical composition of item 17 or the method of item 18, wherein said bacterial infection induces the formation of biofilms or is associated with the formation of biofilms.
29. The compound of item 17 or 28 or the pharmaceutical composition of item 17 or 28 or the method of item 18 or 28, wherein said bacterial infection is selected from the group consisting of urinary tract infections, infections in cystic fibrosis, middle-ear infections, formation of dental plaques, gingivitis, caries, endocarditis, catheter infections, contact lens associated eye infections, and medical implant associated infections.
30. The compound of item 17 or the pharmaceutical composition of item 17 or the method of item 18, wherein said protozoan infection is caused by protozoa that belong to the group Apicomplexa.
31. The compound of item 30 or the pharmaceutical composition of item 30 or the method item 30, wherein said Apicomplexa are selected from the group consisting of: *Plasmodium* spp., in particular *Plasmodium falciparum, Plasmodium malariae, Plasmodium vivax, Plasmodium ovale,* and *Plasmodium knowlesi*; *Leishmania* spp., in particular *Leishmania major, Leishmania tropica, Leishmania aethiopica, Leishmania mexicana, Leishmania braziliensis, Leishmania donovani*, *Leishmania infantum,* and *Leishmania chagasi*; *Trypanosoma* spp., in particular *Trypanosoma brucei, Trypanosoma cruzi, Trypanosoma simiae, Trypanosoma avium, Trypanosoma congolense, Trypanosoma equinum, Trypanosoma equiperdum, Trypanosoma evansi,* and *Trypanosoma suis; Babesia* spp., in particular *Babesia microti,* and *Babesia bigemina*; and *Toxoplasma,* in particular *Toxoplasma gondii.*
32. The compound of any of items 15, 17 or 19 to 31 or the pharmaceutical composition of any of items 14, 15, 17 or 19 to 31 or the method of any of items 16 or 18 to 31, whereby said compound or said pharmaceutical composition is to be administered in combination with one or more antibiotics and/or antiseptics.
33. The compound of any of items 15, 17 or 19 to 32 or the pharmaceutical composition of any of items 14, 15, 17 or 19 to 32 or the method of any of items 16 or 18 to 32, whereby said compound or said pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route.
34. The method of any of items 16 or 18 to 33, wherein said subject is a human.
35. A composition comprising a compound as defined in any of items 1 to 13.
36. Non-therapeutical use of a compound as defined in any of items 1 to 13 or of the composition of item 35 for preventing the formation of biofilms or eliminating biofilms on a surface of a technical device.
37. Method of preventing the formation of biofilms or eliminating biofilms on a surface of a technical device, the method comprising the application of a compound as defined in any of items 1 to 13 or of the composition of item 35 on a surface of said technical device or parts thereof.
38. Method for sterilizing or anti-bacterial cleaning of a technical device, the method comprising the application of a compound as defined in any of items 1 to 13 or of the composition of item 35 on a surface of said technical device or parts thereof.
39. The use of item 36 or the method of item 37 or 38, wherein said technical device is selected from a catheter, a surgical instrument, contact lenses, a medical implant, or a food-processing device.
40. The use of item 39 or the method of item 39, wherein said medical implant is selected from a drug delivery device, a prostheses, a devices to be placed over or within bones to hold a fracture reduction, a dental implant, a cochlear implant, a pacemaker, a heart valve replacement, or an artificial joint.

The invention is also described by the following illustrative figures. The appended figures show:
**Figure 1****:** Hemolytic activity of *S. aureus* on sheep blood agar after treatment with the compounds according to the invention or reference compounds.
**Figure 2****:** Inhibition of *S. aureus* hemolysis by beta-lactone virulence inhibitor reference substances **(A)** or compounds according to the invention **(B).**
**Figure 3****:** In vitro *S. aureus* CIpP inhibition by beta-lactone reference substances and compounds according to the invention.
**Figure 4****:** Competitive proteome labeling of living *S. aureus* cells using U1AP (beta-lactone probe) and AVU1AA and VK273 according to the invention as competitors for covalent U1AP binding.
**Figure 5****:** *In vivo* efficacy of AVU1AA in the treatment of *S. aureus* infections in SKH1 mice by subcutaneous application after subcutaneous infection with *S. aureus* NCTC8325.
**Figure 6****:** *In vivo* efficacy of AVU1AA in the treatment of *S. aureus* infections in BALB/C mice by systemic application after subcutaneous infection with *S. aureus* NCTC8325.
**Figure 7****:** Stability of AVU1 in human plasma.
**Figure 8****:** Stability of AVU1 in mouse plasma.
**Figure 9****:** Stability of AVU1AA in human plasma.
**Figure 10****:** Stability of AVU1AA in mouse plasma.
**Figure 11****:** Stability of VK273 in mouse plasma.

The invention will now be described by reference to the following examples which are merely illustrative and are not to be construed as a limitation of the scope of the present invention.

### Examples

### Example 1: Preparation of compounds according to the invention

Several strategies were established to synthesize the compounds of the present invention.

### 1) Synthesis of 3-hydroxy-2-(non-8-enyl)-5-phenylpentanoic acid (AVU1Acid)

The compound AVU1Acid was synthesized from a beta-lactone precursor by hydrolysis using LiOH in high yields (91%):

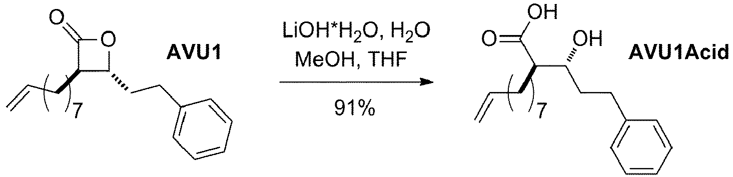

The β-lactone AVU1 (also referred to as "U1") (300 mg, 1 mmol; WO 2009/106211) was dissolved in MeOH (5 mL). THF (5 mL) was added, followed by the solution of NaOH (400 mg, 10 mmol) in water (10 mL). The reaction mixture was stirred at room temperature (rt) overnight. Volatiles were evaporated under reduced pressure and the residue was acidified using aq. HCl. The solution obtained was extracted with ethyl acetate, and the combined organic phase was dried over Na₂SO₄. The solvent was evaporated under reduced pressure and the residue was purified by column chromatography (ethyl acetate : hexane 1 : 2), yielding 240 mg (75%) of the product as a crystallizing oil.

**¹H-NMR** (360 MHz, CDCl₃): δ (ppm) = 7.37-7.15 (m, 5H), 5.90-5.73 (m, 1 H), 4.91-5.09 (m, 2H), 3.81-3.70 (m, 1 H), 2.97-2.82 (m, 1 H), 2.79-2.67 (m, 1 H), 2.55-2.44 (m, 1 H), 2.11-2.00 (m, 2H), 1.94-1.79 (m, 2H), 1.78 - 1.57 (m, 2H), 1.45- 1.22 (m, 11H). **¹³C-NMR:** 180.31, 141.59, 139.12, 128.47, 128.44, 125.98, 114.18, 71.46, 51.03, 37.17, 33.75, 32.05, 29.41, 29.20, 29.01, 28.86, 27.25

### 2) Synthesis of 3-hydroxy-2-(non-8-enyl)-5-phenylpentanamide (AVU1AA)

The amide AVU1AA was produced likewise by a ring opening reaction with ammonia in methanol:

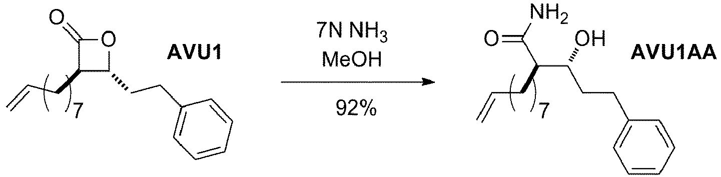

A solution of NH₃ in MeOH (4.77 mL, 7 M in MeOH, 33.4 mmol) was added at rt to the β-lacton AVU1 ("U1") (240 mg, 0.80 mmol; WO 2009/106211) and the reaction mixture was stirred for 24 h. Volatiles were evaporated under reduced pressure and the residue was purified by column chromatography (hexane / ethyl acetate = 20 : 1 to 1 : 1), yielding 230 mg (91%) of a product as a colorless solid.

**¹H-NMR** (360 MHz, CDCl₃): δ (ppm) = 7.34-7.17 (m, 5H), 6.48 (s, 1H), 6.32 (s, 1H), 5.88-5.76 (m, 1H), 4.90-5.06 (m, 2H), 3.75-3.65 (m, 1H), 2.87 (dt, J=14 Hz, 7 Hz, 1H), 2.69 (dt, J=14 Hz, 8 Hz, 1H), 2.24 (dt, J=10 Hz, 5 Hz, 1H), 2.10-2.01 (m, 2H), 1.92-1.80 (m, 2H), 1.80-1.68 (m, 1H), 1.66-1.50 (m, 1H), 1.46-1.23 (m, 11H). **¹³C-NMR:** 179.24, 141.67, 139.12, 128.49, 128.45, 126.00, 114.19, 71.78, 51.84, 37.41, 33.77, 32.24, 30.28, 29.56, 29.28, 29.03, 28.87, 27.44.

### 3) Synthesis of 3-acetoxy-2-(non-8-enyl)-5-phenylpentanoic acid (AVU1AAc) as well as AVU1AME, AVU1Acid and AVU1AAcTE (reference)

The methylester AVU1AME was produced via aldol condensation of the corresponding carboxylic acid methylester and 3-phenylpropanal. Hydrolysis and acetylation leads via AVU1Acid to AVU1AAc which can further be thioesterified with N-acetylcysteine methylester to give AVU1AAcTE (reference compound):

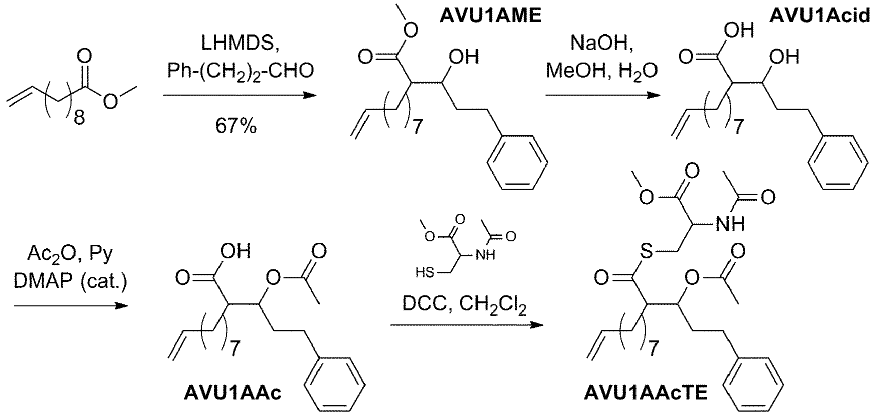

Accordingly, for the preparation of AVU1AAc the compound AVU1Acid (100 mg, 0,31 mmol) was dissolved in pyridine (3 mL) and acetic anhydride (3 mL) was added, followed by a catalytic amount of 4-dimethylaminopyridine (DMAP). The reaction mixture was stirred overnight. Volatiles were evaporated under reduced pressure. The residue was separated by column chromatography (hexane / ethyl acetate = 20 : 1 to 1 : 1) to yield 75 mg (67%) of the product.

AVU1AAc: **¹H-NMR** (360 MHz, CDCl₃): δ (ppm) = 7.38-7.14 (m, 5H), 5.90-5.76 (m, 1H), 5.25-5.18 (m, 1H), 4.92-5.06 (m, 2H), 2.82-2.74 (m, 1H), 2.73-2.62 (m, 2H), 2.50-2.60 (m, 1H), 2.29-2.19 (m, 2H), 2.06 (s, 3H), 1.40-1.26 (m, 13H). **¹³C-NMR:** 172.94, 170.47, 141.90, 139.15, 128.49, 128.42, 125.92, 114.19, 73.50, 49.31, 37.74, 34.92, 32.39, 32.13, 29.56, 29.41, 29.03, 28.87, 27.35, 20.96.

### 4) Synthesis of 2-(non-8-enyl)-5-phenylpentandiol-1,3 (VK273)

The compound VK273 was synthesized from a beta-lactone precursor by reduction using LiAlH₄ in high yields (78%):

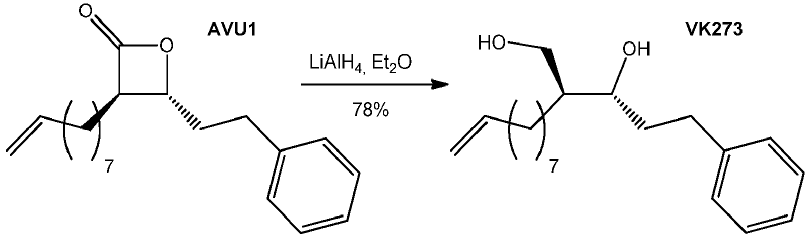

The β-lactone AVU1 ("U1") (300 mg, 1 mmol; WO 2009/106211) was dissolved in Et₂O (10 mL) and cooled to 0 °C. LiAlH₄ (76 mg, 2 mmol) was added and the reaction mixture was stirred 1 h at room temperature (rt). The reaction was quenched by saturated aq. NH₄Cl. The solution obtained was extracted with ethyl acetate, and the combined organic phase was dried over Na₂SO₄. The solvent was evaporated under reduced pressure and the residue was purified by column chromatography (ethyl acetate : hexane 1 : 1), yielding 237 mg (78%) of the product as an oil.

**¹H-NMR** (360 MHz, CDCl₃): δ (ppm) = 7.39-7.18 (m, 5H), 5.91-5.76 (m, 1 H), 5.08-4.91 (m, 2H), 4.02-3.92 (m, 1 H), 3.81-3.62 (m, 2H), 2.93-2.81 (m, 1 H), 2.77-2.63 (m, 4H), 2.11-2.00 (m, 2H), 1.94-1.87 (m, 2H), 1.55-1.22 (m, 12H). **¹³C-NMR:** 141.94, 139.17, 128.49, 128.41, 125.98, 114.16, 75.49, 64.25, 44.35, 37.42, 33.78, 33.24, 29.84, 29.38, 29.08, 28.90, 38.58, 27.23.

### 5) Synthesis of various substituted 3-hydroxyamides (VK508-3, VK375, VK403-1, VK453-1)

Amides were produced from corresponding β-lactones by a ring opening reaction with ammonia in either methanol (MeOH) or in isopropanol (*i*PrOH).

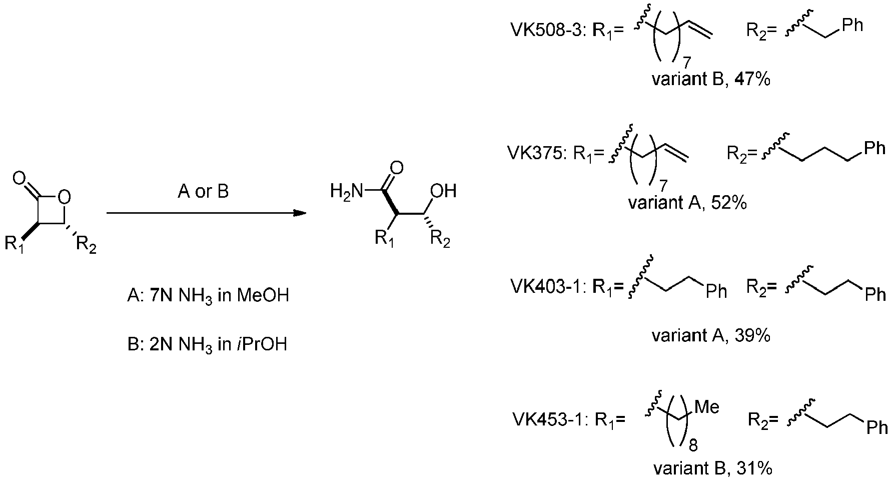

### General Procedure A (GPA)

A solution of NH₃ (7 mL, 7 N in MeOH) was added to the corresponding *trans*-lactone (1.4 mmol). The reaction mixture was stirred for 18 h at rt. Volatiles were removed under reduced pressure and the crude product was isolated by column chromatography (CH₂Cl₂ / MeOH = 30 : 1). Some products were additionally purified by preparative HPLC.

### General Procedure B (GPB)

A solution of NH₃ (30 mL, 2 N in *i*PrOH) was added to the corresponding *trans*-lactone (3.1 mmol). The reaction mixture was stirred for 18 h at rt. Volatiles were removed under reduced pressure and the crude product was isolated by column chromatography (CH₂Cl₂ / MeOH = 30 : 1). Some products were additionally purified by preparative HPLC.

### (2R*,3R*)-3-Hydroxy-2-(non-8-enyl)-4-phenylbutanamide (VK508-3)

(2*R**,3*R**)-3-Hydroxy-2-(non-8-enyl)-4-phenylbutanamide **(VK508-3)** was synthesized according to GPB starting from (3*R**,4*R**)-3-(non-8-enyl)-4-benzyl-oxetan-2-one (10 mg, 0.035 mmol) and solution of NH₃ (5 mL, 2 N in *i*PrOH). The crude product was purified by HPLC. Yield: 4.5 mg, (42%).

**¹H-NMR** (360 MHz, CDCl₃): δ 7.43-7.17 (m, 5 H), 6.00 (s, 1 H), 5.88 - 5.76 (m, 2H), 5.04 - 4.91 (m, 2H), 4.00 - 3.92 (m, 1 H), 2.95-2.82 (m, 2H), 2.23 (ddd, *J* = 9.0, 6.2, 3.1 Hz, 1H), 2.08 - 2.00 (m, 3 H), 1.88 - 1.73 (m, 1H), 1.73 - 1.60 (m, 1H), 1.42 - 1.24 (m, 10H). ESI-MS (m/z): 304.2269 [M+H], calcd for: C₁₉H₃₀NO₂: 304.2277

### (2R*,3R*)-3-Hydroxy-2-(non-8-enyl)-6-phenylhexanamide (VK375)

(2*R**,3*R**)-3-Hydroxy-2-(non-8-enyl)-6-phenylhexanamide **(VK375)** was synthesized according to GPA starting from (3*R**, 4*R**)-3-(non-8-enyl)-4-(3-phenylpropyl)-oxetan-2-one (440 mg, 1.4 mmol) and solution of NH₃ (7 mL, 7 N in MeOH). After purification by column chromatography the product was additionally purified by HPLC. Yield: 240 mg (52%).

**¹H-NMR** (360 MHz, CDCl₃): δ 7.32-7.17 (m, 5 H), 6.00 (s, 1 H), 5.89 - 5.76 (m, 1 H), 5.72 (s, 1 H), 5.36 (s, 1 H), 5.04 - 4.92 (m, 2H), 3.74 - 3.66 (m, 1 H), 2.97 (d, *J* = 7.2 Hz, 1 H), 2.66 (t, *J* = 7.5 Hz, 2H), 2.20 - 2.11 (m, 1 H), 2.09 - 2.01 (m, 2H), 1.92 - 1.66 (m, 2H), 1.64 - 1.52 (m, 4H), 1.42 - 1.24 (m, 9H). **¹³C-NMR** (91 MHz, CDCl₃): δ 177.62, 142.21, 139.11, 128.40, 128.32, 125.78, 114.19, 72.19, 51.50, 35.67, 35.36, 33.74, 30.43, 29.55, 29.28,

29.02, 29.28, 28.87, 27.77. ESI-MS (m/z): 332.2586 [M+H], calcd for: C₂₁H₃₄NO₂: 332.2590.

### (2R*,3R*)-3-Hydroxy-2-(2-phenylethyl)-5-phenylpentanamide (VK403-1)

(2*R**,3*R**)-3-Hydroxy-2-(2-phenylethyl)-5-phenylpentanamide **(VK403-1)** was synthesized according to GPA starting from (3*R**,4*R**)-3,4-bis-(2-phenylethyl)-oxetan-2-one (57 mg, 0.2 mmol) and solution of NH₃ (4 mL, 7 N in MeOH). After purification by column chromatography the product was additionally purified by HPLC. Yield: 23 mg (39%).

**¹H-NMR** (360 MHz, CDCl₃): δ 7.32-7.17 (m, 10H), 5.61 (s, 1H), 5.42 (s, 1H), 3.77 - 3.69 (m, 1 H), 3.13 (d, *J =* 7.5 Hz, 1 H), 2.92 - 2.82 (m, 1 H), 2.79 - 2.60 (m, 3H), 2.23 - 2.10 (m, 2H), 2.05 - 1.73 (m, 3H). ESI-MS (m/z): 298.1806 [M+H], calcd for: C₁₉H₂₄NO₂: 298.1807.

### (2R*,3R*)-3-Hydroxy-2-nonyl-5-phenylpentanamide (VK453-1)

(2*R**,3*R**)-3-Hydroxy-2-nonyl-5-phenylpentanamide **(VK453-1)** was synthesized according to GPB starting from (3*R**,4*R**)-3-nonyl-4-(2-phenylethyl)-oxetan-2-one (920 mg, 3.1 mmol) and solution of NH₃ (30 mL, 2 N in *i*PrOH). The product was purified by column chromatography. Yield: 300 mg (31%).

**¹H-NMR** (360 MHz, CDCl₃): δ 7.33-7.17 (m, 5 H), 5.76 (s, 1 H), 5.57 (s, 1 H), 3.75 - 3.66 (m, 1H), 3.24 (d, *J* = 7.5 Hz, 1H), 2.89 (ddd, *J* = 14.6, 8.8, 5.7 Hz, 1H), 2.76 - 2.65 (m, 1H), 2.22 - 2.14 (m, 1H), 1.94 - 1.73 (m, 3H), 1.66 - 1.56 (m, 1H), 1.40 - 1.22 (m, 14H), 0.90 (t, *J* = 6.9 Hz, 3H). **¹³C-NMR** (91 MHz, CDCl₃): δ 177.86, 141.89, 128.49, 128.44, 125.91, 71.79, 51.57, 37.71, 32.38, 31.86, 30.44, 29.61, 29.53, 29.46, 29.28, 27.55, 22.67, 14.10.

ESI-MS (m/z): 320.2589 [M+H], calcd for: C₂₀H₃₄NO₂: 320.2590.

### 6) Synthesis of 3-hydroxyacids (JS-11 1, JS-11 2), their methyl esters (JS-14 1, JS-14 2) and amides (JS-14 1Amid and JS-14 2Amid) containing 3H-diazirine moiety.

For the synthesis of further compounds methyl 11-(trimethylsilyl)undec-10-inoate was coupled with 3-(4-(3-trifluoromethyl-3H-diazirin-yl)-phenyl)-propanal. The diastereomeric products obtained could be separated and converted into 3-hydroxyacids **JS-11_1** and **JS-11_2.** After HBTU-mediated cyclisation the corresponding *cis-* and *trans-* beta-lactones were obtained. Their reaction with NH₃ in MeOH gave rise to the formation of amides **(JS-14_1Amid and JS-14_2Amid)** and esters **(JS-14_1** and **JS-14_2,** respectively).

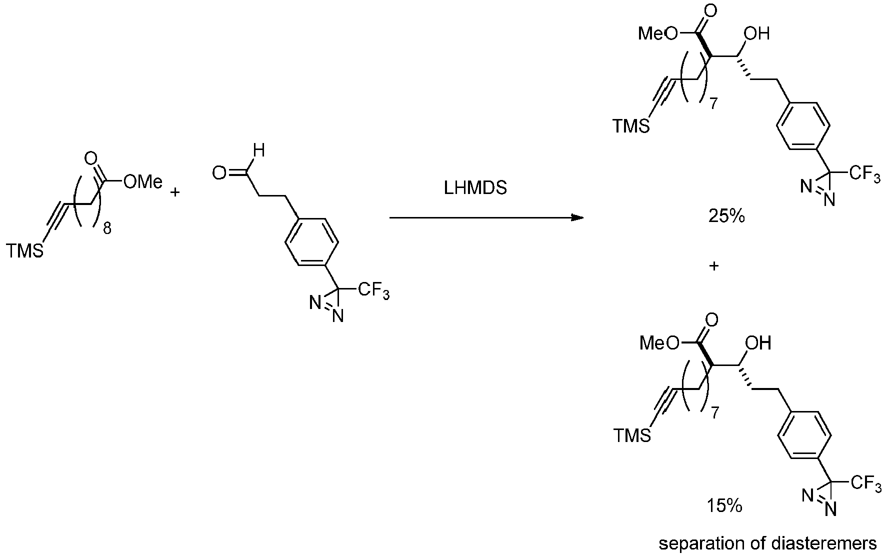

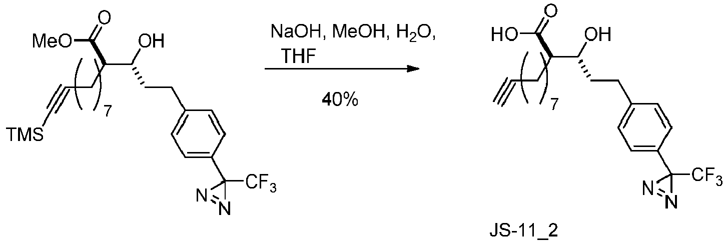

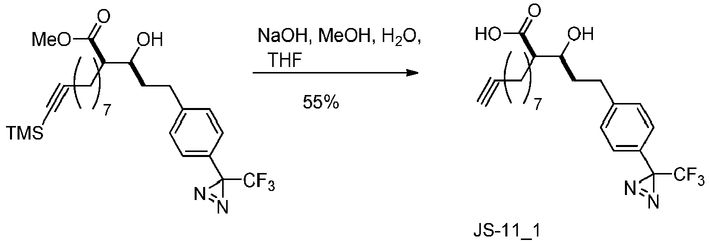

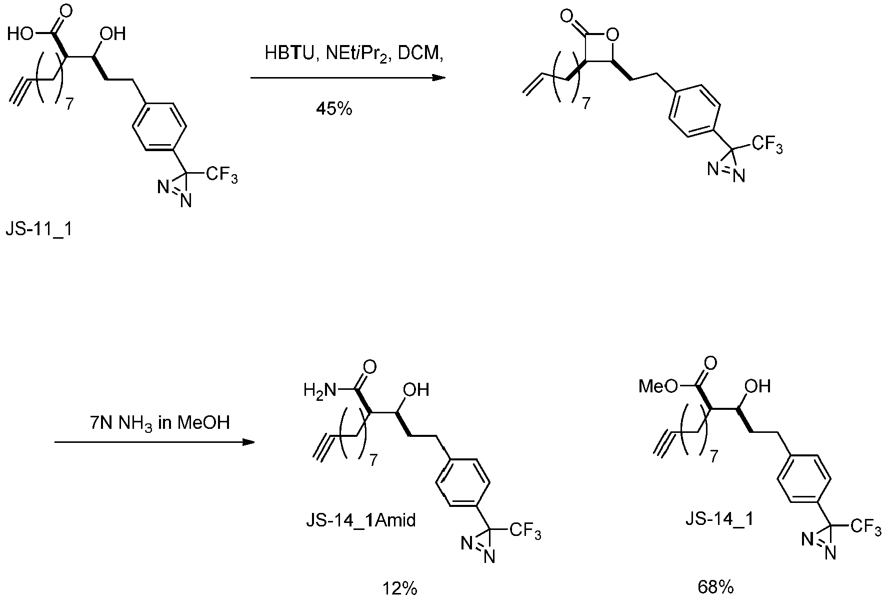

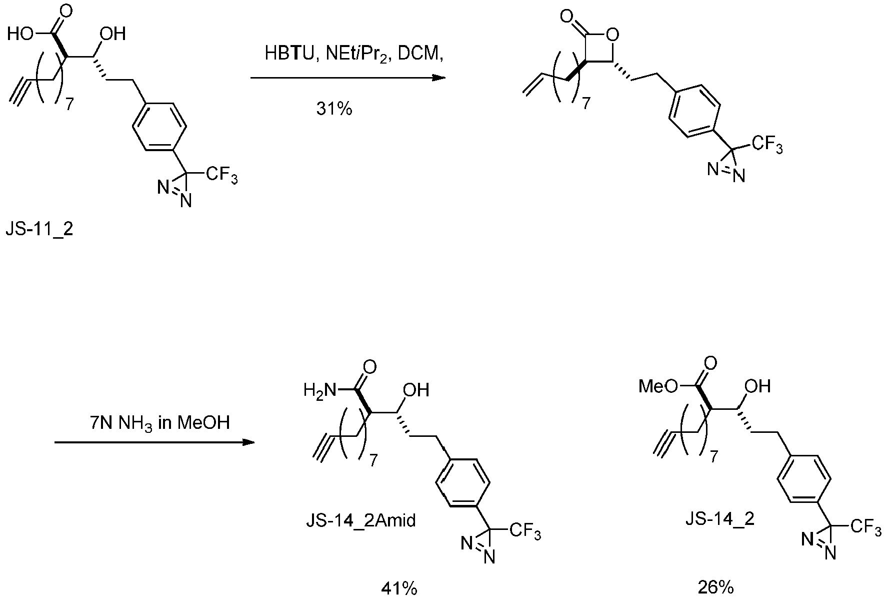

### Methyl 3-Hydroxy-2-(9-trimethylsilyl-8-noninyl)-5-(4-(3-trifluoromethyl-3H-diazirin-yl)-phenyl)-pentanoate

Lithium bis(trimethylsilyl)amide (LHMDS) (17.3 mL, 1 M in THF, 17.3 mmol) was slowly added to the solution of methyl 11-(trimethylsilyl)undec-10-inoate (4.08 g, 15.2 mmol) in dry THF (40 mL) at -78°C under Ar. The mixture was stirred for 30 min at this temperature. A solution of 3-(4-(3-trifluoromethyl-3H-diazirin-yl)-phenyl)-propanal (3.35 g, 13.8 mmol) in dry THF (40 mL) was slowly added to the reaction mixture and the reaction was stirred for 30 min at -78°C and for 2 h at 0°C. The reaction was quenched by the addition of H₂O (100 mL). The mixture obtained was extracted with EtOAc (3 x 100 mL). Combined organic phases were dried over Na₂SO₄ and concentrated under reduced pressure. The crude product was separated by column chromatography (hexane/EtOAc = 11/1 to 3/1) yielding 1.03 g (14.5%) of the (*R**,*S**)- diastereomer and 1.74 g (24.6%) of (*R**,*R**)*-*diastereomer.

**(*R**,*S**)- Methyl 3-Hydroxy-2-(9-trimethylsilyl-8-noninyl)-5-(4-(3-trifluoromethyl-3*H-*diazirin-yl)-phenyl)-pentanoate:** *R*_{f} = 0.40 (hexane / EtOAc = 5 : 1), **¹H-NMR** (360 MHz, CDCl₃): δ 7.23 (dd, *J* = 8.4, 2.1 Hz, 2H), 7.12 (t, *J =* 6.8 Hz, 2H), 3.77 (dd, *J* = 9.5, 4.7 Hz, 1 H), 3.70 (s, 3H), 2.95 - 2.81 (m, 2H), 2.73 - 2.60 (m, 2H), 2.47 - 2.42 (m, 1 H), 1.87 - 1.61 (m, 6H), 1.37 - 1.25 (m, 8H), 0.14 (s, 9H).
ESI-MS (m/z): 511.2597 [M+H], calcd for: C₂₆H₃₈F₃N₂O₃Si: 511.2598

### (R*,R*)- Methyl 3-Hydroxy-2-(9-trimethylsilyl-8-noninyl)-5-(4-(3-trifluoromethyl-3H-diazirin-yl)-phenyl)-pentanoate:

*R*_{f} = 0.31 (hexane/EtOAc = 5 : 1), **¹H-NMR** (360 MHz, CDCl₃): δ 7.22 (d, *J* = 8.4 Hz, 2H), 7.10 (t, *J* = 6.8 Hz, 2H), 3.70 (s, 3H), 3.65 (dd, *J* = 8.7, 4.6 Hz, 1H), 2.89 - 2.83 (m, 2H), 2.74 - 2.65 (m, 2H), 2.47 - 2.40 (m, 1 H), 1.77 - 1.67 (m, 6H), 1.37 - 1.24 (m, 8H), 0.14 (s, 9H).
ESI-MS (m/z): 511.2596 [M+H], calcd for: C₂₆H₃₈F₃N₂O₃Si: 511.2598

### (3R*,2S*)-3-Hydroxy-2-(8-noninyl)-5-(4-(3-trifluoromethyl-3H-diazirinyl)-phenyl)-pentanoic acid (JS11_1)

A solution of NaOH (266 mg, 6.7 mmol) in H₂O (7 mL) and MeOH (7 mL) was added to the solution of (*R**,*S**)- methyl 3-hydroxy-2-(9-trimethylsilyl-8-noninyl)-5-(4-(3-trifluoromethyl-3*H*-diazirin-yl)-phenyl)-pentanoate (340 mg, 0.67 mmol) in THF (27 mL).

The reaction mixture was stirred for 3 h at rt. The reaction was quenched by the addition of conc. HCl (6 mL). Phases were separated and the aq. phase was extracted with EtOAc(2 x 50 mL). Combined organic phases were dried over Na₂SO₄ and concentrated under reduced pressure. The crude product was purified by column chromatography (2.5 cm × 20 cm, hexane/EtOAc = 2 : 1 to 1 : 2) to yield 133 mg (47%) of a product as a colorless oil.

*R*_{f} = 0.31 (Hexane / EtOAc = 2 : 1), **¹H**-**NMR** (360 MHz, CDCl₃): δ 7.23 (d, *J* = 8.2 Hz, 2H), 7.12 (d, *J* = 8.2 Hz, 2H), 3.83 (dt, *J* = 9.5, 3.8 Hz, 1 H), 2.89 (ddd, *J* = 14.4, 9.5, 5.3 Hz, 2H), 2.68 (ddd, *J* = 14.0, 9.2, 7.1 Hz, 2H), 2.49 (dt, *J* = 9.4, 4.4 Hz, 1 H), 1.93 (t, *J* = 2.6 Hz, 1 H), 1.89 - 1.65 (m, 4H), 1.60 - 1.45 (m, 4H), 1.45 - 1.18 (m, 6H). **¹³C-NMR** (91 MHz, CDCl₃): δ 178.63, 143.63, 129.11, 126.78, 71.22, 68.29, 50.76, 35.53, 32.04, 29.53, 28.97, 28.54, 27.80, 26.94, 18.52.
ESI-MS (m/z): 425.2047 [M+H], calcd for: C₂₂H₂₈F₃N₂O₃: 425.2047

### (3R*,2R*)-3-Hydroxy-2-(8-noninyl)-5-(4-(3-trifluoromethyl-3H-diazirinyl)-phenyl)-pentanoic acid (JS11_2)

(3*R**,2*R**)-3-Hydroxy-2-(8-noninyl)-5-(4-(3-trifluoromethyl-diazirinyl)-phenyl)-pentanoic acid **(JS11_2)** was synthesized according to the procedure for (3*R**,2*S**)-3-hydroxy-2-(8-noninyl)-5-(4-(3-trifluoromethyl-3*H*-diazirinyl)-phenyl)-pentanoic acid **(JS11_1)** starting from (*R**,*R**)- methyl 3-hydroxy-2-(9-trimethylsilyl-8-noninyl)-5-(4-(3-trifluoromethyl-3*H-*diazirin-yl)-phenyl)-pentanoate (510 mg, 1.0 mmol), NaOH (400 mg, 10.0 mmol), H₂O (10 mL), MeOH (10 mL) and THF (40 mL). Yield: 102 mg (24%) as colorless oil.

*R*_{f} = 0.29 (hexane / EtOAc = 2 : 1), **¹H-NMR** (250 MHz, CDCl₃): δ 7.31 - 7.18 (m, 2H), 7.12 (d, *J* = 8.1 Hz, 2H), 3.73 - 3.66 (m, 1 H), 3.01 - 2.61 (m, 2H), 2.61 - 2.25 (m, 1H), 2.18 (td, *J* = 7.0, 2.6 Hz, 2H), 1.93 (t, *J* = 2.7 Hz, 1 H), 1.87 - 1.45 (m, 6H), 1.45 - 1.10 (m, 8H). **¹³C-NMR** (91 MHz, CDCl₃): δ 178.63, 143.63, 129.11, 126.78, 71.22, 68.29, 50.76, 35.53, 32.04, 29.53, 28.97, 28.54, 27.80, 26.94, 18.52.
ESI-MS (m/z): 425.2046 [M+H], calcd for: C₂₂H₂₈F₃N₂O₃: 425.2047

### (3R*,4S*)-3-(8-Noninyl)-4-(4-(3-(trifluoromethyl)-3H-diazirinyl)phenylethyl)oxetan-2-one

To the solution of (3*R**,2*S**)-3-hydroxy-2-(8-noninyl)-5-(4-(3-trifluoromethyl-3*H*-diazirinyl)-phenyl)-pentanoic acid **(JS11_1)** (65 mg, 0.15 mmol) in dry CH₂Cl₂ (2.5 mL) diisopropylethylamine (73 µL, 0.43 mmol) was added followed by HBTU (70 mg, 0.18 mmol). The reaction mixture was stirred for 1.5 h at rt. H₂O (20 mL) and CH₂Cl₂ (20 mL) were added and phases were separated. Aqueous phase were extracted with CH₂Cl₂ (20 mL). Combined organic phases were dried over Na₂SO₄ and concentrated under reduced pressure. The crude product was purified by column chromatography (1.5 cm × 20 cm, hexane/EtOAc = 20 : 1 to 10 : 1) yielding 28 mg (45%) of product as a colorless oil.

*R*_{f} = 0.43 (hexane / EtOAcE = 5 : 1). IR [cm⁻¹]: 2934.16, 2858.95, 2359.48, 2348.87, 2341.16, 1818.54, 1616.06, 1519.63, 1456.96, 1345.11, 1313.29, 1303.64, 1285.32, 1232.29, 1214.93, 1184.08, 1171.54, 1154.19, 1110.8, 1100.19, 1078.98, 1066.44, 1061.62, 1053.91, 1040.41, 1009.55, 939.163, 922.771, 905.415, 890.952, 838.883. **¹H-NMR** (250 MHz, CDCl₃): δ 7.24 (d, *J* = 8.5 Hz, 2H), 7.14 (d, *J* = 8.5 Hz, 2H), 4.50 (ddd, *J =* 10.0, 6.4, 3.4 Hz, 1H), 3.61 (dt, *J* = 9.1, 6.7 Hz, 1H), 2.91 (ddd, *J* = 14.2, 9.4, 5.1 Hz, 1H), 2.72 (dt, *J* = 13.8, 8.2 Hz, 1H), 2.17 (td, *J* = 6.9, 2.7 Hz, 2H), 2.09 - 1.74 (m, 7H), 1.67-1.17 (m, 8H). ESI-MS (m/z): 405.1794 [M-H], calcd for: C₂₂H₂₄F₃N₂O₂-: 405.1795.

### (3R*,4R*)-3-(8-Noninyl)-4-(4-(3-(trifluoromethyl)-3H-diazirinyl)phenylethyl)oxetan-2-one

(3*R**,4*R**)-3-(8-Noninyl)-4-(4-(3-(trifluoromethyl)-3*H*-diazirinyl)phenylethyl)oxetan-2-one was obtained according to the procedure for (3*R**,4*S**)-3-(8-noninyl)-4-(4-(3-(trifluoromethyl)-3*H*-diazirinyl)phenylethyl)oxetan-2-one starting from (3*R**, 2*R**)-3-hydroxy-2-(8-noninyl)-5-(4-(3-trifluoromethyl-3*H*-diazirinyl)-phenyl)-pentanoic acid **(JS11_2)** (50 mg, 0.12 mmol), diisopropylethylamine (57 µL, 0.33 mmol) and HBTU (54 mg, 0.14 mmol). Yield: 15 mg (31%) as a colorless oil.

*R*_{f} = 0.49 (hexane / EtOAc = 5/1). IR [cm⁻¹]: 2930.31, 2340.19, 1819.51, 1345.11, 1232.29, 1183.11, 1154.19, 1119.48, 1081.87, 1053.91, 1011.48, 938.199, 894.809, 877.452, 844.669, 808.992, 725.104. **¹H-NMR** (250 MHz, CDCl₃): δ 7.22 (d, *J* = 8.1 Hz, 2H), 7.14 (d, *J* = 8.1 Hz, 2H), 4.19 (ddd, *J* = 7.9, 5.3, 3.9 Hz, 1H), 3.18 (ddd, *J* = 8.3, 6.9, 3.9 Hz, 1 H), 2.98 - 2.60 (m, 2H), 2.24 - 2.00 (m, 3H), 1.93 (t, *J* = 2.6 Hz, 1 H), 1.81 - 1.63 (m 1 H), 1.61 - 1.17 (m, 12H). ESI-MS (m/z): 405.1794 [M-H], calcd for: C₂₂H₂₄F₃N₂O₂: 405.1795

### (3R*,2S*)-3-Hydroxy-2-(8-noninyl)-5-(4-(3-trifluoromethyl-3H-diazirinyl)-phenyl)-pentanamide (JS-14_1Amid) and Methyl (3R*,2S*)-3-Hydroxy-2-(8-noninyl)-5-(4-(3-trifluoromethyl-3H-diazirinyl)-phenyl)-pentanoate (JS-14_1)

A solution of NH₃ (2.5 mL, 7 N in MeOH) was added to (3*R**,4*S**)-3-(8-Noninyl)-4-(4-(3-(trifluoromethyl)-3*H*-diazirinyl)phenylethyl)oxetan-2-one (20.3 mg, 0.05 mmol). The reaction mixture was stirred for 18 h at rt. Volatiles were removed under reduced pressure and the crude product was separated by HPLC yielding 2.6 mg (12%) of the amide **(JS-14_1Amid)** and 15.0 mg (68%) of the corresponding methyl ester **(JS-14_1).**

**(3*R**,2*S**)-3-Hydroxy-2-(8-noninyl)-5-(4-(3-trifluoromethyl-3*H*-diazirinyl)-phenyl)-pentanamide (JS-14_1Amid):** IR [cm⁻¹]: 3373.85, 3186.79, 2919.7, 2856.06, 2359.48, 2342.12, 2331.52, 1716.34, 1698.02, 1650.77, 1558.2, 1540.85, 1520.6, 1508.06, 1488.78, 1456.96, 1420.32, 1347.03, 1295.93, 1258.32, 1230.36, 1191.79, 1152.26, 1109.83, 1063.55, 1022.09, 940.128, 921.807, 914.093, 886.131, 873.596, 811.885, 802.242, 762.709, 722.211. **¹H-NMR** (250 MHz, CDCl₃): δ 7.23 (d, *J* = 8.2 Hz, 2H), 7.11 (d, *J* = 8.2 Hz, 2H), 5.64 (s, 1 H), 5.40 (s, 1 H), 3.88 - 3.73 (m, 1 H), 3.07 (s, 1 H), 2.88 (ddd, *J =* 14.4, 9.6, 5.2 Hz, 1 H), 2.76 - 2.56 (m, 1 H), 2.17 (td, *J =* 7.0, 2.9 Hz, 3H), 1.93 (t, *J =* 2.7 Hz, 1H), 1.89 - 1.61 (m, 5H), 1.61 - 1.11 (m, 9H). **¹³C-NMR** (91 MHz, CDCl₃): δ 143.83, 129.13, 126.76, 76.65, 71.41, 68.28, 51.51, 35.62, 32.21, 29.71, 29.04, 28.77, 28.55, 28.00, 18.51. ESI-MS (m/z): 424.2201 [M+H], calcd for: C₂₂H₂₉F₃N₃O₂: 424.2206

**Methyl (3*R**,2*S**)-3-Hydroxy-2-(8-noninyl)-5-(4-(3-trifluoromethyl-3*H*-diazirinyl)-phenyl)-pentanoate (JS-14_1):** IR [cm⁻¹]: 2360.44, 2341.16, 1731.76, 1715.37, 1616.06, 1519.63, 1455.99, 1435.74, 1345.11, 1301.72, 1231.33, 1184.08, 1154.19, 1106.94, 1052.94, 1021.12, 939.163, 855.275, 834.062, 812.849, 725.104. **¹H-NMR** (360 MHz, CDCl₃): δ 7.22 (d, *J* = 8.1 Hz, 2H), 7.11 (d, *J* = 8.1 Hz, 2H), 3.84 - 3.72 (m, 1 H), 3.70 (s, 3H), 2.88 (ddd, *J* = 14.5, 9.7, 5.2 Hz, 1 H), 2.66 (ddd, *J* = 13.7, 9.4, 7.0 Hz, 1 H), 2.44 (dt, , *J* = 9.6, 4.3 Hz, 2H), 2.17 (td, *J =* 7.0, 2.6 Hz, 3H), 1.93 (t, *J* = 2.6 Hz, 1 H), 1.84 - 1.61 (m, 2H), 1.61 - 1.44 (m, 2H), 1.44 - 1.16 (m, 8H). **¹³C-NMR** (91 MHz, CDCl₃): δ 175.99, 143.89, 129.12, 126.81 (d, *J* = 10.8 Hz), 77.51, 71.31, 68.26, 51.85, 51.15, 35.78, 32.05, 29.53, 28.99, 28.65 (d, *J =* 18.6 Hz), 27.82, 27.13, 18.51. ESI-MS (m/z): 439.2199 [M+H], calcd for: C₂₃H₃₀F₃N₂O₃+: 439.2203

### (3R*,2R*)-3-Hydroxy-2-(8-noninyl)-5-(4-(3-trifluoromethyl-3H-diazirinyl)-phenyl)-pentanamide (JS-14_2Amid) and Methyl (3R*,2R*)-3-Hydroxy-2-(8-noninyl)-5-(4-(3-trifluoromethyl-3H-diazirinyl)-phenyl)-pentanoate (JS-14_2)

A mixture of (3*R**,2*R**)-3-hydroxy-2-(8-noninyl)-5-(4-(3-trifluoromethyl-3*H*-diazirinyl)-phenyl)-pentanamide **(JS-14_2Amid)** and methyl (3*R**,2*R**)-3-hydroxy-2-(8-noninyl)-5-(4-(3-trifluoromethyl-3*H*-diazirinyl)-phenyl)-pentanoate **(J-14_2)** was obtained according to the procedure for (3*R**,2*S**)-3-hydroxy-2-(8-noninyl)-5-(4-(3-trifluoromethyl-3H-diazirinyl)-phenyl)-pentanamide **(JS-14_1Amid)** and methyl (3*R**,2*S**)-3-hydroxy-2-(8-noninyl)-5-(4-(3-trifluoromethyl-3H-diazirinyl)-phenyl)-pentanoate **(JS-14_1)** starting from (3*R**,4*R**)-3-(8-noninyl)-4-(4-(3-(trifluoromethyl)-3H-diazirinyl)phenylethyl)oxetan-2-one (11.9 mg, 0.03 mmol) and solution of NH₃ (2.5 mL, 7 N in MeOH). Yield: 5.1 mg (41%) of the amide **(JS-14_2Amid)** and 3.3 mg (26%) of methyl ester **(JS-14_2).**

**(3*R**,2*R**)-3-Hydroxy-2-(8-noninyl)-5-(4-(3-trifluoromethyl-3*H*-diazirinyl)-phenyl)-pentanamide (JS-14_2Amid):** IR [cm⁻¹]: 732.55, 3688.19, 2929.34, 2856.06, 2360.44, 2341.16, 1653.66, 1540.85, 1520.6, 1507.1, 1455.99, 1428.99, 1347.03, 1261.22, 1232.29, 1184.08, 1148.4, 1113.69, 1078.98, 1055.84, 1036.55, 1020.16, 940.128, 914.093, 838.883, 806.099. **¹H-NMR** (250 MHz, CDCl₃): δ 7.23 (d, *J* = 8.2 Hz, 2H), 7.11 (d, *J* = 8.2 Hz, 2H), 5.68 (s, 1 H), 5.46 (s, 1 H), 3.64 (dt, *J* = 8.5, 4.0 Hz, 1 H), 2.89 (ddd, *J =* 14.6, 9.3, 5.7 Hz, 1 H), 2.80 - 2.61 (m, 1 H), 2.15 (dtd, *J* = 9.2, 6.2, 5.5, 3.2 Hz, 3H), 1.93 (t, 1 H), 1.90 - 1.68 (m, 5H), 1.68 - 1.19 (m, 9H). **¹³C-NMR** (63 MHz, CDCl₃): δ 143.96, 129.12, 126.74, 77.67, 77.36, 77.16, 76.77, 76.65, 71.69, 68.28, 51.69, 37.62, 32.18, 30.56, 29.60, 29.03, 28.76, 28.54, 27.62, 18.51. ESI-MS (m/z): 424.2203 [M+H], calcd for: C₂₂H₂₉F₃N₃O₂: 424.2206

**Methyl (3*R**,2*R**)-3-Hydroxy-2-(8-noninyl)-5-(4-(3-trifluoromethyl-3*H*-diazirinyl)-phenyl)-pentanoate (JS-14_2):** IR [cm⁻¹]: 732.55, 3688.19, 2929.34, 2856.06, 2360.44, 2341.16, 1653.66, 1540.85, 1520.6, 1507.1, 1455.99, 1428.99, 1347.03, 1261.22, 1232.29, 1184.08, 1148.4, 1113.69, 1078.98, 1055.84, 1036.55, 1020.16, 940.128, 914.093, 838.883, 806.099

**¹H-NMR** (360 MHz, CDCl₃): δ 7.22 (d, *J* = 8.2 Hz, 2H), 7.11 (d, *J* = 8.2 Hz, 2H), 3.70 (s, 3H), 3.64 (dt, *J* = 9.0, 4.4 Hz, 1 H), 2.87 (ddd, *J* = 14.6, 9.4, 5.4 Hz, 1 H), 2.70 (ddd, *J =* 13.9, 9.4, 7.0 Hz, 1H), 2.43 (dt, *J* = 8.9, 5.3 Hz, 2H), 2.17 (td, *J =* 7.0, 2.7 Hz, 3H), 1.93 (t, *J* = 2.6 Hz, 1 H), 1.72 (dddd, *J* = 14.5, 9.0, 6.8, 4.5 Hz, 2H), 1.64 - 1.45 (m, 2H), 1.42 - 1.20 (m, 9H). **¹³C-NMR** (91 MHz, CDCl₃): δ 176.21, 143.93, 129.12, 126.75, 71.52, 68.27, 51.79, 51.05, 37.39, 31.97, 29.80, 29.45, 28.99, 28.65 (d, *J* = 18.2 Hz), 27.44, 18.53. ESI-MS (m/z): 439.2201 [M+H], calcd for: C₂₃H₃₀F₃N₂O₃+: 439.2203

### 7) Synthesis of 3-hydroxyacid (JS-64) and its amide (JS-65) containing benzophenone-moiety

For the synthesis of compounds with benzophenone-moiety methyl 11-(trimethylsilyl)undec-10-inoate was coupled with 3-(4-(2-phenyl-1,3-dioxolanyl)phenyl)propanal. Stepwise hydrolysis of the product formed lead to the acid JS-64 that was further converted into its amide JS-65.

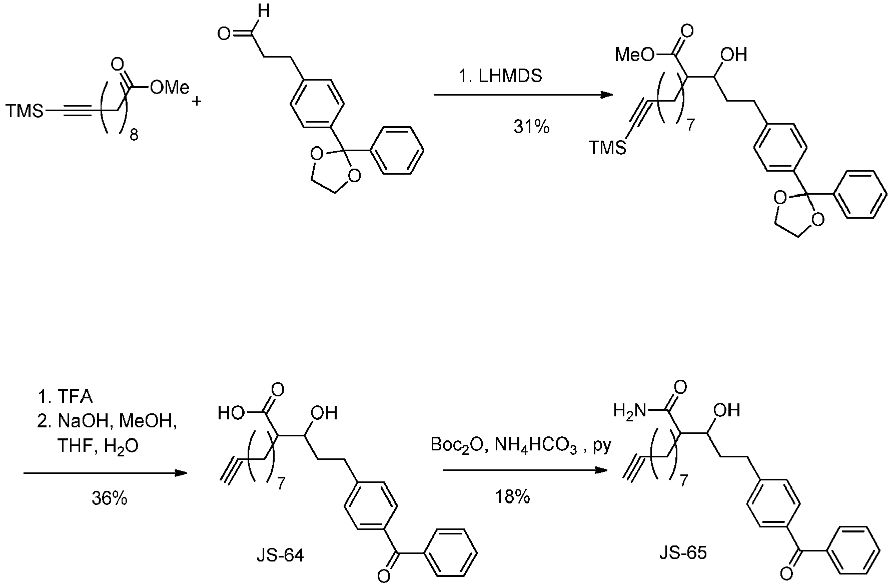

### Methyl 2-Hydroxy-3-(9-trimethylsilyl-8-noninyl)-5-(4-(2-phenyl-1,3-dioxolan-2-yl)phenyl)-pentanoate

LHMDS (15 mL, 1 M in THF, 15 mmol) was slowly added to the solution of methyl 11-(trimethylsilyl)undec-10-inoate (3.6 g, 13.2 mmol) in dry THF (25 mL) at -78°C under Ar. The mixture was stirred for 30 min at this temperature. A solution of 3-(4-(2-phenyl-1,3-dioxolanyl)phenyl)propanal (3.4 g, 12.0 mmol) in dry THF (25 mL) was slowly added to the reaction mixture and the reaction was stirred for 30 min at -78°C and for 2 h at 0°C. The reaction was quenched by the addition of H₂O (100 mL). The mixture obtained was extracted with EtOAc (3 x 100 mL). The combined organic phases were dried over Na₂SO₄ and concentrated under reduced pressure. The crude product was purified by column chromatography (hexane/EtOAc = 11 : 1 to 0 : 1) yielding 2.05 g (31%) of methyl 2-hydroxy-3-(9-trimethylsilyl-8-noninyl)-5-(4-(2-phenyl-1,3-dioxolan-2-yl)phenyl)-pentanoate as a colorless oil.

*R*_{f} = 0.33 (hexane/EtOAc = 5 : 1). **¹H-NMR** (250 MHz, CDCl₃): δ 7.51 (ddp, *J* = 6.6, 2.8, 1.2 Hz, 2H), 7.43 (dd, *J* = 8.1, 4.6 Hz, 2H), 7.39 - 7.23 (m, 3H), 7.15 (d, *J* = 8.0 Hz, 2H), 4.06 (d, *J* = 1.9 Hz, 4H), 3.88 - 3.73 (m, 1 H), 3.70 (s, 3H), 3.00 - 2.36 (m, 4H), 2.21 (t, *J =* 7.0 Hz, 1H), 1.83 - 1.41 (m, 4H), 1.41 - 1.14 (m, 8H), 0.15 (s, 9H). **¹³C-NMR** (63 MHz, CDCl₃): δ 128.38, 128.27, 128.14, 126.60, 126.42, 126.30, 76.65, 65.02, 28.72, 19.98, 0.34. ESI-MS (m/z): 551.3176 [M+H], calcd for: C₃₃H₄₇O₅Si: 551.3187

### 5-(4-Benzoylphenyl)-3-hydroxy-2-(8-noninyl)-pentanoic acid (JS-64)

Methyl 3-hydroxy-2-(9-trimethylsilyl-8-noninyl)-5-(4-(2-phenyl-1,3-dioxolan-2-yl)phenyl)-pentanoate (1.1 g, 2.0 mmol) was dissolved in trifluoroacetic acid (TFA) (10 mL) and stirred for 20 h at rt. The reaction mixture was poured in H₂O (100 mL) and extracted with CH₂Cl₂ (3 x 100 mL). The combined organic phases were dried over Na₂SO₄, concentrated under reduced pressure and the product obtained was used for the next reaction without any further purification, *R*_{f} = 0.14 (hexane/EtOAc = 5 : 1). This product was dissolved in THF (100 mL) and added to the solution of NaOH (1.0 g, 25.0 mmol) in H₂O (25 mL) and MeOH (25 mL). The reaction mixture was stirred for 4 h at rt. The volatiles were distilled off under reduced pressure and of conc. HCl (10 mL) was added. Phases were separated and the aq. phase was extracted with CH₂Cl₂ (2 x 100 mL) and EtOAc (100 mL). The combined organic phases were dried over Na₂SO₄ and concentrated under reduced pressure. The crude product was purified by column chromatography (5 cm × 20 cm, hexane/EtOAc = 3 : 1 to 1 : 2) to yield 300 mg (36%) of a product as a colorless solid.

*Rf* = 0.42 (hexane/EtOAc = 1 : 1). **¹H-NMR** (360 MHz, CDCl₃): δ 7.84 - 7.69 (m, 4H), 7.61 - 7.53 (m, 1 H), 7.46 (dd, *J* = 8.3, 6.9 Hz, 2H), 7.30 (d, *J* = 7.9 Hz, 2H), 3.88 (dt, *J* = 9.4, 3.8 Hz, 1 H), 2.96 (ddd, *J* = 14.4, 9.5, 5.2 Hz, 1 H), 2.75 (ddd, *J* = 13.7, 9.2, 7.1 Hz, 1 H), 2.51 (dt, *J* = 9.5, 4.3 Hz, 1 H), 2.15 (td*, J =* 7.0, 2.7 Hz, 2H), 1.92 (t, *J* = 2.6 Hz, 1 H), 1.90-1.68 (m, 4H), 1.66 - 1.43 (m, 2H), 1.43 - 1.19 (m, 8H). **¹³C-NMR** (91 MHz, CDCl₃): δ 196.76, 180.17, 147.05, 137.86, 135.49, 132.43, 130.61, 130.09, 129.54, 128.54, 128.43, 128.36, 84.78, 84.76, 77.36, 71.28, 68.31, 51.05, 36.90, 35.46, 32.40, 32.21, 29.52, 29.43, 28.94, 28.72, 28.54, 28.49, 27.81, 27.34, 26.93, 18.46. ESI-MS (m/z): 421.2375 [M+H], calcd for: C₂₇H₃₃O₄: 421.2373

### 5-(4-Benzoylphenyl)-3-hydroxy-2-(8-noninyl)-pentanamide (JS-65)

Di-*tert*-butyl dicarbonate (179 mg, 0.82 mmol), NH₄HCO₃ (62 mg, 0.78 mmol) and pyridine (30 µL, 0.36 mmol) were added to the solution of 5-(4-benzoylphenyl)-3-hydroxy-2-(8-noninyl)-pentanoic acid **(JS-64)** (150 mg, 0.36 mmol) in acetonitril (3 mL). The reaction mixture was stirred for 14 h at rt. Volatiles were distilled off under reduced pressure and the residue was purified by column chromatography (1.5 cm × 20 cm, hexane/EtOAc = 2/1 to 1/3), yielding 26.3 mg (18%) of the product as a light yellow solid. *R*_{f} = 0.19 (hexane / EtOAc = 1 : 1). ¹H NMR (360 MHz, CDCl₃): δ 7.82 - 7.66 (m, 4H), 7.66 - 7.53 (m, 1 H), 7.47 (dd, *J* = 8.4, 6.8 Hz, 2H), 7.40 - 7.16 (m, 2H), 5.84 (s, 1 H), 5.75 (s, 1 H), 3.84 (dq, *J* = 9.9, 3.4 Hz, 1 H), 3.28 (d, *J* = 3.6 Hz, 1 H), 2.94 (ddd, *J* = 14.4, 9.7, 5.1 Hz, 1 H), 2.86 - 2.61 (m, 1 H), 2.19 (dtd, *J* = 20.4, 7.3, 3.2 Hz, 2H), 1.96 - 1.84 (m, 3H), 1.73 (tdd, *J =* 13.0, 5.3, 3.0 Hz, 2H), 1.50 (dq, *J =* 14.4, 6.9 Hz, 2H), 1.43 - 1.12 (m, 8H). ¹³C NMR (91 MHz, CDCl₃): δ 196.64, 177.91, 147.19, 137.92, 135.52, 132.50, 132.41, 130.56, 130.24, 130.20, 130.07, 128.60, 128.57, 128.45, 128.38, 127.48, 84.80, 77.36, 71.46, 68.29, 51.55, 35.54, 32.58, 29.71, 29.03, 28.75, 28.52, 27.98, 26.55, 18.48. ESI-MS (m/z): 420.2537 [M+H], calcd for C₂₇H₃₄NO₃+: 420.2533

### Example 2: Activity against S. aureus virulence

Several compounds of the invention as well as reference compounds have been tested against *S*. *aureus* NCTC 8325 on sheep blood agar. Hemolysins are key virulence factors of *S. aureus* that contribute to the pathogenicity of these bacteria. The production of hemolysins can be easily monitored by lysis of sheep blood and generation of a clear zone around the bacterial colonies on the agar plate. Hemolysins are co-regulated with the majority of important virulence factors and have been qualified in previous studies as a reliable measure of agr quorum sensing regulated virulence (T. Böttcher, S. A. Sieber, J Am Chem Soc 2008, 130, 14400).

Agar plate-based hemolysis was tested on 5% sheep blood agar plates (Heipha Diagnostika, Eppelheim, Germany). Sterile circles of Whatman^{®} cards (No. 1, Schleicher & Schuell) with 5.5 mm diameter were placed on the agar plates and inoculated with 2.5 µL of the corresponding lactone/DMSO and 2.5 µL stationary phase culture of S. *aureus* NCTC 8325 diluted to OD₆₀₀ = 0.13. The plates were incubated over night at 37°C and the diameters of the zones around the bacterial colonies were precisely measured using a vernier caliper. Doses of the compounds required to produce the half maximum effect (ED₅₀) were calculated from curve fittings by Microcal™ Origin 6.0.

The structures of the compounds tested in the agar plate-based hemolysis assay are shown in the following (AVU4ADE and AVU1AAcTE are reference compounds):

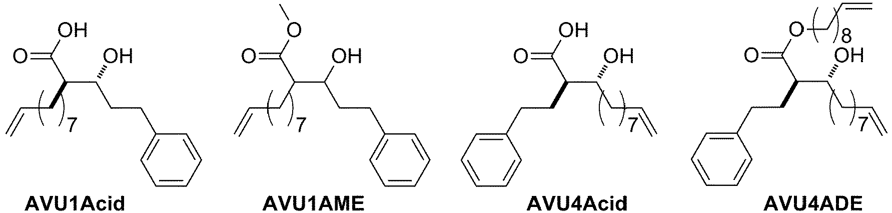

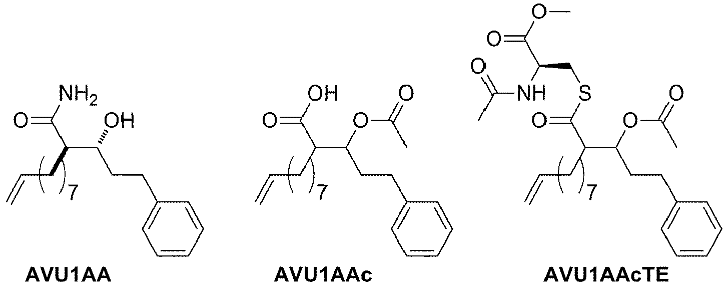

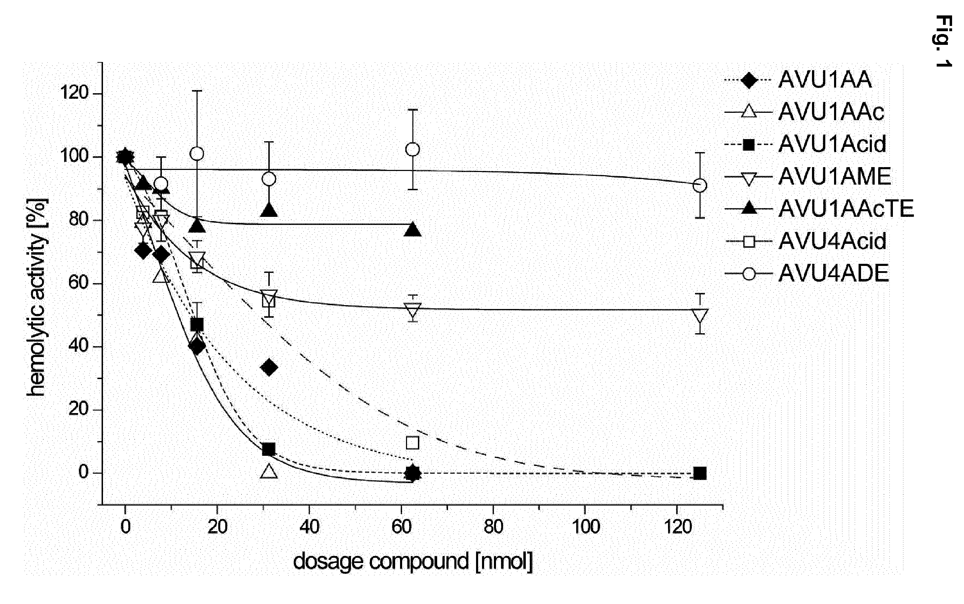

The results of this agar plate-based test are shown in Figure 1. As can be seen, the compounds AVU1Acid and AVU4Acid which have free carboxylic acid groups were found to be potent virulence inhibitors. The amide AVU1AA also turned out to be a potent inhibitor. The beta-O-acetyl compound AVU1AAc and the ester AVU1AME showed high and moderate activities, respectively. Very long chained modifications like a 9-decenyl residue in AVU4ADE (reference compound), however, impaired activity. Also, a bulky thioester of AVU1Acid with beta-O-acetyl modification, AVU1AAcTE (reference compound), showed only low efficiency and incomplete inhibition of hemolysis.

Agar-plate based assays address the compound activity against adherent bacterial colonies, more closely reflecting clinical patterns with bacterial tissue colonization such as abscesses or local infections, whereas systemic application of a compound against circulating pathogens can be better mimicked by liquid cultures.

The inhibition of hemolysin production has thus been further analysed in liquid bacterial cultures in order to evaluate their potential effects on bacterial growth simultaneously. Besides AVU1Acid, AVU1AAc, AVU1AA and VK273 the following further compounds were tested in liquid bacterial cultures:

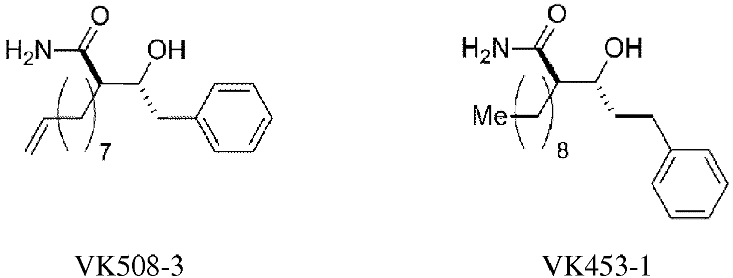

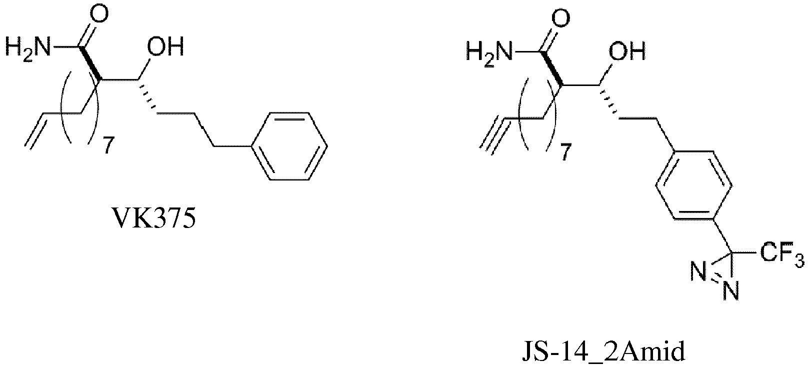

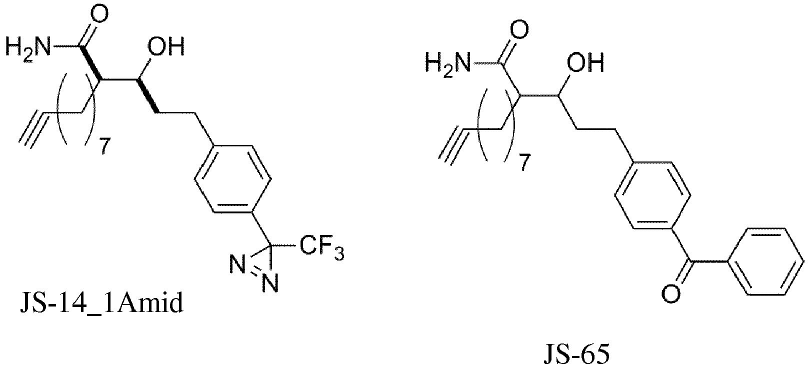

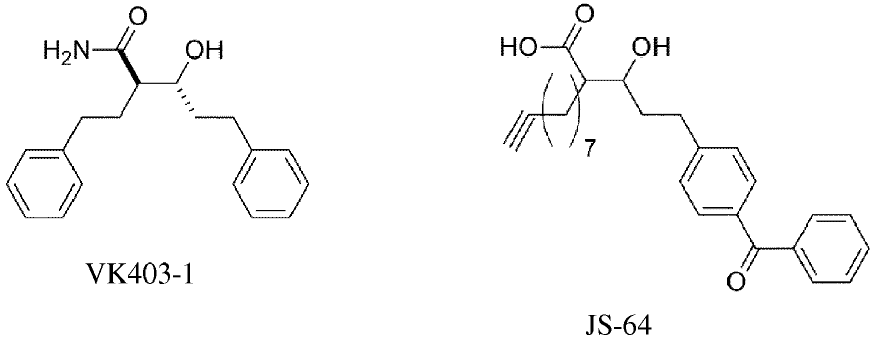

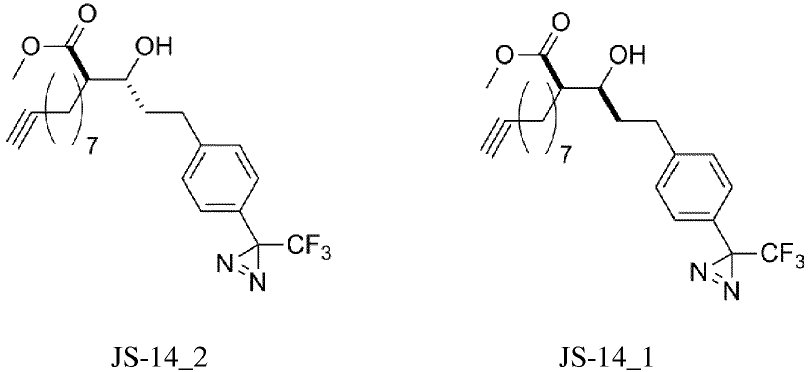

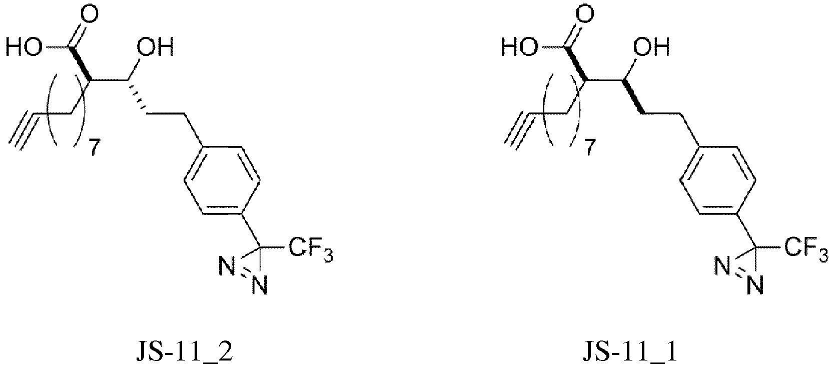

1 ml of an appropriate bacterial broth for the standard laboratory *S. aureus* strain NCTC 8325 were inoculated with 1*10⁶ cfu. The cultures were kept overnight at 37°C with gentle shaking 300 rpm in the presence of compounds AVU1Acid, AVU1AAc, AVU1AA or VK273 according to the present invention at different concentrations or dimethyl sulfoxide (DMSO) as control. Different beta-lactone virulence inhibitors were used as reference compounds. Following overnight incubation the optical density was measured in order to determine the minimal inhibiting concentration (MIC), which is defined as the lowest concentration of an antimicrobial that will inhibit the visible growth of a microorganism after overnight incubation. A 100 µl fraction of the supernatant of the grown bacterial cultures was subsequently analysed for the content of secreted bacterial hemolysins by addition of sheep red blood cells (10% v/v in PBS) and the measurement of the optical density at 410 nm after 30 min incubation at 37°C.

The following beta-lactones AVU1, AVU4, AVU5, AVU6 and AVU7 were used as reference compounds in the liquid culture-based hemolysis assay:

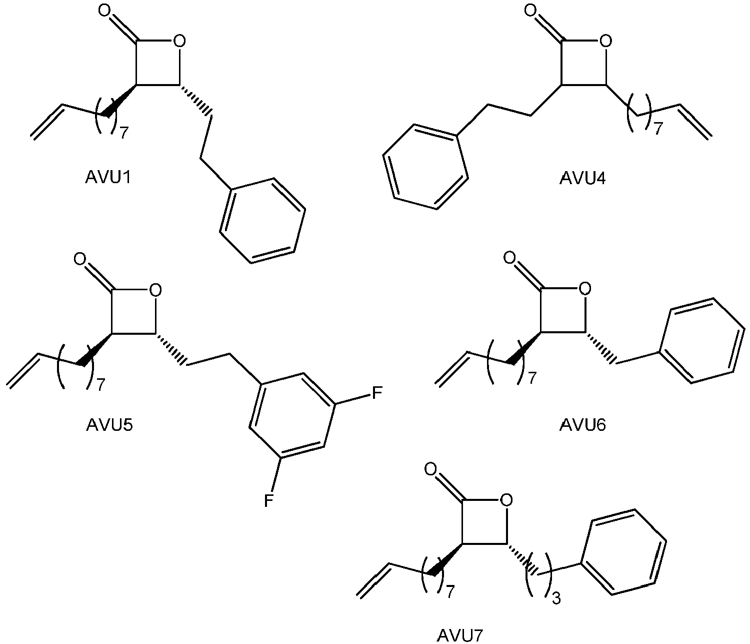

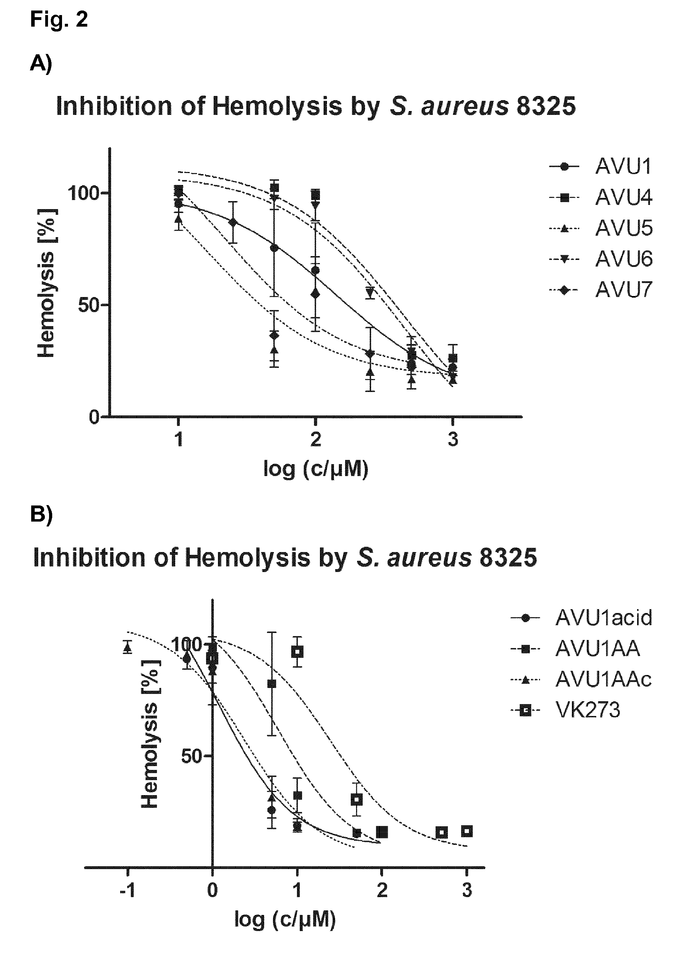

The following results were obtained in the liquid culture-based hemolysis assay, as also shown in Figure 2:

**Assay 1:**

| | **AVU1** | **AVU4** | **AVU5** | **AVU6** | **AVU7** | **AVU1Acid** | **AVU1AA** | **AVU1AAc** | **VK273** |
|---|---|---|---|---|---|---|---|---|---|
| **MIC [µM]** | >1000 | >1000 | >1000 | >1000 | >1000 | 50-100 | >1000 | 50-100 | >1000 |
| **EC₅₀ [µM]** | 141.5 | 434.8 | 16.10 | 440.2 | 22.04 | 1.175 | 6.031 | 2.426 | 24.78 |

The following results were obtained in further liquid culture-based hemolysis assays. Since the assays were performed at different times with different charges of sheep red blood cells AVU1 has been tested as a reference substance in each assay. The resulting EC₅₀ values determined for the tested compounds have to be assessed with respect to the EC₅₀ value determined for AVU1.

**Assay 2:**

| | **AVU1** | **VK453-1** | **JS-64** | **JS-65** |
|---|---|---|---|---|
| **MIC [µM]** | >1000 | >1000 | >1000 | >1000 |
| **EC₅₀ [µM]** | 10-50 | 1-5 | 1-5 | 1-5 |

**Assay 3:**

| | **AVU1** | **VK375** | **VK508-3** | **VK403-1** |
|---|---|---|---|---|
| **MIC [µM]** | >1000 | >1000 | >1000 | >1000 |
| **EC₅₀ [µM]** | 50-100 | 1-5 | 10-50 | 50-100 |

**Assay 4:**

| | **AVU1** | **JS-11_2** | **JS-11_1** | **JS-14_2Amid** | **JS-14_1Amid** | **JS-14_2** | **JS-14_1** |
|---|---|---|---|---|---|---|---|
| **MIC [µM]** | >1000 | 10-50 | 10-50 | >1000 | >1000 | >1000 | >1000 |
| **EC₅₀ [µM]** | 31 | 2.2 | 3.6 | 2.8 | 2.3 | 64 | 166 |

For the compounds AVU1Acid, AVU1AAc, JS-11_2 and JS-11_1 according to the invention, an antimicrobial effect was observed, as shown by MIC values of 50-100 µM in assay 1 and 10-50 µM in assay 4, in combination with a clear anti-hemolytic effect in sub-inhibitory concentrations with EC₅₀ values of 1.175 µM, 2.426 µM, 2.2 µM and 3.6 µM, respectively. Certain members of this compound class, such as AVU1AA, VK273, VK453-1, JS-64, JS-65, VK375, VK508-3, VK403-1, JS-14_2Amid, JS-14_1Amid, JS-14_2 and JS-14_1 show no antimicrobial effect at concentrations up to 1000 µM but have potent activity in virulence inhibition (EC₅₀ = 6.031 µM, 24.78 µM, 1-5 µM, 1-5 µM, 1-5 µM, 1-5 µM, 10-50 µM, 50-100 µM, 2.8 µM, 2.3 µM, 64 µM and 166 µM, respectively).

As also shown in Figure 2A, beta-lactone compounds tested for comparison are less active in virulence inhibition under these assay conditions than the compounds of the invention. These results demonstrate that the compounds of the present invention are advantageous in that they allow specifically targeting virulence with up to several orders of magnitude between their EC₅₀ values for virulence inhibition and the corresponding MIC values.

The compounds of the present invention have thus been demonstrated to be highly effective in inhibiting the production of hemolysins by *S. aureus* and, thus, to be potent virulence inhibitors. These results indicate the usefulness of the compounds according to the invention in the therapeutic intervention in bacterial infections, including particularly infections with *S.aureus.*

### Example 3: In vitro inhibition of recombinant S. aureus ClpP

The ClpP protease from *S. aureus* NCTC8325 (SaClpP) was recombinantly overexpressed in *E*. *coli* and purified in order to investigate enzyme inhibition using SLY-AMC (N-succinyl-Leu-Tyr-7-amido-4-methylcoumarin) as fluorogenic model substrate.

Precisely, 9 µl of SaClpP in buffered solution (final concentration 0.05 mg/ml, 2 µM; buffer: 100 mM NaCl, 20 mM Tris, pH 7.0) were incubated in the presence of 1 µl of various inhibitors (final concentration 2 mM = 1000x) for 30 min at room temperature (RT). Subsequently, SLY-AMC was added to a final concentration of 200 µM and the increase in fluorescence due to enzymatic substrate proteolysis was measured using a TECAN GENios Pro™ plate reader at 380/440 nm (excitation/emission) at time intervals of 60 s over 40 min at 37°C. The enzymatic activity was determined as the slope of fluorescence (λ = 440 nm) over time. As positive controls, the *in vivo* active beta-lactone compound AVU1 ("U1"; WO2009/106211) and the *in vitro* potent ClpP beta-lactone inhibitor D3 (WO 2009/106211) were employed.

The results obtained in this experiment are shown in Figure 3. As can be seen, the compounds VK273, AVU1AA, AVU4Acid, AVU1AAc and AVU1AME according to the invention have been found to inhibit SaClpP at an at least comparable level as the *in vivo* active beta-lactone reference compound AVU1. These results indicate that the compounds of the present invention are potent inhibitors of ClpP.

### Example 4: Competition of S. aureus proteome labeling

The compound U1 P was used as a probe for activity-based labeling (activity-based protein profiling, ABPP) of the *S. aureus* NCTC 8325 proteome, as described in WO 2009/106211.

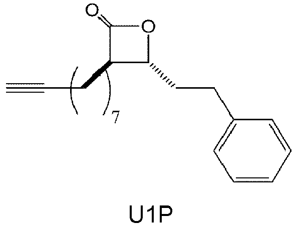

The U1 P ABPP probe represents the lead structures of the known beta-lactone ClpP inhibitors modified with a terminal alkyne group. This compound is both active in hemolysis inhibition and ClpP inhibition. The terminal alkyne group comprised in U1P can be used for appending a fluorophore such as rhodamine-azide using click chemistry (1,3-dipolar Huisgen cycloaddition), which allows to scan the proteome of S. *aureus* for possible covalent binding partners. This compound consistently labels ClpP protease in a covalent manner in *S. aureus* proteomes.

The inventors used this compound in order to investigate whether compounds according to the present invention are able to reduce or suppress by non-covalent binding the covalent binding of the U1 P ABPP probe to proteins of the S. *aureus* proteome. For a detailed description of the technique underlying these experiments see T. Böttcher, M. Pitscheider S.A. Sieber, Angew Chem Int Ed Engl. 2010, 2680.

Living *S. aureus* NCTC8325 cells from a fresh stationary liquid culture were aliquoted to equivalents of 1 ml of OD₆₀₀ = 2. Bacterial cells were washed and resuspended in 100 µl PBS. Bacterial cell aliquots were then pre-incubated with the compounds AVU1AA or VK273 of the invention for 1 h at RT at final concentrations of 5 or 10 mM. Subsequently, the covalent ABPP probe U1P was added to a final concentration of 100 µM for 30 min at RT. After the competitive labeling procedure bacterial cells were washed twice, disrupted and labeled with rhodamine-azide using click chemistry. The labeled *S. aureus* proteome samples were separated by SDS-PAGE and visualized using fluorescence scanning.

In these tests, the U1P probe (covalent beta-lactone compound) gave a strong labeling signal with ClpP as the main target (Figure 4) when applied in the absence of the any competitive compounds of the present invention. However, pre-incubation with two different ClpP inhibitors according to the invention (AVU1AA and VK273) significantly reduced target binding of U1 P in S. *aureus* proteomes. The incubation of bacterial cells with the ClpP inhibitors AVU1AA and VK273 in the absence of U1 P did not result in any unspecific labeling (Figure 4). These results indicate that the compounds according to the present invention, including AVU1AA and VK273, bind to ClpP in a non-covalent manner.

### Example 5: In vivo efficacy in the treatment of S. aureus infections in mice by subcutaneous application

Further investigations to identify the *in vivo* efficacy for the treatment of systemic *S. aureus* infections in mice have been performed. Test compounds were formulated in DMSO. DMSO alone served as control vehicle. Two groups of each 8 individual hairless, immunocompetent mice (SKH1) were included in the precise experiments. All mice were infected by subcutaneous injection of 1.5*10⁸ cfu of *S. aureus* NCTC8325 in the dorsum of the animal. Subsequently, 1.5 hours after the infection, the mice were treated by a single subcutaneous infection at the same place than the infection was performed. The mice were injected with either 40 µl DMSO alone (vehicle control group) or 1.3 mg AVU1AA in 40 µl DMSO. The mice were monitored for abscess size and body weigth over a time period of 14 days. Data are represented in Fig. 5 as mean +/- standard error of the mean. For normalization purposes the abscess size of the vehicle control group at day 1 was set to 100%.

### Example 6: In vivo efficacy in the treatment of S. aureus infections in mice by systemic application

In order to investigate the possibility for systemic application for treatment of the compounds presented herein further efficacy studies were performed. Test compounds: AVU1AA and Vancomycin (standard antibiotic treatment for comparison) were dissolved in DMSO. AVU1AA DMSO stock was diluted 1:10 in corn oil for intraperitoneal injection. Vancomycin was diluted 1:10 in PBS for intraperitoneal injection. The vehicle control group obtained 10% DMSO in PBS. Three groups of each 8 individual mice (BALB/C) were included in the precise experiments. All mice were infected by subcutaneous injection of 1.5*10⁸ cfu of S. *aureus* NCTC8325 in the dorsum of the animal. Subsequently, 1.5 hours after the infection, the mice were treated by a single intraperitoneal injection. The mice were injected with each 100µl injection solution. The AVU1AA group obtained 150mg/kg of AVU1AA. The Vancomycin group received 40mg/kg of Vancomycin. The control group received the vehicle control as described above. The mice were monitored for abscess size and body weigth over a time period of 14 days. Data are represented in Fig. 6 as mean +/- standard error of the mean. For normalization purposes the abscess size of the vehicle control group at day 1 was set to 100%.

These data show that the compounds of the present invention, including in particular AVU1AA, are effective *in vivo* in the treatment of bacterial infections, such as, e.g., infections with *S. aureus.* Since the administration of test compounds was started only 1.5 hours after infection with *S. aureus* and, thus, before the onset of pathological symptoms, these data also indicate that the compounds of the invention are useful in the amelioration or prevention of bacterial infections, including infections with *S. aureus.* Moreover, the compound AVU1AA according to the invention was found to be effective also in a systemic application regime, which indicates the advantageous stability properties of the compounds of the invention in the medical intervention of bacterial infections.

### Example 7: Comparison of stability of AVU1 and AVU1AA in human and mouse plasma.

We investigated the stability of the compound herein in plasma preparations in comparison the beta-lactone compounds. Human plasma was obtained lyophilized from Sigma and reconstituted in sterile H2O. For human plasma 3.8% trisodium citrate was used as anticoagulant. Mouse plasma was prepared from whole blood samples from SKH-1 and Balb/C mice using Lithium-Heparin as anticoagulant and pooled from at least three different donor animals. Plasma samples were sterile filtered and stored in aliquots at -20°C. Every experiment contained positive control samples with Procaine at 100 µM final concentration, where the half life a Procaine showed to be in a range of 6-9 minutes and therefore demonstrated the reproducible functionality of the plasma stability assay. Test compounds were diluted 1:100 from a DMSO stock solution to a final concentration of 100µM in both human and mouse plasma prewarmed for 10 min in an orbital shaker to 37°C. Test compounds were further incubated at 37°C in the orbital shaker. Samples were withdrawn at time points 0, 5, 10, 20, 30, 60, 120, 240 min and immediately quenched by the addition of an equal volume of ice-cold acetonitrile. Subsequently, all samples were spun at maximum speed in a microcentrifuge to pellet precipitated proteins and samples were prepared for LC-MS analysis. Compound quantification occured through analysis via the LTQ-FT high resolution mass-spectrometer and XCalibur-Software purchased at Thermo Scientific, Dreieich, D-63303 Germany.

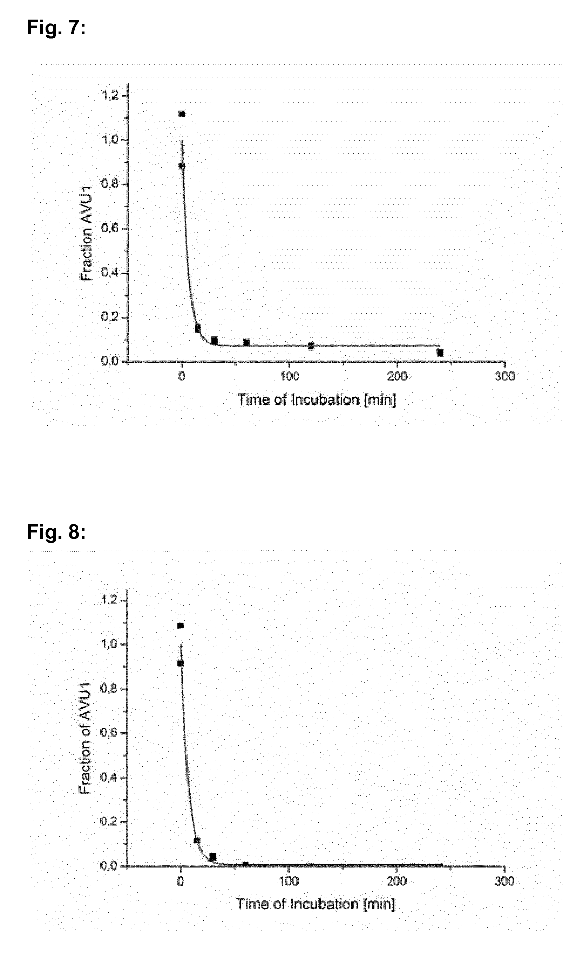

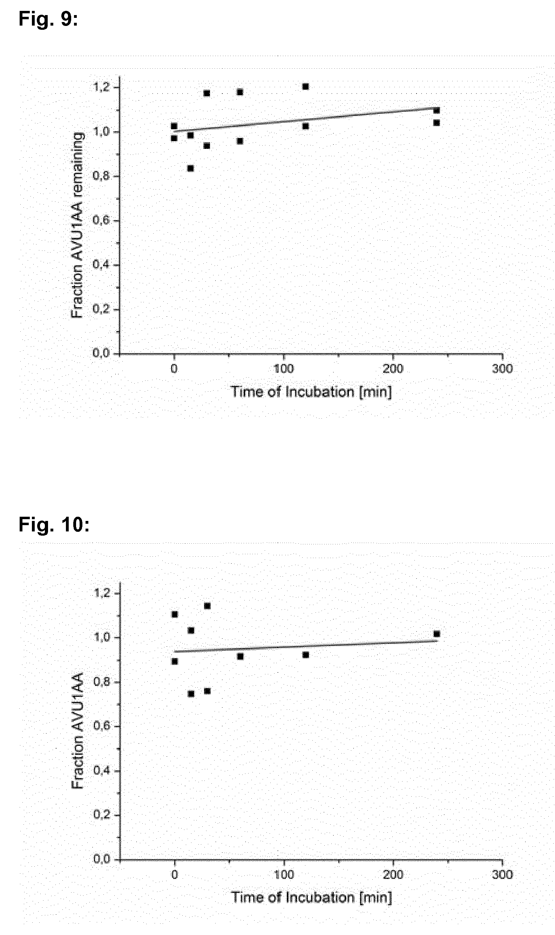

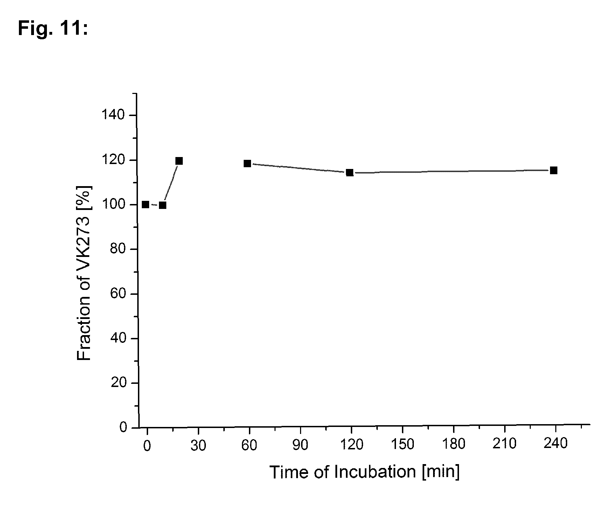

Fig. 7 shows time dependence presence of AVU1 in human and Fig. 8 in mouse plasma. Fig. 9 shows time dependence presence of AVU1AA in human and Fig. 10 in mouse plasma. As can be seen from these figures AVU1 is degraded within a few minutes in human as well as mouse plasma whereas AVU1AA is stable in both plasmas for hours. The totally unexpected stability of the compounds according to the invention allows for a systemic application of the compounds, e. g. for the treatment or prevention of an infection, and results in an increased bioavailability in organisms. Fig. 11 shows time dependence of the presence of VK273 in mouse plasma.

## Claims

1. A compound of formula (I) 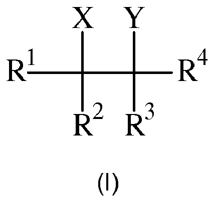 wherein:
X is selected from -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, -CH₂-NH(C₁₋₄ alkyl), -CO-O(C₁₋₆ alkyl), -CO-O(C₂₋₆ alkenyl), -CO-O(C₂₋₆ alkynyl), -CO-O(aryl), -CO-O(heteroaryl), -CO-S(C₁₋₆ alkyl), -CO-S(C₂₋₆ alkenyl), -CO-S(C₂₋₆ alkynyl), -CO-S(aryl), or -CO-S(heteroaryl);
Y is selected from -OH, -O-CO-(C₁₋₆ alkyl), -O(C₁₋₆ alkyl), -O(C₂₋₆ alkenyl), -O(C₂₋₆ alkynyl), -O-CO-(C₂₋₆ alkenyl), -O-CO-(C₂₋₆ alkynyl), -O(aryl), -O(heteroaryl), -SH, -S(C₁₋₆ alkyl), -S(C₂₋₆ alkenyl), -S(C₂₋₆ alkynyl), -S-CO-(C₁₋₆ alkyl), -S-CO-(C₂₋₆ alkenyl), -S-CO-(C₂₋₆ alkynyl), -S(aryl), -S(heteroaryl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl).
R¹ and R³ are each independently selected from C₂₋₁₂ alkenyl, -(C₁₋₁₂alkylene)-aryl, C₁₋₁₂ alkyl, C₂₋₁₂ alkynyl, aryl, heteroaryl, -(C₂₋₁₂ alkenylene)-aryl, -(C₂₋₁₂ alkynylene)-aryl, -(C₁₋₁₂ alkylene)-heteroaryl, -(C₂₋₁₂ alkenylene)-heteroaryl, -(C₂₋₁₂ alkynylene)-heteroaryl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, wherein said C₁₋₁₂ alkyl, said C₂₋₁₂ alkenyl, said C₂₋₁₂ alkynyl, said aryl, said heteroaryl, said -(C₁₋₁₂ alkylene)-aryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, said -(C₂₋₁₂ alkynylene)-heteroaryl, said polyethylene glycol moiety, or said C₃₋₁₂ alkyl is optionally substituted with one or more groups independently selected from halogen, -CN, -CF₃,-CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), heteroaryl, polyethylene glycol moiety, alkylene-polyethylene glycol moiety or polyethylene glycol-alkyl moiety, wherein at least one of R¹ and R³ is said -(C₁₋₁₂ alkylene)-aryl, said aryl, said heteroaryl, said -(C₂₋₁₂ alkenylene)-aryl, said -(C₂₋₁₂ alkynylene)-aryl, said -(C₁₋₁₂ alkylene)-heteroaryl, said -(C₂₋₁₂ alkenylene)-heteroaryl, or said -(C₂₋₁₂ alkynylene)-heteroaryl or comprises at least 4 consecutively arranged C-atoms that are covalently linked together if X is -CH₂-OH; and
R² and R⁴ are each -H;
or a pharmaceutically acceptable salt, solvate or prodrug thereof
for use as a medicament.

2. The compound of claim 1 for use as a medicament, wherein X is selected from -COOH, -CH₂-OH, -CO-O(C₁₋₄ alkyl), -CO-SH, -CO-S(C₁₋₄ alkyl), -CO-NH(C₁₋₄ alkyl), -CO-N(C₁₋₄ alkyl)(C₁₋₄ alkyl), -CH₂-SH, -CH₂-NH₂, or -CH₂-NH(C₁₋₄ alkyl).

3. The compound of claim 1 or 2 for use as a medicament, wherein Y is selected from -OH, -O-CO-(C₁₋₆ alkyl), -O(C₁₋₆ alkyl), -NH₂, -NH(C₁₋₆ alkyl), -N(C₁₋₆ alkyl)(C₁₋₆ alkyl), -NH-CO-(C₁₋₆ alkyl), or -N(C₁₋₆ alkyl)-CO-(C₁₋₆ alkyl).

4. The compound of any of claims 1 to 3 for use as a medicament, wherein one of R¹ and R³ is C₃₋₁₂ alkenyl, C₃₋₁₂ alkyl, C₃₋₁₂ alkynyl, a polyethylene glycol moiety, or C₃₋₁₂ alkyl in which at least one and up to one third of the C atoms are in each case substituted by an atom or a moiety independently selected from -O-, -S-, -NH-, -SO-, or -SO₂-, and the other one of R¹ and R³ is -(C₁₋₆ alkylene)-phenyl, -(C₂₋₆ alkenylene)-phenyl, -(C₂₋₆ alkynylene)-phenyl or phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety,
or
wherein R¹ and R³ are each independently selected from C₃₋₁₂ alkenyl, C₃₋₁₂ alkyl, C₃₋₁₂ alkynyl, a polyethylene glycol moiety, C₃₋₁₂ alkyl in which at least one and up to one third of the C atoms are in each case substituted by an atom or a moiety independently selected from -O-, -S-, -NH-, -SO-, or -SO₂-, -(C₁₋₆ alkylene)-phenyl, -(C₂₋₆ alkenylene)-phenyl, -(C₂₋₆ alkynylene)-phenyl or phenyl, wherein at least one of R¹ and R³ is said -(C₁₋₆ alkylene)-phenyl, said -(C₂₋₆ alkenylene)-phenyl, said -(C₂₋₆ alkynylene)-phenyl or said phenyl, wherein said phenyl, the phenyl moiety comprised in said -(C₁₋₆ alkylene)-phenyl, the phenyl moiety comprised in said -(C₂₋₆ alkenylene)-phenyl or the phenyl moiety comprised in said -(C₂₋₆ alkynylene)-phenyl is optionally substituted with one or more groups independently selected from halogen, -CN, -CF₃, -CN₂CF₃, benzoyl, C₁₋₆ alkyl, C₂₋₆ alkenyl, C₂₋₆ alkynyl, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CO-(C₁₋₆ alkyl), -CO-NH₂, -CO-NH(C₁₋₆ alkyl), -CO-N(C₁₋₆ alkyl)(C₁₋₆ alkyl), polyethylene glycol moiety, alkylene-polyethylene glycol moiety or polyethylene glycol-alkyl moiety.

5. The compound of any of claims 1 to 4 for use as a medicament, wherein one of R¹ and R³ is linear C₃₋₁₂ alkenyl, linear C₃₋₁₂ alkyl, or linear C₃₋₁₂ alkynyl, and the other one of R¹ and R³ is -(linear C₁₋₆ alkylene)-phenyl, -(linear C₂₋₆ alkenylene)-phenyl, -(linear C₂₋₆ alkynylene)-phenyl or phenyl.

6. The compound of any of claims 1 to 5 for use as a medicament, wherein at least one of R¹ and R³ comprises at least 4 consecutively arranged C-atoms that are covalently linked together.

7. The compound of any of claims 1 to 6 for use as a medicament, wherein R¹ is -CH₂CH₂OCH₂CH₂OCH₃.

8. A compound of formula (I) 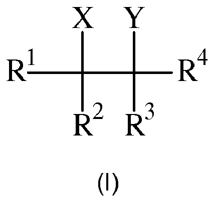 wherein:
X is selected from -COOH, -CH₂-OH, -CO-NH(C₁₋₄ alkyl), -CO-N(C₁₋₄ alkyl)(C₁₋₄ alkyl), -CO-SH, -CH₂-SH, -CH₂-NH₂, or -CH₂-NH(C₁₋₄ alkyl);
Y is selected from -OH, -O-CO-(C₁₋₆ alkyl), -NH₂, or -NH-CO-(C₁₋₆ alkyl);
R¹ and R³ are each independently selected from C₃₋₁₂ alkenyl, -(C₁₋₆ alkylene)-phenyl, C₃₋₁₂ alkyl, C₃₋₁₂ alkynyl, phenyl, -(C₂₋₆ alkenylene)-phenyl, or -(C₂₋₆ alkynylene)-phenyl, wherein at least one of R¹ and R³ is -(C₁₋₆ alkylene)-phenyl, phenyl, -(C₂₋₆ alkenylene)-phenyl, or -(C₂₋₆ alkynylene)-phenyl or comprises at least 4 consecutively arranged C-atoms that are covalently linked together if X is -CH₂-OH; and
R² and R⁴ are each -H;
or a pharmaceutically acceptable salt, solvate or prodrug thereof.

9. The compound of claim 1 for use as a medicament or the compound of claim 8, wherein said compound is a compound of one of the following formulae: 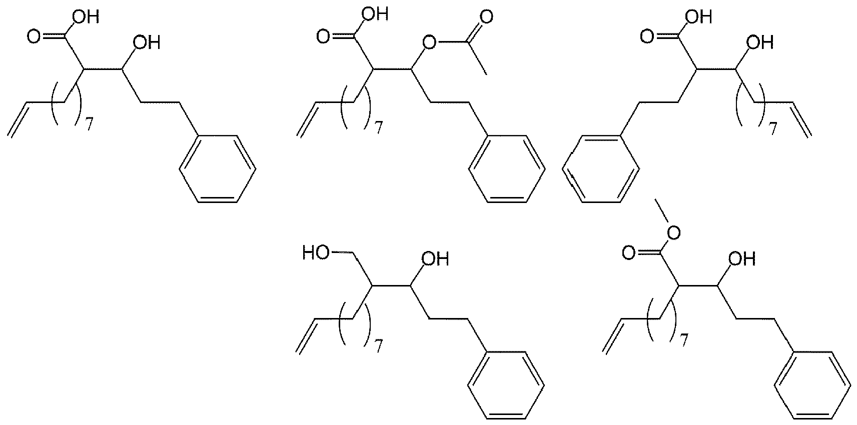 or a pharmaceutically acceptable salt, solvate or prodrug thereof.

10. The compound of claim 1 for use as a medicament, wherein said compound is a compound of one of the following formula: 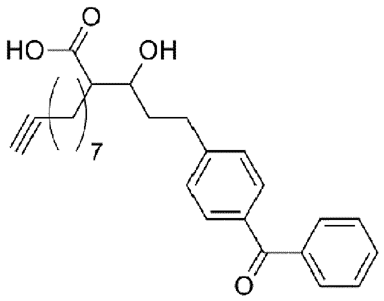 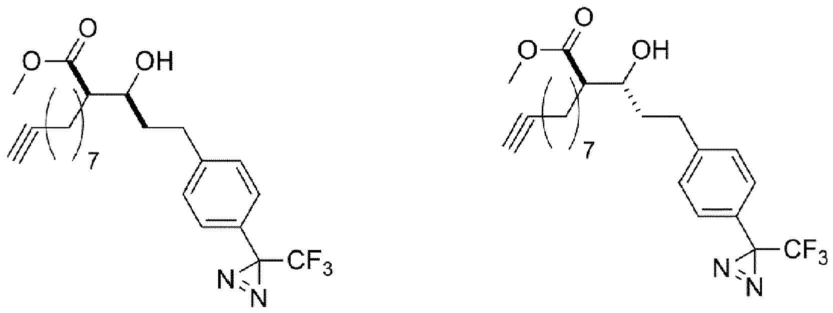 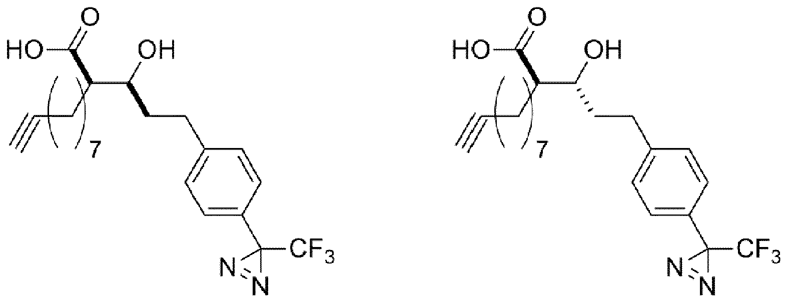 or a pharmaceutically acceptable salt, solvate or prodrug thereof.

11. The compound of any of claims 1 to 10 for use in the treatment or prevention of a bacterial or protozoan infection.

12. The compound of claim 11, wherein said bacterial infection is caused by Gram-positive bacteria selected from the group consisting of: Listeria spp., in particular *Listeria monocytogenes,* and *Listeria welshimeri;* Staphylococcus spp., in particular *Staphylococcus aureus, Staphylococcus epidermidis, Staphylococcus saprophyticus, Staphylococcus lugdunensis, Staphylococcus schleiferi,* and *Staphylococcus caprae*; Streptococcus spp., in particular *Streptococcus pneumoniae*, *Streptococcus viridans*, *Streptococcus pyogenes,* and *Streptococcus agalactiae*; Enterococcus spp., in particular *Enterococcus faecalis,* and *Enterococcus faecium;* Bacillus spp., in particular *Bacillus licheniformis, Bacillus subtilis, Bacillus anthracis*, *Bacillus cereus, Bacillus thuringiensis*, and Bacillus larvae; Mycobacterium spp., in particular *Mycobacterium tuberculosis, Mycobacterium leprae*, *Mycobacterium ulcerans*, *Mycobacterium kanasasii*, *Mycobacterium avium*, *Mycobacterium paratuberculosis*, *Mycobacterium scrofulaceam*, *Mycobacterium microti, Mycobacterium africanum*, *Mycobacterium canettii*, *Mycobacterium intracellulare*, *Mycobacterium simiae, Mycobacterium szulgai*, *Mycobacterium xenopi*, *Mycobacterium fortuitum*, *Mycobacterium chelonei,* and *Mycobacterium marinum*; *Nocardia asteroids;* and Rhodococcus equi; wherein said Gram-positive bacteria may be multidrug-resistant bacteria;
or said bacterial infection is caused by Gram-negative bacteria selected from the group consisting of: Neisseria spp., in particular *Neisseria gonorrhoeae*, and *Neisseria meningitides*; *Moraxella catarrhalis*; *Hemophilus influenzae*; Klebsiella spp., in particular *Klebsiella pneumoniae*; Legionella spp., in particular *Legionella pneumophila*; Pseudomonas spp., in particular *Pseudomonas aeruginosa*, and *Pseudomonas putida; Escherichia coli*; *Proteus mirabilis*; *Enterobacter cloaceae*; *Serratia marcescens; Helicobacter pylori;* and Salmonella spp., in particular *Salmonella enteritidis*, and *Salmonella typhi*; wherein said Gram-negative bacteria may be multidrug-resistant bacteria;
or said bacterial infection is caused by multidrug-resistant *Staphylococcus aureus* (MRSA).

13. The compound of claim 11, wherein said protozoan infection is caused by protozoa selected from the group consisting of: *Plasmodium* spp., in particular *Plasmodium falciparum, Plasmodium malariae*, *Plasmodium vivax*, *Plasmodium ovale,* and *Plasmodium knowlesi*; *Leishmania* spp., in particular *Leishmania major, Leishmania tropica*, *Leishmania aethiopica, Leishmania mexicana*, *Leishmania braziliensis*, *Leishmania donovani*, *Leishmania infantum*, and *Leishmania chagasi*; *Trypanosoma* spp., in particular *Trypanosoma brucei, Trypanosoma cruzi, Trypanosoma simiae, Trypanosoma avium*, *Trypanosoma congolense*, *Trypanosoma equinum*, *Trypanosoma equiperdum, Trypanosoma evansi*, and *Trypanosoma suis; Babesia* spp., in particular *Babesia microti,* and *Babesia bigemina*; and *Toxoplasma,* in particular *Toxoplasma gondii*; and further wherein said protozoa may be multidrug-resistant protozoa.

14. Method for sterilizing or anti-bacterial cleaning of a technical device, the method comprising the application of a compound as defined in any of claims 1 to 10 on a surface of said technical device or parts thereof.

15. Non-therapeutical use of a compound as defined in any of claims 1 to 10 for preventing the formation of biofilms or eliminating biofilms on a surface of a technical device.
